# EUROPEAN PATENT SPECIFICATION

(11) **EP 1 338 657 B1**
(45) Date of publication and mention of the grant of the patent: **18.04.2007**
(21) Application number: 02290458.5
(22) Date of filing: 25.02.2002
(51) Int. Cl.: C12Q 1/68, C12R 1/32, C07K 16/12, C07K 14/35, A61K 39/04, G01N 33/569, C12N 5/10, C12N 15/70

(54) **Sequences specifcally deleted from Mycobacterium tuberculosis genome and their use in diagnosistics and as vaccines**
Spezifisch vom Genom von Mycobacterium tuberculosis deletierte Sequenzen und deren Verwendung in der Diagnostik und als Vakzine
Séquences spécifiquement délétées du génome de Mycobacterium tuberculosis et leur utilisation dans des diagnostics et comme vaccins

(43) Date of publication of application: 27.08.2003
(73) Proprietor: INSTITUT PASTEUR, 75015 Paris (FR)
(72) Inventor: Cole, Stewart, 75724 Paris Cedex 15 (FR); Brosch, Roland, 75724 Paris Cedex 15 (FR); Gordon, Stephen, Glyn Hewinson, New Haw, Addelstone, Surrey KT15 3NB (GB); Eiglmeier, Karin, 75724 Paris Cedex 15 (FR); Garnier, Thierry, 75724 Paris Cedex 15 (FR)
(74) Representative: Ahner, Francis

(56) References cited:
- WO-A-00/55362
- US-B1- 6 291 190
- COLE S T ET AL: "Deciphering the biology of Mycobacterium tuberculosis from the complete genome sequence" NATURE, MACMILLAN JOURNALS LTD. LONDON, GB, vol. 393, 11 June 1998 (1998-06-11), pages 537-544, XP002087941 ISSN: 0028-0836 & DATABASE GENBANK [Online] NCBI7 September 2001 (2001-09-07) COLE S.T. ET AL.: "Mycobacterium tuberculosis H37Rv complete genome." retrieved from HTTP://WWW.NCBI.NLM.NIH.GOV Database accession no. NC_000962
- DATABASE GENBANK [Online] NCBI3 August 2001 (2001-08-03) COLE S.T. ET AL.: "Mycobacterium tuberculosis H37Rv complete genome; segment 69/162" retrieved from HTTP://WWW.NCBI.NLM.NIH.GOV Database accession no. Z74020 XP002206252
- DATABASE TAXONOMY BROWSER [Online] NCBI Host: http://www.ncbi.nih.gov, "Mycobacterium tuberculosis complex" XP002206354
- GORDON S V ET AL: "Genomics of Mycobacterium bovis." TUBERCULOSIS (EDINBURGH), vol. 81, no. 1-2, 2001, pages 157-163, XP001088346 ISSN: 1472-9792
- SREEVATSAN SRINAND ET AL: "Restricted structural gene polymorphism in the Mycobacterium tuberculosis complex indicates evolutionarily recent global dissemination." PROCEEDINGS OF THE NATIONAL ACADEMY OF SCIENCES OF THE UNITED STATES, vol. 94, no. 18, 1997, pages 9869-9874, XP002206250 1997 ISSN: 0027-8424
- BROSCH R ET AL: "A new evolutionary scenario for the Mycobacterium tuberculosis complex." PROCEEDINGS OF THE NATIONAL ACADEMY OF SCIENCES OF THE UNITED STATES, vol. 99, no. 6, 19 March 2002 (2002-03-19), pages 3684-3689, XP002206251 http://www.pnas.org March 19, 2002 ISSN: 0027-8424 & DATABASE EBMBL [Online] EBI16 March 2002 (2002-03-16) BROSCH R ET AL.: "Mycobacterium tuberculosis mmpS6 gene and mmpL6 gene"

### SEQUENCE LISTING

<110> Institut PASTEUR
<120> DELETED SEQUENCE IN M. TUBERCULOSIS, METHOD FOR DETECTING MYCOBACTERIA USING THESE SEQUENCES AND VACCINES
<130> D20110
<160> 20
<170> Patent In Ver. 2.1
<210> 1
   <211> 3953
   <212> DNA
   <213> Mycobacterium tuberculosis
<220>
   <221> CDS
   <222> (735)..(3638)
<400> 1
   tccagcgcgg ccatcagcga tgaactctgg gacctgctac ccggctacct catcttccgg 60
   tccatcatcc ccaaccggcc gcccacccag gacacggtgc aagccctcgt cgacgacgtg 120
   atactcccca gcctcacccg atccaccggt tgagtcagcg gtgcgaatgg ctgggcaccg 180
   ttgtggtgtc cggtcccgta ccgtactgtt gaatccgcgg atccccgcct gaggtacggg 240
   gcgtggtcgc gccccgggca atagcgtcgc cggttatcga aaggctaacg ggtgcagggg 300
   atttcagtga ctggcctggt caaacgcggc tggatggtgc tggttgccgt ggcggtggtg 360
   gcggtcgcgg gattcagcgt ctatcggttg cacggcatct tcggctcgca cgacaccacc 420
   tcgaccgccg gtggtgtcgc gaacgacatc aagccgttca accccaaaca ggtaaccctc 480
   gaggtctttg gcgctcccgg aaccgtggca acgatcaatt atctggacgt ggatgccaca 540
   cctcggcaag tcctggacac gaccctgccg tggtcataca cgatcacgac gaccctgccc 600
   gcggtcttcg ccaatgttgt cgcgcaaggc gacagcaatt ccatcggctg ccgcatcacc 660
   gtcaacggtg tagtcaagga cgaaaggatc gtcaacgaag tgcgcgccta taccttctgc 720
   ctcgacaagt cctc atg agc aac cac cac cgc ccg cgg cct tgg ttg ccg    770
                   Met Ser Asn His His Arg Pro Arg Pro Trp Leu Pro
                    1               5                  10
   cac acc atc cga cgg ctt tcg ttg ccg atc ttg ctg ttt tgg gtg ggt    818
   His Thr Ile Arg Arg Leu Ser Leu Pro Ile Leu Leu Phe Trp Val Gly
           15                  20                  25
   gtg gcc gcc ata acc aat gcc gcc gtg ccg caa ttg gag gtg gtc ggg    866
   Val Ala Ala Ile Thr Asn Ala Ala Val Pro Gln Leu Glu Val Val Gly
       30                  35                  40
   gag gcg cat aac gtc gca cag agc tcc ccg gat gac ccg tcg ctg cag    914
   Glu Ala His Asn Val Ala Gln Ser Ser Pro Asp Asp Pro Ser Leu Gln
    45                  50                  55                  60
   gcg atg aaa cgc atc ggc aag gtg ttc cac gag ttc gat tcc gac agt     962
   Ala Met Lys Arg Ile Gly Lys Val Phe His Glu Phe Asp Ser Asp Ser
                   65                  70                  75
   gcg gcc atg atc gtc ttg gaa ggc gat aag ccg ctc ggc aac gac gcc    1010
   Ala Ala Met Ile Val Leu Glu Gly Asp Lys Pro Leu Gly Asn Asp Ala
               80                  85                  90
   cac cgg ttc tac gac acc ctg ctc cgc aac ctt tca aac gac acc aaa    1058
   His Arg Phe Tyr Asp Thr Leu Leu Arg Asn Leu Ser Asn Asp Thr Lys
           95                 100                 105
   cac gtc gag cac gtt cag gac ttc tgg ggc gat ccg ctg acc gcg gcc    1106
   His Val Glu His Val Gln Asp Phe Trp Gly Asp Pro Leu Thr Ala Ala
       110                 115                 120
   ggc tcg caa agc acc gac ggc aaa gcc gcc tac gtt cag gtc tat ctc    1154
   Gly Ser Gln Ser Thr Asp Gly Lys Ala Ala Tyr Val Gln Val Tyr Leu
   125                 130                 135                 140
   gcc ggc aac caa ggc gag gcg ttg tca atc gag tcc gtc gac gcg gtg    1202
   Ala Gly Asn Gln Gly Glu Ala Leu Ser Ile Glu Ser Val Asp Ala Val
                   145                 150                 155
   cgc gac atc gtc gcc cat acg cca cca ccg gcc ggg gtc aag gcc tac    1250
   Arg Asp Ile Val Ala His Thr Pro Pro Pro Ala Gly Val Lys Ala Tyr
               160                 165                 170
   gtc acc ggc gcg gcc ccg ctc atg gcc gat cag ttt cag gtg ggc agc    1298
   Val Thr Gly Ala Ala Pro Leu Met Ala Asp Gln Phe Gln Val Gly Ser
           175                 180                 185
   aaa gga acc gcg aaa gtt acc ggg ata act ctg gtt gtg atc gcg gtg    1346
   Lys Gly Thr Ala Lys Val Thr Gly Ile Thr Leu Val Val Ile Ala Val
       190                 195                 200
   atg ttg ctc ttc gta tac cgt tcc gtc gtc acc atg gtc ctg gtg ctt    1394
   Met Leu Leu Phe Val Tyr Arg Ser Val Val Thr Met Val Leu Val Leu
   205                 210                 215                 220
   atc acg gtt ctt att gag ttg gcc gcg gcc cgc ggg atc gtc gct ttt    1442
   Ile Thr Val Leu Ile Glu Leu Ala Ala Ala Arg Gly Ile Val Ala Phe
                   225                 230                 235
   ctc gga aac gcc ggg gta atc ggg ctg tcg aca tac tcg acg aat ctg    1490
   Leu Gly Asn Ala Gly Val Ile Gly Leu Ser Thr Tyr Ser Thr Asn Leu
               240                 245                 250
   ctc aca cta ttg gta atc gcg gcg ggc aca gac tac gcg att ttt gtc    1538
   Leu Thr Leu Leu Val Ile Ala Ala Gly Thr Asp Tyr Ala Ile Phe Val
           255                 260                 265
   ctc ggc cgc tat cac gag gcg cgc tac gcc gca cag gat cgg gaa acg    1586
   Leu Gly Arg Tyr His Glu Ala Arg Tyr Ala Ala Gln Asp Arg Glu Thr
       270                 275                 280
   gcc ttc tac acg atg tat cgc ggg acc gcc cac gtc gtc ttg ggc tcg    1634
   Ala Phe Tyr Thr Met Tyr Arg Gly Thr Ala His Val Val Leu Gly Ser
   285                 290                 295                 300
   ggt ctg acc gtt gcc ggc gcg gtg tat tgc ctg agc ttt acc cgg cta    1682
   Gly Leu Thr Val Ala Gly Ala Val Tyr Cys Leu Ser Phe Thr Arg Leu
                   305                 310                 315
   ccc tat ttt caa agc ctg ggt att ccc gcc tcg ata ggg gtg atg att    1730
   Pro Tyr Phe Gln Ser Leu Gly Ile Pro Ala Ser Ile Gly Val Met Ile
               320                 325                 330
   gcg ttg gca gcc gcg ctc agc ctg gcc cca tcc gtg ctc atc ttg ggc    1778
   Ala Leu Ala Ala Ala Leu Ser Leu Ala Pro Ser Val Leu Ile Leu Gly
           335                 340                 345
   agt cgt ttc ggt tgt ttc gaa ccc aag cgc agg atg agg acc agg gga    1826
   Ser Arg Phe Gly Cys Phe Glu Pro Lys Arg Arg Met Arg Thr Arg Gly
       350                 355                 360
   tgg cgg cgc atc ggc acg gcc atc gtg cgt tgg ccg gga ccc atc ctg    1874
   Trp Arg Arg Ile Gly Thr Ala Ile Val Arg Trp Pro Gly Pro Ile Leu
   365                 370                 375                 380
   gca gtg gcg tgc gca att gcg gtg gtg ggt ctg ctc gcg ctg ccg gga    1922
   Ala Val Ala Cys Ala Ile Ala Val Val Gly Leu Leu Ala Leu Pro Gly
                   385                 390                 395
   tac aaa acg agc tac gac gct cgc tat tac atg ccc gcc acc gcc ccg    1970
   Tyr Lys Thr Ser Tyr Asp Ala Arg Tyr Tyr Met Pro Ala Thr Ala Pro
               400                 405                 410
   gcc aat att ggc tac atg gcc gcg gag cga cat ttt ccc caa gcg cgg    2018
   Ala Asn Ile Gly Tyr Met Ala Ala Glu Arg His Phe Pro Gln Ala Arg
           415                 420                 425
   ctg aat ccc gaa cta ctg atg atc gag acg gat cac gat atg cgc aat    2066
   Leu Asn Pro Glu Leu Leu Met Ile Glu Thr Asp His Asp Met Arg Asn
       430                 435                 440
   ccg gcc gac atg ctc atc ttg gat agg atc gcc aag gct gtc ttc cat    2114
   Pro Ala Asp Met Leu Ile Leu Asp Arg Ile Ala Lys Ala Val Phe His
   445                 450                 455                 460
   ctg ccc ggc ata ggg ctg gtg cag gcc atg acc cgg ccg cta gga acc    2162
   Leu Pro Gly Ile Gly Leu Val Gln Ala Met Thr Arg Pro Leu Gly Thr
                   465                 470                 475
   ccg att gac cac agc tcg ata ccg ttt cag atc agc atg caa agc gtc    2210
   Pro Ile Asp His Ser Ser Ile Pro Phe Gln Ile Ser Met Gln Ser Val
               480                 485                 490
   ggc cag att cag aat ctc aag tat cag agg gac cga gca gcc gac ttg    2258
   Gly Gln Ile Gln Asn Leu Lys Tyr Gln Arg Asp Arg Ala Ala Asp Leu
           495                 500                 505
   ctg aag cag gcc gaa gag ctg ggg aag acg atc gaa atc ttg cag cgc    2306
   Leu Lys Gln Ala Glu Glu Leu Gly Lys Thr Ile Glu Ile Leu Gln Arg
       510                 515                 520
   caa tat gcc cta cag cag gaa ctc gcg gcc gct act cac gag caa gcc    2354
   Gln Tyr Ala Leu Gln Gln Glu Leu Ala Ala Ala Thr His Glu Gln Ala
   525                 530                 535                 540
   gaa agc ttt cac caa acg atc gcc acg gta aac gaa ctg cga gat agg    2402
   Glu Ser Phe His Gln Thr Ile Ala Thr Val Asn Glu Leu Arg Asp Arg
                   545                 550                 555
   atc gcc aat ttc gac gat ttc ttc agg ccg att cgt agt tac ttt tac    2450
   Ile Ala Asn Phe Asp Asp Phe Phe Arg Pro Ile Arg Ser Tyr Phe Tyr
               560                 565                 570
   tgg gaa aag cac tgc tac gat atc ccg agc tgc tgg gcg ctg aga tcc    2498
   Trp Glu Lys His Cys Tyr Asp Ile Pro Ser Cys Trp Ala Leu Arg Ser
           575                 580                 585
   gtc ttt gac acg atc gac ggt atc gac caa ctc ggc gag cag ctg gcc    2546
   Val Phe Asp Thr Ile Asp Gly Ile Asp Gln Leu Gly Glu Gln Leu Ala
       590                 595                 600
   agc gtg acc gta acc ttg gac aag ttg gct gcg atc cag cct caa ttg    2594
   Ser Val Thr Val Thr Leu Asp Lys Leu Ala Ala Ile Gln Pro Gln Leu
   605                 610                 615                 620
   gtg gcg ctg cta cca gac gag atc gcc agc cag cag atc aat cgg gaa    2642
   Val Ala Leu Leu Pro Asp Glu Ile Ala Ser Gln Gln Ile Asn Arg Glu
                   625                 630                 635
   ctg gcg ctg gct aac tac gcc acc atg tcc ggg atc tat gcc cag acg    2690
   Leu Ala Leu Ala Asn Tyr Ala Thr Met Ser Gly Ile Tyr Ala Gln Thr
               640                 645                 650
   gcg gcc ttg atc gaa aac gct gcc gcc atg gga caa gcc ttt gac gcc    2738
   Ala Ala Leu Ile Glu Asn Ala Ala Ala Met Gly Gln Ala Phe Asp Ala
           655                 660                 665
   gcc aag aac gac gac tcc ttc tat ctg ccg ccg gag gct ttt gac aac    2786
   Ala Lys Asn Asp Asp Ser Phe Tyr Leu Pro Pro Glu Ala Phe Asp Asn
       670                 675                 680
   cca gat ttc cag cgc ggc ctg aaa ttg ttc ctg tcg gca gac ggt aag    2834
   Pro Asp Phe Gln Arg Gly Leu Lys Leu Phe Leu Ser Ala Asp Gly Lys
   685                 690                 695                 700
   gcg gct cgg atg atc atc tcc cat gaa ggc gat ccc gcc acc ccc gaa    2882
   Ala Ala Arg Met Ile Ile Ser His Glu Gly Asp Pro Ala Thr Pro Glu
                   705                 710                 715
   ggc att tcg cat atc gac gcg atc aag cag gcg gcc cac gag gcc gtg    2930
   Gly Ile Ser His Ile Asp Ala Ile Lys Gln Ala Ala His Glu Ala Val
               720                 725                 730
   aag ggc act ccc atg gcg ggt gct ggg atc tat ctg gcc ggc acg gcc    2978
   Lys Gly Thr Pro Met Ala Gly Ala Gly Ile Tyr Leu Ala Gly Thr Ala
           735                 740                 745
   gcc acc ttc aag gac att caa gac ggc gcc acc tac gac ctc ctg atc    3026
   Ala Thr Phe Lys Asp Ile Gln Asp Gly Ala Thr Tyr Asp Leu Leu Ile
       750                 755                 760
   gcc gga ata gcc gcg ctg agc ttg att ttg ctc atc atg atg atc att    3074
   Ala Gly Ile Ala Ala Leu Ser Leu Ile Leu Leu Ile Met Met Ile Ile
   765                 770                 775                 780
   acc cga agc ctg gtt gcg gcg ctg gtg atc gtg ggc acg gtg gcg ctg    3122
   Thr Arg Ser Leu Val Ala Ala Leu Val Ile Val Gly Thr Val Ala Leu
                   785                 790                 795
   tcg ttg ggc gct tct ttt ggc ctg tcc gtg ctg gtg tgg cag cat ctt    3170
   Ser Leu Gly Ala Ser Phe Gly Leu Ser Val Leu Val Trp Gln His Leu
               800                 805                 810
   ctc ggt atc cag ttg tac tgg atc gtg ctc gcg ctg gcc gtc atc ctg    3218
   Leu Gly Ile Gln Leu Tyr Trp Ile Val Leu Ala Leu Ala Val Ile Leu
           815                 820                 825
   ctc ctg gcc gtg gga tcg gac tat aac ttg ctg ctg att tcc cga ttc    3266
   Leu Leu Ala Val Gly Ser Asp Tyr Asn Leu Leu Leu Ile Ser Arg Phe
       830                 835                 840
   aag gag gag atc ggt gca ggt ttg aac acc ggc atc atc cgt gcg atg    3314
   Lys Glu Glu Ile Gly Ala Gly Leu Asn Thr Gly Ile Ile Arg Ala Met
   845                 850                 855                 860
   gcc ggc acc ggc ggg gtg gtg acc gct gcc ggc ctg gtg ttc gcc gcc    3362
   Ala Gly Thr Gly Gly Val Val Thr Ala Ala Gly Leu Val Phe Ala Ala
                   865                 870                 875
   act atg tct tcg ttc gtg ttc agt gat ttg cgg gtc ctc ggt cag atc    3410
   Thr Met Ser Ser Phe Val Phe Ser Asp Leu Arg Val Leu Gly Gln Ile
               880                 885                 890
   ggg acc acc att ggt ctt ggg ctg ctg ttc gac acg ctg gtg gtg cgc    3458
   Gly Thr Thr Ile Gly Leu Gly Leu Leu Phe Asp Thr Leu Val Val Arg
           895                 900                 905
   gcg ttc atg acc ccg tcc atc gcg gtg ctg ctc ggg cgc tgg ttc tgg    3506
   Ala Phe Met Thr Pro Ser Ile Ala Val Leu Leu Gly Arg Trp Phe Trp
       910                 915                 920
   tgg ccg caa cga gtg cgc ccg cgc cct gcc agc agg atg ctt cgg ccg    3554
   Trp Pro Gln Arg Val Arg Pro Arg Pro Ala Ser Arg Met Leu Arg Pro
   925                 930                 935                 940
   tac ggc ccg cgg ccc gtg gtt cgt gaa ttg ctg ctg cgc gag ggc aac    3602
   Tyr Gly Pro Arg Pro Val Val Arg Glu Leu Leu Leu Arg Glu Gly Asn
                   945                 950                 955
   gat gac ccg aga act cag gtg gct acc cac cgt taa ggtggtggga         3648
   Asp Asp Pro Arg Thr Gln Val Ala Thr His Arg
               960                 965
   tgccgctttc aggggaatat gcgccgagcc cgctcgactg gtcgcgcgag caagccgaca 3708
   cgtatatgaa gtccggcgga accgagggca cacagctgca gggaaagccg gtcatcctgc 3768
   tcaccaccgt cggggcgaag accggcaaac tccgtaagac cccgctgatg cgcgtcgagc 3828
   acgacggcca gtacgcgatc gtcgcctcgc tgggtggggc gccgaaaaat ccggtctggt 3888
   accacaacgt cgtgaagaac ccacgggtcg agctgcagga cggcaccgga ccggcgacta 3948
   cgacg                                                             3953
<210> 2
   <211> 967
   <212> PRT
   <213> Mycobacterium tuberculosis
<220>
   <223> mmpL6 protein
<400> 2
   Met Ser Asn His His Arg Pro Arg Pro Trp Leu Pro His Thr Ile Arg
    1               5                  10                  15
   Arg Leu Ser Leu Pro Ile Leu Leu Phe Trp Val Gly Val Ala Ala Ile
               20                  25                  30
   Thr Asn Ala Ala Val Pro Gln Leu Glu Val Val Gly Glu Ala His Asn
           35                  40                  45
   Val Ala Gln Ser Ser Pro Asp Asp Pro Ser Leu Gln Ala Met Lys Arg
       50                  55                  60
   Ile Gly Lys Val Phe His Glu Phe Asp Ser Asp Ser Ala Ala Met Ile
   65                  70                  75                  80
   Val Leu Glu Gly Asp Lys Pro Leu Gly Asn Asp Ala His Arg Phe Tyr
                   85                  90                  95
   Asp Thr Leu Leu Arg Asn Leu Ser Asn Asp Thr Lys His Val Glu His
               100                 105                 110
   Val Gln Asp Phe Trp Gly Asp Pro Leu Thr Ala Ala Gly Ser Gln Ser
           115                 120                 125
   Thr Asp Gly Lys Ala Ala Tyr Val Gln Val Tyr Leu Ala Gly Asn Gln
       130                 135                 140
   Gly Glu Ala Leu Ser Ile Glu Ser Val Asp Ala Val Arg Asp Ile Val
   145                 150                 155                 160
   Ala His Thr Pro Pro Pro Ala Gly Val Lys Ala Tyr Val Thr Gly Ala
                   165                 170                 175
   Ala Pro Leu Met Ala Asp Gln Phe Gln Val Gly Ser Lys Gly Thr Ala
               180                 185                 190
   Lys Val Thr Gly Ile Thr Leu Val Val Ile Ala Val Met Leu Leu Phe
           195                 200                 205
   Val Tyr Arg Ser Val Val Thr Met Val Leu Val Leu Ile Thr Val Leu
       210                 215                 220
   Ile Glu Leu Ala Ala Ala Arg Gly Ile Val Ala Phe Leu Gly Asn Ala
   225                 230                 235                 240
   Gly Val Ile Gly Leu Ser Thr Tyr Ser Thr Asn Leu Leu Thr Leu Leu
                   245                 250                 255
   Val Ile Ala Ala Gly Thr Asp Tyr Ala Ile Phe Val Leu Gly Arg Tyr
               260                 265                 270
   His Glu Ala Arg Tyr Ala Ala Gln Asp Arg Glu Thr Ala Phe Tyr Thr
           275                 280                 285
   Met Tyr Arg Gly Thr Ala His Val Val Leu Gly Ser Gly Leu Thr Val
       290                 295                 300
   Ala Gly Ala Val Tyr Cys Leu Ser Phe Thr Arg Leu Pro Tyr Phe Gln
   305                 310                 315                 320
   Ser Leu Gly Ile Pro Ala Ser Ile Gly Val Met Ile Ala Leu Ala Ala
                   325                 330                 335
   Ala Leu Ser Leu Ala Pro Ser Val Leu Ile Leu Gly Ser Arg Phe Gly
               340                 345                 350
   Cys Phe Glu Pro Lys Arg Arg Met Arg Thr Arg Gly Trp Arg Arg Ile
           355                 360                 365
   Gly Thr Ala Ile Val Arg Trp Pro Gly Pro Ile Leu Ala Val Ala Cys
       370                 375                 380
   Ala Ile Ala Val Val Gly Leu Leu Ala Leu Pro Gly Tyr Lys Thr Ser
   385                 390                 395                 400
   Tyr Asp Ala Arg Tyr Tyr Met Pro Ala Thr Ala Pro Ala Asn Ile Gly
                   405                 410                 415
   Tyr Met Ala Ala Glu Arg His Phe Pro Gln Ala Arg Leu Asn Pro Glu
               420                 425                 430
   Leu Leu Met Ile Glu Thr Asp His Asp Met Arg Asn Pro Ala Asp Met
           435                 440                 445
   Leu Ile Leu Asp Arg Ile Ala Lys Ala Val Phe His Leu Pro Gly Ile
       450                 455                 460
   Gly Leu Val Gln Ala Met Thr Arg Pro Leu Gly Thr Pro Ile Asp His
   465                 470                 475                 480
   Ser Ser Ile Pro Phe Gln Ile Ser Met Gln Ser Val Gly Gln Ile Gln
                   485                 490                 495
   Asn Leu Lys Tyr Gln Arg Asp Arg Ala Ala Asp Leu Leu Lys Gln Ala
               500                 505                 510
   Glu Glu Leu Gly Lys Thr Ile Glu Ile Leu Gln Arg Gln Tyr Ala Leu
           515                 520                 525
   Gln Gln Glu Leu Ala Ala Ala Thr His Glu Gln Ala Glu Ser Phe His
       530                 535                 540
   Gln Thr Ile Ala Thr Val Asn Glu Leu Arg Asp Arg Ile Ala Asn Phe
   545                 550                 555                 560
   Asp Asp Phe Phe Arg Pro Ile Arg Ser Tyr Phe Tyr Trp Glu Lys His
                   565                 570                 575
   Cys Tyr Asp Ile Pro Ser Cys Trp Ala Leu Arg Ser Val Phe Asp Thr
               580                 585                 590
   Ile Asp Gly Ile Asp Gln Leu Gly Glu Gln Leu Ala Ser Val Thr Val
           595                 600                 605
   Thr Leu Asp Lys Leu Ala Ala Ile Gln Pro Gln Leu Val Ala Leu Leu
       610                 615                 620
   Pro Asp Glu Ile Ala Ser Gln Gln Ile Asn Arg Glu Leu Ala Leu Ala
   625                 630                 635                 640
   Asn Tyr Ala Thr Met Ser Gly Ile Tyr Ala Gln Thr Ala Ala Leu Ile
                   645                 650                 655
   Glu Asn Ala Ala Ala Met Gly Gln Ala Phe Asp Ala Ala Lys Asn Asp
               660                 665                 670
   Asp Ser Phe Tyr Leu Pro Pro Glu Ala Phe Asp Asn Pro Asp Phe Gln
           675                 680                 685
   Arg Gly Leu Lys Leu Phe Leu Ser Ala Asp Gly Lys Ala Ala Arg Met
       690                 695                 700
   Ile Ile Ser His Glu Gly Asp Pro Ala Thr Pro Glu Gly Ile Ser His
   705                 710                 715                 720
   Ile Asp Ala Ile Lys Gln Ala Ala His Glu Ala Val Lys Gly Thr Pro
                   725                 730                 735
   Met Ala Gly Ala Gly Ile Tyr Leu Ala Gly Thr Ala Ala Thr Phe Lys
               740                 745                 750
   Asp Ile Gln Asp Gly Ala Thr Tyr Asp Leu Leu Ile Ala Gly Ile Ala
           755                 760                 765
   Ala Leu Ser Leu Ile Leu Leu Ile Met Met Ile Ile Thr Arg Ser Leu
       770                 775                 780
   Val Ala Ala Leu Val Ile Val Gly Thr Val Ala Leu Ser Leu Gly Ala
   785                 790                 795                 800
   Ser Phe Gly Leu Ser Val Leu Val Trp Gln His Leu Leu Gly Ile Gln
                   805                 810                 815
   Leu Tyr Trp Ile Val Leu Ala Leu Ala Val Ile Leu Leu Leu Ala Val
               820                 825                 830
   Gly Ser Asp Tyr Asn Leu Leu Leu Ile Ser Arg Phe Lys Glu Glu Ile
           835                 840                 845
   Gly Ala Gly Leu Asn Thr Gly Ile Ile Arg Ala Met Ala Gly Thr Gly
       850                 855                 860
   Gly Val Val Thr Ala Ala Gly Leu Val Phe Ala Ala Thr Met Ser Ser
   865                 870                 875                 880
   Phe Val Phe Ser Asp Leu Arg Val Leu Gly Gln Ile Gly Thr Thr Ile
                   885                 890                 895
   Gly Leu Gly Leu Leu Phe Asp Thr Leu Val Val Arg Ala Phe Met Thr
               900                 905                 910
   Pro Ser Ile Ala Val Leu Leu Gly Arg Trp Phe Trp Trp Pro Gln Arg
           915                 920                 925
   Val Arg Pro Arg Pro Ala Ser Arg Met Leu Arg Pro Tyr Gly Pro Arg
       930                 935                 940
   Pro Val Val Arg Glu Leu Leu Leu Arg Glu Gly Asn Asp Asp Pro Arg
   945                 950                 955                 960
   Thr Gln Val Ala Thr His Arg
                   965
<210> 3
   <211> 148
   <212> PRT
   <213> Mycobacterium tuberculosis
<220>
   <223> mmpS6 protein
<400> 3
   Val Gln Gly Ile Ser Val Thr Gly Leu Val Lys Arg Gly Trp Met Val
    1               5                  10                  15
   Leu Val Ala Val Ala Val Val Ala Val Ala Gly Phe Ser Val Tyr Arg
               20                  25                  30
   Leu His Gly Ile Phe Gly Ser His Asp Thr Thr Ser Thr Ala Gly Gly
           35                  40                  45
   Val Ala Asn Asp Ile Lys Pro Phe Asn Pro Lys Gln Val Thr Leu Glu
       50                  55                  60
   Val Phe Gly Ala Pro Gly Thr Val Ala Thr Ile Asn Tyr Leu Asp Val
   65                  70                  75                  80
   Asp Ala Thr Pro Arg Gln Val Leu Asp Thr Thr Leu Pro Trp Ser Tyr
                   85                  90                  95
   Thr Ile Thr Thr Thr Leu Pro Ala Val Phe Ala Asn Val Val Ala Gln
               100                 105                 110
   Gly Asp Ser Asn Ser Ile Gly Cys Arg Ile Thr Val Asn Gly Val Val
           115                 120                 125
   Lys Asp Glu Arg Ile Val Asn Glu Val Arg Ala Tyr Thr Phe Cys Leu
       130                 135                 140
   Asp Lys Ser Ser
   145
<210> 4
   <211> 2153
   <212> DNA
   <213> Mycobacterium tuberculosis
<210> 5
   <211> 2904
   <212> DNA
   <213> Mycobacterium tuberculosis
<220>
   <223> mmpL6 sequence and protein
<220>
   <221> CDS
   <222> (1)..(2901)
<400> 5
   atg agc aac cac cac cgc ccg cgg cct tgg ttg ccg cac acc atc cga    48
   Met Ser Asn His His Arg Pro Arg Pro Trp Leu Pro His Thr Ile Arg
     1               5                  10                  15
   cgg ctt tcg ttg ccg atc ttg ctg ttt tgg gtg ggt gtg gcc gcc ata    96
   Arg Leu Ser Leu Pro Ile Leu Leu Phe Trp Val Gly Val Ala Ala Ile
                20                  25                  30
   acc aat gcc gcc gtg ccg caa ttg gag gtg gtc ggg gag gcg cat aac   144
   Thr Asn Ala Ala Val Pro Gln Leu Glu Val Val Gly Glu Ala His Asn
            35                  40                  45
   gtc gca cag agc tcc ccg gat gac ccg tcg ctg cag gcg atg aaa cgc   192
   Val Ala Gln Ser Ser Pro Asp Asp Pro Ser Leu Gln Ala Met Lys Arg
           50                  55                  60
   atc ggc aag gtg ttc cac gag ttc gat tcc gac agt gcg gcc atg atc    240
   Ile Gly Lys Val Phe His Glu Phe Asp Ser Asp Ser Ala Ala Met Ile
    65                  70                  75                  80
   gtc ttg gaa ggc gat aag ccg ctc ggc aac gac gcc cac cgg ttc tac    288
   Val Leu Glu Gly Asp Lys Pro Leu Gly Asn Asp Ala His Arg Phe Tyr
                    85                  90                  95
   gac acc ctg ctc cgc aac ctt tca aac gac acc aaa cac gtc gag cac    336
   Asp Thr Leu Leu Arg Asn Leu Ser Asn Asp Thr Lys His Val Glu His
               100                 105                 110
   gtt cag gac ttc tgg ggc gat ccg ctg acc gcg gcc ggc tcg caa agc    384
   Val Gln Asp Phe Trp Gly Asp Pro Leu Thr Ala Ala Gly Ser Gln Ser
           115                 120                 125
   acc gac ggc aaa gcc gcc tac gtt cag gtc tat ctc gcc ggc aac caa    432
   Thr Asp Gly Lys Ala Ala Tyr Val Gln Val Tyr Leu Ala Gly Asn Gln
       130                 135                 140
   ggc gag gcg ttg tca atc gag tcc gtc gac gcg gtg cgc gac atc gtc    480
   Gly Glu Ala Leu Ser Ile Glu Ser Val Asp Ala Val Arg Asp Ile Val
   145                 150                 155                 160
   gcc cat acg cca cca ccg gcc ggg gtc aag gcc tac gtc acc ggc gcg    528
   Ala His Thr Pro Pro Pro Ala Gly Val Lys Ala Tyr Val Thr Gly Ala
                   165                 170                 175
   gcc ccg ctc atg gcc gat cag ttt cag gtg ggc agc aaa gga acc gcg    576
   Ala Pro Leu Met Ala Asp Gln Phe Gln Val Gly Ser Lys Gly Thr Ala
               180                 185                 190
   aaa gtt acc ggg ata act ctg gtt gtg atc gcg gtg atg ttg ctc ttc    624
   Lys Val Thr Gly Ile Thr Leu Val Val Ile Ala Val Met Leu Leu Phe
           195                 200                 205
   gta tac cgt tcc gtc gtc acc atg gtc ctg gtg ctt atc acg gtt ctt    672
   Val Tyr Arg Ser Val Val Thr Met Val Leu Val Leu Ile Thr Val Leu
       210                 215                 220
   att gag ttg gcc gcg gcc cgc ggg atc gtc gct ttt ctc gga aac gcc    720
   Ile Glu Leu Ala Ala Ala Arg Gly Ile Val Ala Phe Leu Gly Asn Ala
   225                 230                 235                 240
   ggg gta atc ggg ctg tcg aca tac tcg acg aat ctg ctc aca cta ttg    768
   Gly Val Ile Gly Leu Ser Thr Tyr Ser Thr Asn Leu Leu Thr Leu Leu
                   245                 250                 255
   gta atc gcg gcg ggc aca gac tac gcg att ttt gtc ctc ggc cgc tat    816
   Val Ile Ala Ala Gly Thr Asp Tyr Ala Ile Phe Val Leu Gly Arg Tyr
               260                 265                 270
   cac gag gcg cgc tac gcc gca cag gat cgg gaa acg gcc ttc tac acg    864
   His Glu Ala Arg Tyr Ala Ala Gln Asp Arg Glu Thr Ala Phe Tyr Thr
           275                 280                 285
   atg tat cgc ggg acc gcc cac gtc gtc ttg ggc tcg ggt ctg acc gtt    912
   Met Tyr Arg Gly Thr Ala His Val Val Leu Gly Ser Gly Leu Thr Val
       290                 295                 300
   gcc ggc gcg gtg tat tgc ctg agc ttt acc cgg cta ccc tat ttt caa     960
   Ala Gly Ala Val Tyr Cys Leu Ser Phe Thr Arg Leu Pro Tyr Phe Gln
   305                 310                 315                 320
   agc ctg ggt att ccc gcc tcg ata ggg gtg atg att gcg ttg gca gcc    1008
   Ser Leu Gly Ile Pro Ala Ser Ile Gly Val Met Ile Ala Leu Ala Ala
                   325                 330                 335
   gcg ctc agc ctg gcc cca tcc gtg ctc atc ttg ggc agt cgt ttc ggt    1056
   Ala Leu Ser Leu Ala Pro Ser Val Leu Ile Leu Gly Ser Arg Phe Gly
               340                 345                 350
   tgt ttc gaa ccc aag cgc agg atg agg acc agg gga tgg cgg cgc atc    1104
   Cys Phe Glu Pro Lys Arg Arg Met Arg Thr Arg Gly Trp Arg Arg Ile
           355                 360                 365
   ggc acg gcc atc gtg cgt tgg ccg gga ccc atc ctg gca gtg gcg tgc    1152
   Gly Thr Ala Ile Val Arg Trp Pro Gly Pro Ile Leu Ala Val Ala Cys
       370                 375                 380
   gca att gcg gtg gtg ggt ctg ctc gcg ctg ccg gga tac aaa acg agc    1200
   Ala Ile Ala Val Val Gly Leu Leu Ala Leu Pro Gly Tyr Lys Thr Ser
   385                 390                 395                 400
   tac gac gct cgc tat tac atg ccc gcc acc gcc ccg gcc aat att ggc    1248
   Tyr Asp Ala Arg Tyr Tyr Met Pro Ala Thr Ala Pro Ala Asn Ile Gly
                   405                 410                 415
   tac atg gcc gcg gag cga cat ttt ccc caa gcg cgg ctg aat ccc gaa    1296
   Tyr Met Ala Ala Glu Arg His Phe Pro Gln Ala Arg Leu Asn Pro Glu
               420                 425                 430
   cta ctg atg atc gag acg gat cac gat atg cgc aat ccg gcc gac atg    1344
   Leu Leu Met Ile Glu Thr Asp His Asp Met Arg Asn Pro Ala Asp Met
           435                 440                 445
   ctc atc ttg gat agg atc gcc aag gct gtc ttc cat ctg ccc ggc ata    1392
   Leu Ile Leu Asp Arg Ile Ala Lys Ala Val Phe His Leu Pro Gly Ile
       450                 455                 460
   ggg ctg gtg cag gcc atg acc cgg ccg cta gga acc ccg att gac cac    1440
   Gly Leu Val Gln Ala Met Thr Arg Pro Leu Gly Thr Pro Ile Asp His
   465                 470                 475                 480
   agc tcg ata ccg ttt cag atc agc atg caa agc gtc ggc cag att cag    1488
   Ser Ser Ile Pro Phe Gln Ile Ser Met Gln Ser Val Gly Gln Ile Gln
                   485                 490                 495
   aat ctc aag tat cag agg gac cga gca gcc gac ttg ctg aag cag gcc    1536
   Asn Leu Lys Tyr Gln Arg Asp Arg Ala Ala Asp Leu Leu Lys Gln Ala
               500                 505                 510
   gaa gag ctg ggg aag acg atc gaa atc ttg cag cgc caa tat gcc cta    1584
   Glu Glu Leu Gly Lys Thr Ile Glu Ile Leu Gln Arg Gln Tyr Ala Leu
           515                 520                 525
   cag cag gaa ctc gcg gcc gct act cac gag caa gcc gaa agc ttt cac    1632
   Gln Gln Glu Leu Ala Ala Ala Thr His Glu Gln Ala Glu Ser Phe His
       530                 535                 540
   caa acg atc gcc acg gta aag gaa ctg cga gat agg atc gcc aat ttc    1680
   Gln Thr Ile Ala Thr Val Lys Glu Leu Arg Asp Arg Ile Ala Asn Phe
   545                 550                 555                 560
   gac gat ttc ttc agg ccg att cgt agt tac ttt tac tgg gaa aag cac    1728
   Asp Asp Phe Phe Arg Pro Ile Arg Ser Tyr Phe Tyr Trp Glu Lys His
                   565                 570                 575
   tgc tac gat atc ccg agc tgc tgg gcg ctg aga tcc gtc ttt gac acg    1776
   Cys Tyr Asp Ile Pro Ser Cys Trp Ala Leu Arg Ser Val Phe Asp Thr
               580                 585                 590
   atc gac ggt atc gac caa ctc ggc gag cag ctg gcc agc gtg acc gta    1824
   Ile Asp Gly Ile Asp Gln Leu Gly Glu Gln Leu Ala Ser Val Thr Val
           595                 600                 605
   acc ttg gac aag ttg gct gcg atc cag cct caa ttg gtg gcg ctg cta    1872
   Thr Leu Asp Lys Leu Ala Ala Ile Gln Pro Gln Leu Val Ala Leu Leu
       610                 615                 620
   cca gac gag atc gcc agc cag cag atc aat cgg gaa ctg gcg ctg gct    1920
   Pro Asp Glu Ile Ala Ser Gln Gln Ile Asn Arg Glu Leu Ala Leu Ala
   625                 630                 635                 640
   aac tac gcc acc atg tcc ggg atc tat gcc cag acg gcg gcc ttg atc    1968
   Asn Tyr Ala Thr Met Ser Gly Ile Tyr Ala Gln Thr Ala Ala Leu Ile
                   645                 650                 655
   gaa aac gct gcc gcc atg gga caa gcc ttt gac gcc gcc aag aac gac    2016
   Glu Asn Ala Ala Ala Met Gly Gln Ala Phe Asp Ala Ala Lys Asn Asp
               660                 665                 670
   gac tcc ttc tat ctg ccg ccg gag gct ttt gac aac cca gat ttc cag    2064
   Asp Ser Phe Tyr Leu Pro Pro Glu Ala Phe Asp Asn Pro Asp Phe Gln
           675                 680                 685
   cgc ggc ctg aaa ttg ttc ctg tcg gca gac ggt aag gcg gct cgg atg    2112
   Arg Gly Leu Lys Leu Phe Leu Ser Ala Asp Gly Lys Ala Ala Arg Met
       690                 695                 700
   atc atc tcc cat gaa ggc gat ccc gcc acc ccc gaa ggc att tcg cat    2160
   Ile Ile Ser His Glu Gly Asp Pro Ala Thr Pro Glu Gly Ile Ser His
   705                 710                 715                 720
   atc gac gcg atc aag cag gcg gcc cac gag gcc gtg aag ggc act ccc    2208
   Ile Asp Ala Ile Lys Gln Ala Ala His Glu Ala Val Lys Gly Thr Pro
                   725                 730                 735
   atg gcg ggt gct ggg atc tat ctg gcc ggc acg gcc gcc acc ttc aag    2256
   Met Ala Gly Ala Gly Ile Tyr Leu Ala Gly Thr Ala Ala Thr Phe Lys
               740                 745                 750
   gac att caa gac ggc gcc acc tac gac ctc ctg atc gcc gga ata gcc    2304
   Asp Ile Gln Asp Gly Ala Thr Tyr Asp Leu Leu Ile Ala Gly Ile Ala
           755                 760                 765
   gcg ctg agc ttg att ttg ctc atc atg atg atc att acc cga agc ctg    2352
   Ala Leu Ser Leu Ile Leu Leu Ile Met Met Ile Ile Thr Arg Ser Leu
       770                 775                 780
   gtt gcg gcg ctg gtg atc gtg ggc acg gtg gcg ctg tcg ttg ggc gct      2400
   Val Ala Ala Leu Val Ile Val Gly Thr Val Ala Leu Ser Leu Gly Ala
   785                 790                 795                 800
   tct ttt ggc ctg tcc gtg ctg gtg tgg cag cat ctt ctc ggt atc cag      2448
   Ser Phe Gly Leu Ser Val Leu Val Trp Gln His Leu Leu Gly Ile Gln
                   805                 810                 815
   ttg tac tgg atc gtg ctc gcg ctg gcc gtc atc ctg ctc ctg gcc gtg      2496
   Leu Tyr Trp Ile Val Leu Ala Leu Ala Val Ile Leu Leu Leu Ala Val
               820                 825                 830
   gga tcg gac tat aac ttg ctg ctg att tcc cga ttc aag gag gag atc      2544
   Gly Ser Asp Tyr Asn Leu Leu Leu Ile Ser Arg Phe Lys Glu Glu Ile
           835                 840                 845
   ggt gca ggt ttg aac acc ggc atc atc cgt gcg atg gcc ggc acc ggc      2592
   Gly Ala Gly Leu Asn Thr Gly Ile Ile Arg Ala Met Ala Gly Thr Gly
       850                 855                 860
   ggg gtg gtg acc gct gcc ggc ctg gtg ttc gcc gcc act atg tct tcg      2640
   Gly Val Val Thr Ala Ala Gly Leu Val Phe Ala Ala Thr Met Ser Ser
   865                 870                 875                 880
   ttc gtg ttc agt gat ttg cgg gtc ctc ggt cag atc ggg acc acc att      2688
   Phe Val Phe Ser Asp Leu Arg Val Leu Gly Gln Ile Gly Thr Thr Ile
                   885                 890                 895
   ggt ctt ggg ctg ctg ttc gac acg ctg gtg gtg cgc gcg ttc atg acc      2736
   Gly Leu Gly Leu Leu Phe Asp Thr Leu Val Val Arg Ala Phe Met Thr
               900                 905                 910
   ccg tcc atc gcg gtg ctg ctc ggg cgc tgg ttc tgg tgg ccg caa cga      2784
   Pro Ser Ile Ala Val Leu Leu Gly Arg Trp Phe Trp Trp Pro Gln Arg
           915                 920                 925
   gtg cgc ccg cgc cct gcc agc agg atg ctt cgg ccg tac ggc ccg cgg      2832
   Val Arg Pro Arg Pro Ala Ser Arg Met Leu Arg Pro Tyr Gly Pro Arg
       930                 935                 940
   ccc gtg gtt cgt gaa ttg ctg ctg cgc gag ggc aac gat gac ccg aga      2880
   Pro Val Val Arg Glu Leu Leu Leu Arg Glu Gly Asn Asp Asp Pro Arg
   945                 950                 955                 960
   act cag gtg gct acc cac cgt taa                                      2904
   Thr Gln Val Ala Thr His Arg
                   965
<210> 6
   <211> 967
<212> PRT
   <213> Mycobacterium tuberculosis
<220>
   <223> mmpL6 sequence and protein
<400> 6
   Met Ser Asn His His Arg Pro Arg Pro Trp Leu Pro His Thr Ile Arg
   1 5 10 15
   Arg Leu Ser Leu Pro Ile Leu Leu Phe Trp Val Gly Val Ala Ala Ile
   20 25 30
   Thr Asn Ala Ala Val Pro Gln Leu Glu Val Val Gly Glu Ala His Asn
   35 40 45
   Val Ala Gln Ser Ser Pro Asp Asp Pro Ser Leu Gln Ala Met Lys Arg
   50 55 60
   Ile Gly Lys Val Phe His Glu Phe Asp Ser Asp Ser Ala Ala Met Ile
   65 70 75 80
   Val Leu Glu Gly Asp Lys Pro Leu Gly Asn Asp Ala His Arg Phe Tyr
   85 90 95
   Asp Thr Leu Leu Arg Asn Leu Ser Asn Asp Thr Lys His Val Glu His
   100 105 110
   Val Gln Asp Phe Trp Gly Asp Pro Leu Thr Ala Ala Gly Ser Gln Ser
   115 120 125
   Thr Asp Gly Lys Ala Ala Tyr Val Gln Val Tyr Leu Ala Gly Asn Gln
   130 135 140
   Gly Glu Ala Leu Ser Ile Glu Ser Val Asp Ala Val Arg Asp Ile Val
   145 150 155 160
   Ala His Thr Pro Pro Pro Ala Gly Val Lys Ala Tyr Val Thr Gly Ala
   165 170 175
   Ala Pro Leu Met Ala Asp Gln Phe Gln Val Gly Ser Lys Gly Thr Ala
   180 185 190
   Lys Val Thr Gly Ile Thr Leu Val Val Ile Ala Val Met Leu Leu Phe
   195 200 205
   Val Tyr Arg Ser Val Val Thr Met Val Leu Val Leu Ile Thr Val Leu
   210 215 220
   Ile Glu Leu Ala Ala Ala Arg Gly Ile Val Ala Phe Leu Gly Asn Ala
   225 230 235 240
   Gly Val Ile Gly Leu Ser Thr Tyr Ser Thr Asn Leu Leu Thr Leu Leu
   245 250 255
   Val Ile Ala Ala Gly Thr Asp Tyr Ala Ile Phe Val Leu Gly Arg Tyr
   260 265 270
   His Glu Ala Arg Tyr Ala Ala Gln Asp Arg Glu Thr Ala Phe Tyr Thr
   275 280 285
   Met Tyr Arg Gly Thr Ala His Val Val Leu Gly Ser Gly Leu Thr Val
   290 295 300
   Ala Gly Ala Val Tyr Cys Leu Ser Phe Thr Arg Leu Pro Tyr Phe Gln
   305 310 315 320
   Ser Leu Gly Ile Pro Ala Ser Ile Gly Val Met Ile Ala Leu Ala Ala
   325 330 335
   Ala Leu Ser Leu Ala Pro Ser Val Leu Ile Leu Gly Ser Arg Phe Gly
   340 345 350
   Cys Phe Glu Pro Lys Arg Arg Met Arg Thr Arg Gly Trp Arg Arg Ile
   355 360 365
   Gly Thr Ala Ile Val Arg Trp Pro Gly Pro Ile Leu Ala Val Ala Cys
   370 375 380
   Ala Ile Ala Val Val Gly Leu Leu Ala Leu Pro Gly Tyr Lys Thr Ser
   385 390 395 400
   Tyr Asp Ala Arg Tyr Tyr Met Pro Ala Thr Ala Pro Ala Asn Ile Gly
   405 410 415
   Tyr Met Ala Ala Glu Arg His Phe Pro Gln Ala Arg Leu Asn Pro Glu
   420 425 430
   Leu Leu Met Ile Glu Thr Asp His Asp Met Arg Asn Pro Ala Asp Met
   435 440 445
   Leu Ile Leu Asp Arg Ile Ala Lys Ala Val Phe His Leu Pro Gly Ile
   450 455 460
   Gly Leu Val Gln Ala Met Thr Arg Pro Leu Gly Thr Pro Ile Asp His
   465 470 475 480
   Ser Ser Ile Pro Phe Gln Ile Ser Met Gln Ser Val Gly Gln Ile Gln
   485 490 495
   Asn Leu Lys Tyr Gln Arg Asp Arg Ala Ala Asp Leu Leu Lys Gln Ala
   500 505 510
   Glu Glu Leu Gly Lys Thr Ile Glu Ile Leu Gln Arg Gln Tyr Ala Leu
   515 520 525
   Gln Gln Glu Leu Ala Ala Ala Thr His Glu Gln Ala Glu Ser Phe His
   530 535 540
   Gln Thr Ile Ala Thr Val Lys Glu Leu Arg Asp Arg Ile Ala Asn Phe
   545 550 555 560
   Asp Asp Phe Phe Arg Pro Ile Arg Ser Tyr Phe Tyr Trp Glu Lys His
   565 570 575
   Cys Tyr Asp Ile Pro Ser Cys Trp Ala Leu Arg Ser Val Phe Asp Thr
   580 585 590
   Ile Asp Gly Ile Asp Gln Leu Gly Glu Gln Leu Ala Ser Val Thr Val
   595 600 605
   Thr Leu Asp Lys Leu Ala Ala Ile Gln Pro Gln Leu Val Ala Leu Leu
   610 615 620
   Pro Asp Glu Ile Ala Ser Gln Gln Ile Asn Arg Glu Leu Ala Leu Ala
   625 630 635 640
   Asn Tyr Ala Thr Met Ser Gly Ile Tyr Ala Gln Thr Ala Ala Leu Ile
   645 650 655
   Glu Asn Ala Ala Ala Met Gly Gln Ala Phe Asp Ala Ala Lys Asn Asp
   660 665 670
   Asp Ser Phe Tyr Leu Pro Pro Glu Ala Phe Asp Asn Pro Asp Phe Gln
   675 680 685
   Arg Gly Leu Lys Leu Phe Leu Ser Ala Asp Gly Lys Ala Ala Arg Met
   690 695 700
   Ile Ile Ser His Glu Gly Asp Pro Ala Thr Pro Glu Gly Ile Ser His
   705 710 715 720
   Ile Asp Ala Ile Lys Gln Ala Ala His Glu Ala Val Lys Gly Thr Pro
   725 730 735
   Met Ala Gly Ala Gly Ile Tyr Leu Ala Gly Thr Ala Ala Thr Phe Lys
   740 745 750
   Asp Ile Gln Asp Gly Ala Thr Tyr Asp Leu Leu Ile Ala Gly Ile Ala
   755 760 765
   Ala Leu Ser Leu Ile Leu Leu Ile Met Met Ile Ile Thr Arg Ser Leu
   770 775 780
   Val Ala Ala Leu Val Ile Val Gly Thr Val Ala Leu Ser Leu Gly Ala
   785 790 795 800
   Ser Phe Gly Leu Ser Val Leu Val Trp Gln His Leu Leu Gly Ile Gln
   805 810 815
   Leu Tyr Trp Ile Val Leu Ala Leu Ala Val Ile Leu Leu Leu Ala Val
   820 825 830
   Gly Ser Asp Tyr Asn Leu Leu Leu Ile Ser Arg Phe Lys Glu Glu Ile
   835 840 845
   Gly Ala Gly Leu Asn Thr Gly Ile Ile Arg Ala Met Ala Gly Thr Gly
   850 855 860
   Gly Val Val Thr Ala Ala Gly Leu Val Phe Ala Ala Thr Met Ser Ser
   865 870 875 880
   Phe Val Phe Ser Asp Leu Arg Val Leu Gly Gln Ile Gly Thr Thr Ile
   885 890 895
   Gly Leu Gly Leu Leu Phe Asp Thr Leu Val Val Arg Ala Phe Met Thr
   900 905 910
   Pro Ser Ile Ala Val Leu Leu Gly Arg Trp Phe Trp Trp Pro Gln Arg
   915 920 925
   Val Arg Pro Arg Pro Ala Ser Arg Met Leu Arg Pro Tyr Gly Pro Arg
   930 935 940
   Pro Val Val Arg Glu Leu Leu Leu Arg Glu Gly Asn Asp Asp Pro Arg
   945 950 955 960
   Thr Gln Val Ala Thr His Arg
   965
<210> 7
   <211> 1758
   <212> DNA
   <213> Mycobacterium tuberculosis
<220>
   <221> CDS
   <222> (1)..(1758)
<220>
   <223> mmpL6 truncated sequence and protein
<400> 7
   atg agc aac cac cac cgc ccg cgg cct tgg ttg ccg cac acc atc cga    48
   Met Ser Asn His His Arg Pro Arg Pro Trp Leu Pro His Thr Ile Arg
    1               5                  10                  15
   cgg ctt tcg ttg ccg atc ttg ctg ttt tgg gtg ggt gtg gcc gcc ata    96
   Arg Leu Ser Leu Pro Ile Leu Leu Phe Trp Val Gly Val Ala Ala Ile
               20                  25                  30
   acc aat gcc gcc gtg ccg caa ttg gag gtg gtc ggg gag gcg cat aac   144
   Thr Asn Ala Ala Val Pro Gln Leu Glu Val Val Gly Glu Ala His Asn
           35                  40                  45
   gtc gca cag agc tcc ccg gat gac ccg tcg ctg cag gcg atg aaa cgc   192
   Val Ala Gln Ser Ser Pro Asp Asp Pro Ser Leu Gln Ala Met Lys Arg
       50                  55                  60
   atc ggc aag gtg ttc cac gag ttc gat tcc gac agt gcg gcc atg atc   240
   Ile Gly Lys Val Phe His Glu Phe Asp Ser Asp Ser Ala Ala Met Ile
   65                  70                  75                  80
   gtc ttg gaa ggc gat aag ccg ctc ggc aac gac gcc cac cgg ttc tac   288
   Val Leu Glu Gly Asp Lys Pro Leu Gly Asn Asp Ala His Arg Phe Tyr
                   85                  90                  95
   gac acc ctg ctc cgc aac ctt tca aac gac acc aaa cac gtc gag cac   336
   Asp Thr Leu Leu Arg Asn Leu Ser Asn Asp Thr Lys His Val Glu His
               100                 105                 110
   gtt cag gac ttc tgg ggc gat ccg ctg acc gcg gcc ggc tcg caa agc   384
   Val Gln Asp Phe Trp Gly Asp Pro Leu Thr Ala Ala Gly Ser Gln Ser
           115                 120                 125
   acc gac ggc aaa gcc gcc tac gtt cag gtc tat ctc gcc ggc aac caa   432
   Thr Asp Gly Lys Ala Ala Tyr Val Gln Val Tyr Leu Ala Gly Asn Gln
       130                 135                 140
   ggc gag gcg ttg tca atc gag tcc gtc gac gcg gtg cgc gac atc gtc   480
   Gly Glu Ala Leu Ser Ile Glu Ser Val Asp Ala Val Arg Asp Ile Val
   145                 150                 155                 160
   gcc cat acg cca cca ccg gcc ggg gtc aag gcc tac gtc acc ggc gcg   528
   Ala His Thr Pro Pro Pro Ala Gly Val Lys Ala Tyr Val Thr Gly Ala
                   165                 170                 175
   gcc ccg ctc atg gcc gat cag ttt cag gtg ggc agc aaa gga acc gcg   576
   Ala Pro Leu Met Ala Asp Gln Phe Gln Val Gly Ser Lys Gly Thr Ala
               180                 185                 190
   aaa gtt acc ggg ata act ctg gtt gtg atc gcg gtg atg ttg ctc ttc     624
   Lys Val Thr Gly Ile Thr Leu Val Val Ile Ala Val Met Leu Leu Phe
           195                 200                 205
   gta tac cgt tcc gtc gtc acc atg gtc ctg gtg ctt atc acg gtt ctt     672
   Val Tyr Arg Ser Val Val Thr Met Val Leu Val Leu Ile Thr Val Leu
       210                 215                 220
   att gag ttg gcc gcg gcc cgc ggg atc gtc gct ttt ctc gga aac gcc     720
   Ile Glu Leu Ala Ala Ala Arg Gly Ile Val Ala Phe Leu Gly Asn Ala
   225                 230                 235                 240
   ggg gta atc ggg ctg tcg aca tac tcg acg aat ctg ctc aca cta ttg     768
   Gly Val Ile Gly Leu Ser Thr Tyr Ser Thr Asn Leu Leu Thr Leu Leu
                   245                 250                 255
   gta atc gcg gcg ggc aca gac tac gcg att ttt gtc ctc ggc cgc tat     816
   Val Ile Ala Ala Gly Thr Asp Tyr Ala Ile Phe Val Leu Gly Arg Tyr
               260                 265                 270
   cac gag gcg cgc tac gcc gca cag gat cgg gaa acg gcc ttc tac acg     864
   His Glu Ala Arg Tyr Ala Ala Gln Asp Arg Glu Thr Ala Phe Tyr Thr
           275                 280                 285
   atg tat cgc ggg acc gcc cac gtc gtc ttg ggc tcg ggt ctg acc gtt     912
   Met Tyr Arg Gly Thr Ala His Val Val Leu Gly Ser Gly Leu Thr Val
       290                 295                 300
   gcc ggc gcg gtg tat tgc ctg agc ttt acc cgg cta ccc tat ttt caa     960
   Ala Gly Ala Val Tyr Cys Leu Ser Phe Thr Arg Leu Pro Tyr Phe Gln
   305                 310                 315                 320
   agc ctg ggt att ccc gcc tcg ata ggg gtg atg att gcg ttg gca gcc     1008
   Ser Leu Gly Ile Pro Ala Ser Ile Gly Val Met Ile Ala Leu Ala Ala
                   325                 330                 335
   gcg ctc agc ctg gcc cca tcc gtg ctc atc ttg ggc agt cgt ttc ggt     1056
   Ala Leu Ser Leu Ala Pro Ser Val Leu Ile Leu Gly Ser Arg Phe Gly
               340                 345                 350
   tgt ttc gaa ccc aag cgc agg atg agg acc agg gga tgg cgg cgc atc     1104
   Cys Phe Glu Pro Lys Arg Arg Met Arg Thr Arg Gly Trp Arg Arg Ile
           355                 360                 365
   ggc acg gcc atc gtg cgt tgg ccg gga ccc atc ctg gca gtg gcg tgc     1152
   Gly Thr Ala Ile Val Arg Trp Pro Gly Pro Ile Leu Ala Val Ala Cys
       370                 375                 380
   gca att gcg gtg gtg ggt ctg ctc gcg ctg ccg gga tac aaa acg agc     1200
   Ala Ile Ala Val Val Gly Leu Leu Ala Leu Pro Gly Tyr Lys Thr Ser
   385                 390                 395                 400
   tac gac gct cgc tat tac atg ccc gcc acc gcc ccg gcc aat att ggc     1248
   Tyr Asp Ala Arg Tyr Tyr Met Pro Ala Thr Ala Pro Ala Asn Ile Gly
                   405                 410                 415
   tac atg gcc gcg gag cga cat ttt ccc caa gcg cgg ctg aat ccc gaa     1296
   Tyr Met Ala Ala Glu Arg His Phe Pro Gln Ala Arg Leu Asn Pro Glu
               420                     425                     430
   cta ctg atg atc gag acg gat cac gat atg cgc aat ccg gcc gac atg     1344
   Leu Leu Met Ile Glu Thr Asp His Asp Met Arg Asn Pro Ala Asp Met
           435                     440                     445
   ctc atc ttg gat agg atc gcc aag gct gtc ttc cat ctg ccc ggc ata     1392
   Leu Ile Leu Asp Arg Ile Ala Lys Ala Val Phe His Leu Pro Gly Ile
       450                     455                     460
   ggg ctg gtg cag gcc atg acc cgg ccg cta gga acc ccg att gac cac     1440
   Gly Leu Val Gln Ala Met Thr Arg Pro Leu Gly Thr Pro Ile Asp His
   465                     470                     475                     480
   agc tcg ata ccg ttt cag atc agc atg caa agc gtc ggc cag att cag     1488
   Ser Ser Ile Pro Phe Gln Ile Ser Met Gln Ser Val Gly Gln Ile Gln
                   485                     490                     495
   aat ctc aag tat cag agg gac cga gca gcc gac ttg ctg aag cag gcc     1536
   Asn Leu Lys Tyr Gln Arg Asp Arg Ala Ala Asp Leu Leu Lys Gln Ala
               500                     505                     510
   gaa gag ctg ggg aag acg atc gaa atc ttg cag cgc caa tat gcc cta     1584
   Glu Glu Leu Gly Lys Thr Ile Glu Ile Leu Gln Arg Gln Tyr Ala Leu
           515                     520                     525
   cag cag gaa ctc gcg gcc gct act cac gag caa gcc gaa agc ttt cac     1632
   Gln Gln Glu Leu Ala Ala Ala Thr His Glu Gln Ala Glu Ser Phe His
       530                     535                     540
   caa acg atc gcc acg gta aag gaa ctg cga gat agg atc gcc aat ttc     1680
   Gln Thr Ile Ala Thr Val Lys Glu Leu Arg Asp Arg Ile Ala Asn Phe
   545                     550                     555                     560
   gac gat ttc ttc agg ccg att cgt agt tac ttt tac tgg gaa aag cac     1728
   Asp Asp Phe Phe Arg Pro Ile Arg Ser Tyr Phe Tyr Trp Glu Lys His
                   565                     570                     575
   tgc tac gat atc ccg agc tgc tgg gcg ctg                             1758
   Cys Tyr Asp Ile Pro Ser Cys Trp Ala Leu
               580                     585
<210> 8
   <211> 586
   <212> PRT
   <213> Mycobacterium tuberculosis
<220>
   <223> mmpL6 truncated sequence and protein
<400> 8
   Met Ser Asn His His Arg Pro Arg Pro Trp Leu Pro His Thr Ile Arg
    1               5                  10                  15
   Arg Leu Ser Leu Pro Ile Leu Leu Phe Trp Val Gly Val Ala Ala Ile
               20                  25                  30
   Thr Asn Ala Ala Val Pro Gln Leu Glu Val Val Gly Glu Ala His Asn
           35                  40                  45
   Val Ala Gln Ser Ser Pro Asp Asp Pro Ser Leu Gln Ala Met Lys Arg
       50                  55                  60
   Ile Gly Lys Val Phe His Glu Phe Asp Ser Asp Ser Ala Ala Met Ile
   65                  70                  75                  80
   Val Leu Glu Gly Asp Lys Pro Leu Gly Asn Asp Ala His Arg Phe Tyr
                   85                  90                  95
   Asp Thr Leu Leu Arg Asn Leu Ser Asn Asp Thr Lys His Val Glu His
               100                 105                 110
   Val Gln Asp Phe Trp Gly Asp Pro Leu Thr Ala Ala Gly Ser Gln Ser
           115                 120                 125
   Thr Asp Gly Lys Ala Ala Tyr Val Gln Val Tyr Leu Ala Gly Asn Gln
       130                 135                 140
   Gly Glu Ala Leu Ser Ile Glu Ser Val Asp Ala Val Arg Asp Ile Val
   145                 150                 155                 160
   Ala His Thr Pro Pro Pro Ala Gly Val Lys Ala Tyr Val Thr Gly Ala
                   165                 170                 175
   Ala Pro Leu Met Ala Asp Gln Phe Gln Val Gly Ser Lys Gly Thr Ala
               180                 185                 190
   Lys Val Thr Gly Ile Thr Leu Val Val Ile Ala Val Met Leu Leu Phe
           195                 200                 205
   Val Tyr Arg Ser Val Val Thr Met Val Leu Val Leu Ile Thr Val Leu
       210                 215                 220
   Ile Glu Leu Ala Ala Ala Arg Gly Ile Val Ala Phe Leu Gly Asn Ala
   225                 230                 235                 240
   Gly Val Ile Gly Leu Ser Thr Tyr Ser Thr Asn Leu Leu Thr Leu Leu
                   245                 250                 255
   Val Ile Ala Ala Gly Thr Asp Tyr Ala Ile Phe Val Leu Gly Arg Tyr
               260                 265                 270
   His Glu Ala Arg Tyr Ala Ala Gln Asp Arg Glu Thr Ala Phe Tyr Thr
           275                 280                 285
   Met Tyr Arg Gly Thr Ala His Val Val Leu Gly Ser Gly Leu Thr Val
       290                 295                 300
   Ala Gly Ala Val Tyr Cys Leu Ser Phe Thr Arg Leu Pro Tyr Phe Gln
   305                 310                 315                 320
   Ser Leu Gly Ile Pro Ala Ser Ile Gly Val Met Ile Ala Leu Ala Ala
                   325                 330                 335
   Ala Leu Ser Leu Ala Pro Ser Val Leu Ile Leu Gly Ser Arg Phe Gly
               340                 345                 350
   Cys Phe Glu Pro Lys Arg Arg Met Arg Thr Arg Gly Trp Arg Arg Ile
           355                 360                 365
   Gly Thr Ala Ile Val Arg Trp Pro Gly Pro Ile Leu Ala Val Ala Cys
   370 375 380
   Ala Ile Ala Val Val Gly Leu Leu Ala Leu Pro Gly Tyr Lys Thr Ser
   385 390 395 400
   Tyr Asp Ala Arg Tyr Tyr Met Pro Ala Thr Ala Pro Ala Asn Ile Gly
   405 410 415
   Tyr Met Ala Ala Glu Arg His Phe Pro Gln Ala Arg Leu Asn Pro Glu
   420 425 430
   Leu Leu Met Ile Glu Thr Asp His Asp Met Arg Asn Pro Ala Asp Met
   435 440 445
   Leu Ile Leu Asp Arg Ile Ala Lys Ala Val Phe His Leu Pro Gly Ile
   450 455 460
   Gly Leu Val Gln Ala Met Thr Arg Pro Leu Gly Thr Pro Ile Asp His
   465 470 475 480
   Ser Ser Ile Pro Phe Gln Ile Ser Met Gln Ser Val Gly Gln Ile Gln
   485 490 495
   Asn Leu Lys Tyr Gln Arg Asp Arg Ala Ala Asp Leu Leu Lys Gln Ala
   500 505 510
   Glu Glu Leu Gly Lys Thr Ile Glu Ile Leu Gln Arg Gln Tyr Ala Leu
   515 520 525
   Gln Gln Glu Leu Ala Ala Ala Thr His Glu Gln Ala Glu Ser Phe His
   530 535 540
   Gln Thr Ile Ala Thr Val Lys Glu Leu Arg Asp Arg Ile Ala Asn Phe
   545 550 555 560
   Asp Asp Phe Phe Arg Pro Ile Arg Ser Tyr Phe Tyr Trp Glu Lys His
   565 570 575
   Cys Tyr Asp Ile Pro Ser Cys Trp Ala Leu
   580 585
<210> 9
   <211> 447
   <212> DNA
   <213> Mycobacterium tuberculosis
<220>
   <221> CDS
   <222> (1) .. (444)
<220>
   <223> mmpS6 sequence and protein
<400> 9
   gtg cag ggg att tca gtg act ggc ctg gtc aaa cgc ggc tgg atg gtg     48
   Val Gln Gly Ile Ser Val Thr Gly Leu Val Lys Arg Gly Trp Met Val
   1               5                   10                  15
   ctg gtt gcc gtg gcg gtg gtg gcg gtc gcg gga ttc agc gtc tat cgg     96
   Leu Val Ala Val Ala Val Val Ala Val Ala Gly Phe Ser Val Tyr Arg
               20                  25                  30
   ttg cac ggc atc ttc ggc tcg cac gac acc acc tcg acc gcc ggt ggt    144
   Leu His Gly Ile Phe Gly Ser His Asp Thr Thr Ser Thr Ala Gly Gly
           35                  40                  45
   gtc gcg aac gac atc aag ccg ttc aac ccc aaa cag gta acc ctc gag    192
   Val Ala Asn Asp Ile Lys Pro Phe Asn Pro Lys Gln Val Thr Leu Glu
       50                  55                  60
   gtc ttt ggc gct ccc gga acc gtg gca acg atc aat tat ctg gac gtg    240
   Val Phe Gly Ala Pro Gly Thr Val Ala Thr Ile Asn Tyr Leu Asp Val
   65                  70                  75                  80
   gat gcc aca cct cgg caa gtc ctg gac acg acc ctg ccg tgg tca tac    288
   Asp Ala Thr Pro Arg Gln Val Leu Asp Thr Thr Leu Pro Trp Ser Tyr
                   85                  90                  95
   acg atc acg acg acc ctg ccc gcg gtc ttc gcc aat gtt gtc gcg caa    336
   Thr Ile Thr Thr Thr Leu Pro Ala Val Phe Ala Asn Val Val Ala Gln
               100                 105                 110
   ggc gac agc aat tcc atc ggc tgc cgc atc acc gtc aac ggt gta gtc    384
   Gly Asp Ser Asn Ser Ile Gly Cys Arg Ile Thr Val Asn Gly Val Val
           115                 120                 125
   aag gac gaa agg atc gtc aac gaa gtg cgc gcc tat acc ttc tgc ctc    432
   Lys Asp Glu Arg Ile Val Asn Glu Val Arg Ala Tyr Thr Phe Cys Leu
       130                 135                 140
   gac aag tcc tca tga                                                447
   Asp Lys Ser Ser
   145
<210> 10
   <211> 148
   <212> PRT
   <213> Mycobacterium tuberculosis
<220>
   <223> mmpS6 sequence and protein
<400> 10
   Val Gln Gly Ile Ser Val Thr Gly Leu Val Lys Arg Gly Trp Met Val
   1               5                   10                  15
   Leu Val Ala Val Ala Val Val Ala Val Ala Gly Phe Ser Val Tyr Arg
               20                  25                  30
   Leu His Gly Ile Phe Gly Ser His Asp Thr Thr Ser Thr Ala Gly Gly
           35                  40                  45
   Val Ala Asn Asp Ile Lys Pro Phe Asn Pro Lys Gln Val Thr Leu Glu
       50                  55                  60
   Val Phe Gly Ala Pro Gly Thr Val Ala Thr Ile Asn Tyr Leu Asp Val
   65                  70                  75                  80
   Asp Ala Thr Pro Arg Gln Val Leu Asp Thr Thr Leu Pro Trp Ser Tyr
                   85                  90                  95
   Thr Ile Thr Thr Thr Leu Pro Ala Val Phe Ala Asn Val Val Ala Gln
               100                 105                 110
   Gly Asp Ser Asn Ser Ile Gly Cys Arg Ile Thr Val Asn Gly Val Val
           115                 120                 125
   Lys Asp Glu Arg Ile Val Asn Glu Val Arg Ala Tyr Thr Phe Cys Leu
       130                 135                 140
   Asp Lys Ser Ser
   145
<210> 11
   <211> 399
   <212> DNA
   <213> Mycobacterium tuberculosis
<220>
   <221> CDS
   <222> (1) .. (399)
<220>
   <223> mmpS6 truncated sequence and protein
<400> 11
   ctg gtt gcc gtg gcg gtg gtg gcg gtc gcg gga ttc agc gtc tat cgg    48
   Leu Val Ala Val Ala Val Val Ala Val Ala Gly Phe Ser Val Tyr Arg
    1               5                  10                  15
   ttg cac ggc atc ttc ggc tcg cac gac acc acc tcg acc gcc ggt ggt    96
   Leu His Gly Ile Phe Gly Ser His Asp Thr Thr Ser Thr Ala Gly Gly
               20                  25                  30
   gtc gcg aac gac atc aag ccg ttc aac ccc aaa cag gta acc ctc gag    144
   Val Ala Asn Asp Ile Lys Pro Phe Asn Pro Lys Gln Val Thr Leu Glu
           35                  40                  45
   gtc ttt ggc gct ccc gga acc gtg gca acg atc aat tat ctg gac gtg    192
   Val Phe Gly Ala Pro Gly Thr Val Ala Thr Ile Asn Tyr Leu Asp Val
       50                  55                  60
   gat gcc aca cct cgg caa gtc ctg gac acg acc ctg ccg tgg tca tac    240
   Asp Ala Thr Pro Arg Gln Val Leu Asp Thr Thr Leu Pro Trp Ser Tyr
   65                  70                  75                  80
   acg atc acg acg acc ctg ccc gcg gtc ttc gcc aat gtt gtc gcg caa    288
   Thr Ile Thr Thr Thr Leu Pro Ala Val Phe Ala Asn Val Val Ala Gln
                   85                  90                  95
   ggc gac agc aat tcc atc ggc tgc cgc atc acc gtc aac ggt gta gtc    336
   Gly Asp Ser Asn Ser Ile Gly Cys Arg Ile Thr Val Asn Gly Val Val
               100                 105                 110
   aag gac gaa agg atc gtc aac gaa gtg cgc gcc tat acc ttc tgc ctc    384
   Lys Asp Glu Arg Ile Val Asn Glu Val Arg Ala Tyr Thr Phe Cys Leu
           115                 120                 125
   gac aag tcc tca tga                                                399
   Asp Lys Ser Ser
       130
<210> 12
   <211> 132
   <212> PRT
   <213> Mycobacterium tuberculosis
<220>
   <223> mmpS6 truncated sequence and protein
<400> 12
   Leu Val Ala Val Ala Val Val Ala Val Ala Gly Phe Ser Val Tyr Arg
    1               5                  10                  15
   Leu His Gly Ile Phe Gly Ser His Asp Thr Thr Ser Thr Ala Gly Gly
               20                  25                  30
   Val Ala Asn Asp Ile Lys Pro Phe Asn Pro Lys Gln Val Thr Leu Glu
           35                  40                  45
   Val Phe Gly Ala Pro Gly Thr Val Ala Thr Ile Asn Tyr Leu Asp Val
       50                  55                  60
   Asp Ala Thr Pro Arg Gln Val Leu Asp Thr Thr Leu Pro Trp Ser Tyr
   65                  70                  75                  80
   Thr Ile Thr Thr Thr Leu Pro Ala Val Phe Ala Asn Val Val Ala Gln
                   85                  90                  95
   Gly Asp Ser Asn Ser Ile Gly Cys Arg Ile Thr Val Asn Gly Val Val
               100                 105                 110
   Lys Asp Glu Arg Ile Val Asn Glu Val Arg Ala Tyr Thr Phe Cys Leu
           115                 120                 125
   Asp Lys Ser Ser
       130
<210> 13
   <211> 20
   <212> DNA
   <213> Mycobacterium tuberculosis
<400> 13
   cgttcaaccc caaacaggta 20
<210> 14
   <211> 20
   <212> DNA
   <213> Mycobacterium tuberculosis
<400> 14
   aatcgaactc gtggaacacc 20
<210> 15
   <211> 20
   <212> DNA
   <213> Mycobacterium tuberculosis
<400> 15
   attcagcgtc tatcggttgc 20
<210> 16
   <211> 20
   <212> DNA
   <213> Mycobacterium tuberculosis
<400> 16
   agcagctcgg gatatcgtag 20
<210> 17
   <211> 20
   <212> DNA
   <213> Mycobacterium tuberculosis
<400> 17
   ctacctcatc ttccggtcca 20
<210> 18
   <211> 20
   <212> DNA
   <213> Mycobacterium tuberculosis
<400> 18
   catagatccc ggacatggtg 20
<210> 19
   <211> 2390
   <212> DNA
   <213> Mycobacterium tuberculosis
<220>
   <221> CDS
   <222> (517)..(2307)
<400> 19
   gatcccgtcg ccgcggcgct ggagctggcc gccgggcccg cagccgcccc gcgcgaggtc 60
   gtgctggcga gcaaagccac catgcgcgcc acagccagcc ccggatcgct ggaccttgag 120
   caacacgaac tcgccaaacg cttagaactt gggccgcagg cgaaatcggt ccagtcgccc 180
   gagttcgccg ctcgcttggc tgccgctcaa cacaggtagc gcctaccagc ctcgctggtt 240
   tccatggcgt gccccagtcc gaagctgctg ctgcttgact ccgcgcgctg ggcccgagcg 300
   cgcgctgttg tacggcccaa acggcgtgtc ggtgtacagt cgcgcgctcg cggcttcagt 360
   ccggcccccc gactccggca ggcccgacgg cgcccagcgc tagcccgaag ttcccccttg 420
   taggggcggg ctgagtttcg atctgtttcg tgagcaggtg tttctgtgtt caacttccct 480
   caacatgtac tcatgtatta ttgagaatag ctcggc gtg tca tcc tct gat gac    534
                                           Val Ser Ser Ser Asp Asp
                                            1               5
   gct att atc gcg ctg acc gcg tgt tat aaa gta atc atg tac att acc    582
   Ala Ile Ile Ala Leu Thr Ala Cys Tyr Lys Val Ile Met Tyr Ile Thr
               10                  15                  20
   cgg gta ccc aac cgg gga tcc ccg ccg gcg gtg ctg ttg cgg gaa agc    630
   Arg Val Pro Asn Arg Gly Ser Pro Pro Ala Val Leu Leu Arg Glu Ser
           25                  30                  35
   ttc cgc gaa aac ggc aag gtc aag acg cgt acc ctg gcc aac ctc tca    678
   Phe Arg Glu Asn Gly Lys Val Lys Thr Arg Thr Leu Ala Asn Leu Ser
       40                  45                  50
   cgc tgg ccc gag cac aag ctg gac aga ctg gac cgg gcg ctt aag ggc    726
   Arg Trp Pro Glu His Lys Leu Asp Arg Leu Asp Arg Ala Leu Lys Gly
    55                  60                  65                  70
   ttg ccg ccc gcg gac tgg gat cta gcc gag gcc ttc gat atc acc cgc    774
   Leu Pro Pro Ala Asp Trp Asp Leu Ala Glu Ala Phe Asp Ile Thr Arg
                   75                  80                  85
   agc ctg ccg cac ggg cat gtg gcc gcg gtg gcc ggc acc gcc gag aag    822
   Ser Leu Pro His Gly His Val Ala Ala Val Ala Gly Thr Ala Glu Lys
               90                  95                  100
   ctg ggc ata ccc gag ctg atc gac ccc acc ccg tcg cgg cgg cgc aac    870
   Leu Gly Ile Pro Glu Leu Ile Asp Pro Thr Pro Ser Arg Arg Arg Asn
           105                 110                 115
   ctg gtg ctg gcc atg ctg atc ggg cag atc atc gag ccc gga tcg aaa    918
   Leu Val Leu Ala Met Leu Ile Gly Gln Ile Ile Glu Pro Gly Ser Lys
       120                 125                 130
   ctg gcg atc gcg cgc ggg ctg cgc gcc cag acc gcc acc agc acg ctg    966
   Leu Ala Ile Ala Arg Gly Leu Arg Ala Gln Thr Ala Thr Ser Thr Leu
   135                 140                 145                 150
   ggt gcg gtg ctg ggt gtc tcg ggc gcc gat gag gac gac ctg tat gac    1014
   Gly Ala Val Leu Gly Val Ser Gly Ala Asp Glu Asp Asp Leu Tyr Asp
                   155                 160                 165
   gcg atg gac tgg gcg ctg gag cgc aaa gac ggc atc gaa aac gcc ttg    1062
   Ala Met Asp Trp Ala Leu Glu Arg Lys Asp Gly Ile Glu Asn Ala Leu
               170                 175                 180
   gcc gca cgg cat ctg acc aac ggc acc ctg gtg ctc tat gac gta tcc    1110
   Ala Ala Arg His Leu Thr Asn Gly Thr Leu Val Leu Tyr Asp Val Ser
           185                 190                 195
   tcg gcg gcg ttc gag ggc cac acc tgc ccg ctg gga gcg atc ggg cac    1158
   Ser Ala Ala Phe Glu Gly His Thr Cys Pro Leu Gly Ala Ile Gly His
       200                 205                 210
   gcc cgc gac ggg gtc aaa ggc cgg ctg cag atc gtc tac ggg ctg ctg    1206
   Ala Arg Asp Gly Val Lys Gly Arg Leu Gln Ile Val Tyr Gly Leu Leu
   215                 220                 225                 230
   tgc tca ccc aag gga gcg ccg gtg gcc atc gag gtg ttc aag ggc aac    1254
   Cys Ser Pro Lys Gly Ala Pro Val Ala Ile Glu Val Phe Lys Gly Asn
                   235                 240                 245
   acc gcc gac ccg aaa act ctg aaa gct caa atc gac aag ctc aaa acc    1302
   Thr Ala Asp Pro Lys Thr Leu Lys Ala Gln Ile Asp Lys Leu Lys Thr
               250                 255                 260
   cgg ttc ggg ttg acc cgc atc gcc ctg gtg ggc gat cgg ggc atg ctc    1350
   Arg Phe Gly Leu Thr Arg Ile Ala Leu Val Gly Asp Arg Gly Met Leu
           265                 270                 275
   act tcc gcg cgc atc cgt gac gag ctg cgt ccg gcg cac ctg gat tgg    1398
   Thr Ser Ala Arg Ile Arg Asp Glu Leu Arg Pro Ala His Leu Asp Trp
       280                 285                 290
   atc agc gcg ctg cgc gcc ccg cag atc aag atc ctg ctc gag gac ggg    1446
   Ile Ser Ala Leu Arg Ala Pro Gln Ile Lys Ile Leu Leu Glu Asp Gly
   295                 300                 305                 310
   gcg ctg cag ctg tcg ctg ttc gat gag cag aac ctg ttc gag atc act    1494
   Ala Leu Gln Leu Ser Leu Phe Asp Glu Gln Asn Leu Phe Glu Ile Thr
                   315                 320                 325
   cac ccc gac tat ccc ggt gag cgg ctg gtg tgc tgc cac aac ccc gcc    1542
   His Pro Asp Tyr Pro Gly Glu Arg Leu Val Cys Cys His Asn Pro Ala
               330                 335                 340
   ctg gcc gac gag cgc gcc cgc aaa cgc gcc gag ctg ctg gcg gcc acc    1590
   Leu Ala Asp Glu Arg Ala Arg Lys Arg Ala Glu Leu Leu Ala Ala Thr
           345                 350                 355
   gaa aag gag ctg cag gcc atc gcc gaa gcc acc cgc cgc caa cgc cgg    1638
   Glu Lys Glu Leu Gln Ala Ile Ala Glu Ala Thr Arg Arg Gln Arg Arg
       360                 365                 370
   ccg tta cgc ggt aca gac aag atc ggc ctg cgg gtg ggc aag gtg cgc    1686
   Pro Leu Arg Gly Thr Asp Lys Ile Gly Leu Arg Val Gly Lys Val Arg
   375                 380                 385                 390
   aac aag ttc aag atg gcc aag cac ttt gac ctg cac atc acc gat gag    1734
   Asn Lys Phe Lys Met Ala Lys His Phe Asp Leu His Ile Thr Asp Glu
                   395                 400                 405
   gcc ttc agc ttc acc cgc aac cag aac agt atc gcc gcc gag gcc gcc    1782
   Ala Phe Ser Phe Thr Arg Asn Gln Asn Ser Ile Ala Ala Glu Ala Ala
               410                 415                 420
   ctc gac ggc atc tac gtg cta cgc acc agc ctg ccc gac aac gcc ctg    1830
   Leu Asp Gly Ile Tyr Val Leu Arg Thr Ser Leu Pro Asp Asn Ala Leu
           425                 430                 435
   ggc cgc gac gac gtg gtg ggc cgc tac aaa gac ctc gcc gac gtc gaa    1878
   Gly Arg Asp Asp Val Val Gly Arg Tyr Lys Asp Leu Ala Asp Val Glu
       440                 445                 450
   cgc ttc ttc cgc acc ctc aac agc gaa ctg gac gta cgc ccc atc cgg    1926
   Arg Phe Phe Arg Thr Leu Asn Ser Glu Leu Asp Val Arg Pro Ile Arg
   455                 460                 465                 470
   cat cgg ctg gcc gac cgg gtc cgc gcc cac atg ttc ttg cac atg ctc    1974
   His Arg Leu Ala Asp Arg Val Arg Ala His Met Phe Leu His Met Leu
                   475                 480                 485
   tcc tac tac atc agc tgg cac atg aaa caa gcc ctg gcc cca atc ctg     2022
   Ser Tyr Tyr Ile Ser Trp His Met Lys Gln Ala Leu Ala Pro Ile Leu
               490                 495                 500
   ttc acc gac aac gac aaa ccc gcc gcc gcc gcc aaa cgc gcc gac ccc     2070
   Phe Thr Asp Asn Asp Lys Pro Ala Ala Ala Ala Lys Arg Ala Asp Pro
           505                 510                 515
   gtc gcg cca gcc caa cgc tcc gac gaa gcg ctg aac aag gca gca cgc     2118
   Val Ala Pro Ala Gln Arg Ser Asp Glu Ala Leu Asn Lys Ala Ala Arg
       520                 525                 530
   aaa cgc acc gaa gac aac caa ccg gtg cac agc ttc acc agc ctg ctc     2166
   Lys Arg Thr Glu Asp Asn Gln Pro Val His Ser Phe Thr Ser Leu Leu
   535                 540                 545                 550
   acc gac ctg gcc acc atc tgc gcc aac tac atc caa ccc aca gac gac     2214
   Thr Asp Leu Ala Thr Ile Cys Ala Asn Tyr Ile Gln Pro Thr Asp Asp
                   555                 560                 565
   ctg cca gca ttc acc aaa acc acc acc ccc acc ccc aca caa cgg cgc     2262
   Leu Pro Ala Phe Thr Lys Thr Thr Thr Pro Thr Pro Thr Gln Arg Arg
               570                 575                 580
   gcc ttc gac cta ctg gcc gtt tcc cac cgc cac ggc ctg gcg tag         2307
   Ala Phe Asp Leu Leu Ala Val Ser His Arg His Gly Leu Ala
           585                 590                 595
   tcagtaccga accacaaatg cccaggtcaa cgacacaaac cgcgccggat cagggggaac 2367
   ttcgggctag ccgggcgcgc cgg                                         2390
<210> 20
   <211> 596
   <212> PRT
   <213> Mycobacterium tuberculosis
<400> 20
   Val Ser Ser Ser Asp Asp Ala Ile Ile Ala Leu Thr Ala Cys Tyr Lys
   1 5 10 15
   Val Ile Met Tyr Ile Thr Arg Val Pro Asn Arg Gly Ser Pro Pro Ala
   20 25 30
   Val Leu Leu Arg Glu Ser Phe Arg Glu Asn Gly Lys Val Lys Thr Arg
   35 40 45
   Thr Leu Ala Asn Leu Ser Arg Trp Pro Glu His Lys Leu Asp Arg Leu
   50 55 60
   Asp Arg Ala Leu Lys Gly Leu Pro Pro Ala Asp Trp Asp Leu Ala Glu
   65 70 75 80
   Ala Phe Asp Ile Thr Arg Ser Leu Pro His Gly His Val Ala Ala Val
   85 90 95
   Ala Gly Thr Ala Glu Lys Leu Gly Ile Pro Glu Leu Ile Asp Pro Thr
                   100                 105                 110
   Pro Ser Arg Arg Arg Asn Leu Val Leu Ala Met Leu Ile Gly Gln Ile
           115                 120                 125
   Ile Glu Pro Gly Ser Lys Leu Ala Ile Ala Arg Gly Leu Arg Ala Gln
       130                 135                 140
   Thr Ala Thr Ser Thr Leu Gly Ala Val Leu Gly Val Ser Gly Ala Asp
   145                 150                 155                 160
   Glu Asp Asp Leu Tyr Asp Ala Met Asp Trp Ala Leu Glu Arg Lys Asp
                   165                 170                 175
   Gly Ile Glu Asn Ala Leu Ala Ala Arg His Leu Thr Asn Gly Thr Leu
               180                 185                 190
   Val Leu Tyr Asp Val Ser Ser Ala Ala Phe Glu Gly His Thr Cys Pro
           195                 200                 205
   Leu Gly Ala Ile Gly His Ala Arg Asp Gly Val Lys Gly Arg Leu Gln
       210                 215                 220
   Ile Val Tyr Gly Leu Leu Cys Ser Pro Lys Gly Ala Pro Val Ala Ile
   225                 230                 235                 240
   Glu Val Phe Lys Gly Asn Thr Ala Asp Pro Lys Thr Leu Lys Ala Gln
                   245                 250                 255
   Ile Asp Lys Leu Lys Thr Arg Phe Gly Leu Thr Arg Ile Ala Leu Val
               260                 265                 270
   Gly Asp Arg Gly Met Leu Thr Ser Ala Arg Ile Arg Asp Glu Leu Arg
           275                 280                 285
   Pro Ala His Leu Asp Trp Ile Ser Ala Leu Arg Ala Pro Gln Ile Lys
       290                 295                 300
   Ile Leu Leu Glu Asp Gly Ala Leu Gln Leu Ser Leu Phe Asp Glu Gln
   305                 310                 315                 320
   Asn Leu Phe Glu Ile Thr His Pro Asp Tyr Pro Gly Glu Arg Leu Val
                   325                 330                 335
   Cys Cys His Asn Pro Ala Leu Ala Asp Glu Arg Ala Arg Lys Arg Ala
               340                 345                 350
   Glu Leu Leu Ala Ala Thr Glu Lys Glu Leu Gln Ala Ile Ala Glu Ala
           355                 360                 365
   Thr Arg Arg Gln Arg Arg Pro Leu Arg Gly Thr Asp Lys Ile Gly Leu
       370                 375                 380
   Arg Val Gly Lys Val Arg Asn Lys Phe Lys Met Ala Lys His Phe Asp
   385                 390                 395                 400
   Leu His Ile Thr Asp Glu Ala Phe Ser Phe Thr Arg Asn Gln Asn Ser
                   405                 410                 415
   Ile Ala Ala Glu Ala Ala Leu Asp Gly Ile Tyr Val Leu Arg Thr Ser
               420                 425                 430
   Leu Pro Asp Asn Ala Leu Gly Arg Asp Asp Val Val Gly Arg Tyr Lys
           435                 440                     445
   Asp Leu Ala Asp Val Glu Arg Phe Phe Arg Thr Leu Asn Ser Glu Leu
       450                 455                     460
   Asp Val Arg Pro Ile Arg His Arg Leu Ala Asp Arg Val Arg Ala His
   465                     470                 475                 480
   Met Phe Leu His Met Leu Ser Tyr Tyr Ile Ser Trp His Met Lys Gln
                   485                 490                     495
   Ala Leu Ala Pro Ile Leu Phe Thr Asp Asn Asp Lys Pro Ala Ala Ala
               500                 505                     510
   Ala Lys Arg Ala Asp Pro Val Ala Pro Ala Gln Arg Ser Asp Glu Ala
           515                 520                     525
   Leu Asn Lys Ala Ala Arg Lys Arg Thr Glu Asp Asn Gln Pro Val His
       530                 535                     540
   Ser Phe Thr Ser Leu Leu Thr Asp Leu Ala Thr Ile Cys Ala Asn Tyr
   545                     550                 555                 560
   Ile Gln Pro Thr Asp Asp Leu Pro Ala Phe Thr Lys Thr Thr Thr Pro
                   565                 570                     575
   Thr Pro Thr Gln Arg Arg Ala Phe Asp Leu Leu Ala Val Ser His Arg
               580                 585                     590
   His Gly Leu Ala
           595

## Description

The present invention pertains to the field of biology, more particularly the subject of the present invention is the identification of a nucleotide sequence which make it possible in particular to distinguish an infection resulting from *Mycobacterium tuberculosis* from an infection resulting from *Mycobacterium africanum, Mycobacterium canetti, Mycobacterium microti, Mycobacterium bovis, Mycobacterium bovis BCG.* The subject of the present invention is also a method for detecting the sequences in question by the products of expression of these sequences and the kits for carrying out these methods. Finally, the subject of the present invention is novel vaccines.

Despite more than a century of research since the discovery of *Mycobacterium tuberculosis,* the aetiological agent of tuberculosis, this disease remains one of the major causes of human mortality. *M tuberculosis* is expected to kill 3 million people annually (Snider, 1989 Rev. Inf. Dis. S335) and the number of new people getting infected each year is rising and is estimated at 8.8 million. Although the majority of these are in developing countries, the disease is assuming renewed importance in the western countries due to the increasing number of homeless people, the impact of the AIDS epidemic, the changing global migration, and the travel patterns.

Early tuberculosis often goes unrecognized in an otherwise healthy individual. Classical initial methods of diagnosis include examination of a sputum smear under a microscope for acid-fast mycobacteria and an x-ray of the lungs. However, in a vast majority of cases the sputum smear examination is negative for Mycobacteria in the early stages of the disease, and lung changes may not be obvious on an x-ray until several months following infection. Another complicating factor is that acid-fast bacteria in a sputum smear may often be other species of mycobacteria. Antibiotics used for treating tuberculosis have considerable side effects, and must be taken as a combination of three or more drugs for a six to twelve month period. In addition, the possibility of inducing the appearance of drug resistant tuberculosis prevents therapy from being administered without solid evidence to support the diagnosis. Currently the only absolutely reliable method of diagnosis is based on culturing *M*. *tuberculosis* from the clinical specimen and identifying it morphologically and biochemically. This usually takes anywhere from three to six weeks, during which time a patient may become seriously ill and infect other individuals. Therefore, a rapid test capable of reliably detecting the presence of *M. tuberculosis* is vital for the early detection and treatment. Several molecular tests have been developed recently for the rapid detection and identification of *M. tuberculosis,* such as the Gen-Probe "Amplified *Mycobacterium tuberculosis* Direct Test"; this test amplifies *M tuberculosis* 16S ribosomal RNA from respiratory specimens and uses a chemiluminescent probe to detect the amplified product with a reported sensitivity of about 91%. The discovery of the IS6110 insertion element (Cave et al., Eisenach *et al.*, 1990 J. Infectious Diseases 161:977-981; Thierry *et al.* 1990 J. Clin. Microbiol. 28: 2668-2673) and the belief that this element may only be present in *Mycobacterium* complex (*M. tuberculosis, M.bovis, M.bovis-*BCG*. M africanum and M.microti*) spawned a whole series of rapid diagnostic strategies (Brisson-Noel *et al.,* 1991 Lancet 338: 364-366; Clarridge *et al.* 1993, J. Clin. Microbiol. 31 :2049-2056 ; Cormican et al. 1992 J. Clin. Pathology 1992, 45 : 601-604 ; Cousins et al., 1992 J. Clin. Microbiol. 30 : 255-258 ; Del Portillo et al. 1991 J. Clin. Microbiol. 29 : 2163-2168 ; Folgueira et al., 1994 Neurology 44 :1336-1338 ; Forbes et al 1993, J.Clin.Microbiol. 31 :1688-1694 ; Hermans et al. 1990 J. Clin. Microbiol. 28 ;1204-1213 ;, Kaltwasser et al. 1993 Mol. Cell. Probes 7: 465-470; Kocagoz et al. 1993 J. Clin. Microbiol. 31 :1435-1438 ; Kolk et al. 1992 J. Clin.Microbiol. 30 : 2567-2575 ; Kox et al. 1994 J.Clin.Microbiol. 32 :672-678 ; Liu et al. 1994 Neurology 44 :1161-1164; Miller et al. 1994 J. Clin.Microbiol. 32 : 393-397 ; Reischl et al. 1994 Biotechniques 17:844-845; Schluger et al. 1994 Chest 105 :1116-1121; Shawar et al. 1993 J. Clin. Microbial 31: 61-65; Wilson et al 1993 J.Clin.Microbiol. 28: 2668-2673). These tests employ various techniques to extract DNA from the sputum. PCR is used to amplify IS6110 DNA sequences from the extracted DNA. The successful amplification of this DNA is considered to be an indicator of the presence of *M. tuberculosis* infection. U.S. Pat. Nos. 5,168,039 and 5,370,998 have been issued to Crawford *et al.* for the IS6110 based detection of tuberculosis. European patent EP 0,461,045 has been issued to Guesdon for the IS6110 based detection of tuberculosis.

Thus, these molecular assays used to detect *M. tuberculosis* depend on the IS6110 insertion sequence (about 10 copies) or the 16S ribosomal RNA (thousands of copies). However, these methods do not provide any information regarding the sub-type of the mycobacteria. Indeed several dozen species of Mycobacteria are known, and most are non-pathogenic for humans; tuberculosis is usually caused by infection due to *M. tuberculosis,* with a few cases being caused by *M. bovis, M. canettii,* and *M. africanum.* In order to choose an appropriate treatment and to conduct epidemiological investigations it is absolutely necessary to be able to rapidly and accurately identify isolates, i.e to distinguish the sub-type of mycobacteria of the *Mycobcaterium* complex, originating from potential tuberculosis patients. That's the problem the present invention intends to solve.

The present application describes an isolated or purified nucleic acid wherein said nucleic acid is selected from the group consisting of:
a) SEQ ID N°1,
b) Nucleic acid having a sequence fully complementary to SEQ ID N°1.
c) Nucleic acid fragment comprising at least 8, 15, 20, 25, 30, 50, 100, 250, 500, 750, 1000, 1500, 2000, 2500, 3000 consecutive nucleotides of SEQ ID N°1;
d) Nucleic acid having at least 90% sequence identity after optimal alignment with a sequence defined in a) or b);
e) Nucleic acid that hybridizes under stringent conditions with the nucleic acid defined in a) or b);

However, the object of the invention is as claimed in claim 1. As used herein, the terms « isolated » and « purified » according to the invention refer to a level of purity that is achievable using current technology. The molecules of the invention do not need to be absolutely pure (i.e., contain absolutely no molecules of other cellular macromolecules), but should be sufficiently pure so that one of ordinary skill in the art would recognize that they are no longer present in the environment in which they were . originally found (i.e., the cellular middle). Thus, a purified or isolated molecule according to the present invention is one that have been removed from at least one other macromolecule present in the natural environment in which it was found. More preferably, the molecules of the invention are essentially purified and/or isolated, which means that the composition in which they are present is almost completely, or even absolutely, free of other macromolecules found in the environment in which the molecules of the invention are originally found. Isolation and purification thus does not occur by addition or removal of salts, solvents or elements of the periodic table, but must include the removal of at least some macromolecules. The nucleic acids encompassed by the invention are purified and/or isolated by any appropriate technique known to the ordinary artisan. Such techniques are widely known, commonly practiced, and well within the skill of the ordinary artisan. As used herein, the term " nucleic acid" refers to a polynucleotide sequence such as a single or double stranded DNA sequence, RNA sequence, cDNA sequence; such a polynucleotide sequence has been isolated, purified or synthesized and may be constituted with natural or non natural nucleotides. In a preferred embodiment the DNA molecule of the invention is a double stranded DNA molecule. As used herein, the terms "nucleic acid", "oligonucleotide", "polynucleotide" have the same meaning and are used indifferently.

By the term *"Mycobacterium* complex" as used herein, it is meant the complex of mycobacteria causing tuberculosis which are *Mycobacterium tuberculosis, Mycobacterium bovis, Mycobacterium africanum, Mycobacterium microti, Mycobacterium canettii* and the vaccine strain *Mycobacterium bovis* BCG.

The present application describes not only the entire sequence SEQ ID N°1, its complement, and its double-stranded fonn, but any fragment of this sequence, its complement, and its double-stranded form.

Typically, the fragment of SEQ ID N°1 comprises at least approximately 8 nucleotides. For example, the fragment can be between approximately 8 and 30 nucleotides and can be designed as a primer for polynucleotide synthesis. For instance, the fragment of SEQ ID N°1 comprises between approximately 1,500 and approximately 2,500 nucleotides, and more preferably 2153 nucleotides corresponding to SEQ ID N°4. As used herein, "nucleotides" is used in reference to the number of nucleotides on a single-stranded nucleic acid. However, the term also encompasses double-stranded molecules. Thus, a fragment comprising 2,153 nucleotides according to the invention is a single-stranded molecule comprising 2,153 nucleotides, and also a double stranded molecule comprising 2153 base pairs (bp).

Typically, the nucleic acid fragment as described in the present application is specifically deleted in the genome of *Mycobacterium tuberculosis,* and present in the genome of *Mycobacterium africanum. Mycobacterium canetti, Mycobacterium microti, Mycobacterium bovis, Mycobacterium bovis BCG*.

The nucleic acid fragment as described in the present application is preferably selected from the group consisting of:
a) SEQ ID N°4;
b) Nucleic acid having a sequence fully complementary to SEQ ID N°4.
c) Nucleic acid fragment comprising at least 20, 25, 30, 50, 100, 250, 500, 750, 1000, 1500, 2000, 2500, 3000 consecutive nucleotides of SEQ ID N°4;
d) Nucleic acid having at least 90% sequence identity after optimal alignment with a sequence defined in a) or b);
e) Nucleic acid that hybridizes under stringent conditions with the nucleic acid defined in a) or b);
   However, the object of the invention is as claimed in claim 2. Typically, the stringent conditions under which a sequence according to the application is determined are conditions which are no less stringent than 5xSSPE, 2xDenhardt's solution, and 0.5% (w/v) sodium dodecyl sulfate at 65°C. More stringent conditions can be utilized by the ordinary artisan, and the proper conditions for a given assay can be easily and rapidly determined without undue or excessive experimentation. As an illustrative embodiment, the stringent hybridization conditions used in order to specifically detect a polynucleotide according to the present invention are advantageously the following: pre-hybridiration and hybridization are performed at 65°C in a mixture containing:
   - 5X SSPE (1X SSPE is 3 M NaCl, 30 mM tri-sodium citrate)
   - 2X Denhardt's solution
   - 0.5% (w/v) sodium dodecyl sulfate (SDS)
   - 100 µg ml⁻¹ salmon sperm DNA.

The washings are performed as follows:
- two washings at laboratory temperature (approximately 21-25°C) for 10 min. in the presence of2X SSPE and 0.1% SDS; and
- one washing at 65°C for 15 min. in the presence of 1X SSPE and 0.1% SDS.

The invention also encompasses the isolated or purified nucleic acid of the invention wherein said nucleic acid comprises at least a deletion of a nucleic acid fragment as defined in claim 2.

Polynucleotides as described in the present application can be characterized by the percentage of identity they show with the sequences disclosed herein. For example, polynucleotides having at least 90% identity with the polynucleotides as described in the present application particularly those sequences of the sequence listing, are described in the present application. Preferably, the sequences show at least 90% identity with those of the sequence listing. More preferably, they show at least 92% identity, for example 95% or 99% identity. The skilled artisan can identify sequences according to the invention or as described in the present application the use of the sequence analysis software BLAST (see for example, Coffin et al., eds., *"Retroviruses",* Cold Spring Harbor Laboratory Press, pp. 723-755). Percent identity is calculated using the BLAST sequence analysis program suite, Version 2, available at the NCBI (NIH). All default parameters are used. BLAST (Basic Local Alignment Search Tool) is the heuristic search algorithm employed by the programs blastp, blastn, blastx, tblastn and tblastx, all of which are available through the BLAST analysis software suite at the NCBI. These programs ascribe significance to their findings using the statistical methods of Karlin and Altschul (1990, 1993) with a few enhancements. Using this publicly available sequence analysis program suite, the skilled artisan can easily identify polynucleotides as described in the present application.

It is well within the skill of the ordinary artisan to identify regions of the nucleic acid sequence of the invention or as previously described which would be useful as a probe, primer, or other experimental, diagnostic, or therapeutic aid. For example, the ordinary artisan could utilize any of the widely available sequence analysis programs to select regions (fragments) of these sequences that are useful for hybridization assays such as Southern blots, Northern blots, DNA binding assays, and/or *in vitro, in situ,* or *in vivo* hybridizations. Additionally, the ordinary artisan, with the sequences of the present invention or as previously described can utilize widely available sequence analysis programs to identify regions that can be used as probes and primers, as well as for design of anti-sense molecules. The only practical limitation on the fragment chosen by the ordinary artisan is the ability of the fragment to be useful for the purpose for which it is chosen. For example, if the ordinary artisan wished to choose a hybridization probe, he would know how to choose one of sufficient length, and of sufficient stability, to give meaningful results. The conditions chosen would be those typically used in hybridization assays developed for nucleic acid fragments of the approximate chosen length.

Thus, the present invention provides short oligonucleotides, useful as probes and primers. In embodiments, the probe and/or primer comprises 8 to 30 consecutive nucleotides of the polynucleotide according to the invention or the polynucleotide complementary thereto. Typically a fragment as defined herein has a length of at least 8 nucleotides, which is approximately the minimal length that has been determined to allow specific hybridization. For instance the nucleic fragment has a length of at least 12 nucleotides and more preferably 20 consecutive nucleotides of any of SEQ ID NO:1 or SEQ ID NO:4. The sequence according to the present invention is selected from the following group SEQ ID No. 13, SEQ ID No. 14, SEQ ID N°15, SEQ ID N°16, SEQ ID N°17, SEQ ID N°18. More precisely, the primers pairs SEQ ID N°l3lSEQ ID N°14 and SEQ ID N°15/SEQ ID N°16 are specific for nucleic acid fragment SEQ ID N°4. The primers pair SEQ ID N°17/SEQ ID No. 18 is specific for nucleic acid sequence SEQ ID N°1 and are flanking the nucleic acid fragment of SEQ ID N°4.

Thus, the polynucleotides of SEQ ID N° 1 and SEQ ID N°4, can be used to select nucleotide primers, notably for an amplification reaction, such as the amplification reactions further described.

PCR is described in US Patent No. 4,683,202.

The amplified fragments may be identified by agarose or polyacrylamide gel electrophoresis, by a capillary electrophoresis, or alternatively by a chromatography technique (gel filtration, hydrophobic chromatography, or ion exchange chromatography). The specificity of the amplification can be ensured by a molecular hybridization using as nucleic probes the polynucleotides of SEQ ID N°1 or SEQ ID N°4, and their fragments, oligonucleotides that are complementary to these polynucleotides or fragments thereof, or their amplification products themselves, or even bt DNA sequencing.

The following other techniques related to nucleic acid amplification may also be used and are generally preferred to the PCR technique. The Strand Displacement Amplification (SDA) technique is an isothermal amplification technique based on the ability of a restriction enzyme to cleave one of the strands at a recognition site (which is under a hemiphosphorothioate form) and on the property of a DNA polymerase to initiate the synthesis of a new strand from the 3'OH end generated by the restriction enzyme and on the property of this DNA polymerase to displace the previously synthesized strand being localized downstream. The SDA amplification technique is more easily performed than PCR (a single thermostatted water bath device is necessary), and is faster than the other amplification methods. Thus, the present invention also comprises using the nucleic acid fragments according to the invention (primers) in a method of DNA or RNA amplification according to the SDA technique.

When the target polynucleotide to be detected is a RNA, for example a mRNA, a reverse transcriptase enzyme will be used before the amplification reaction in order to obtain a cDNA from the RNA contained in the biological sample. The generated cDNA is subsequently used as the nucleic acid target for the primers or the probes used in an amplification process or a detection process according to the present invention.

The non-labeled polynucleotides or oligonucleotides-of the-invention can be directly used as probes. Nevertheless, the polynucleotides or oligonucleotides are generally labeled with a radioactive element (³²P, ³⁵S, ³K ¹²⁵I) or by a non-isotopic molecule (for example, biotin, acetylaminofluorene, digoxigenin, 5-bromodesoxyuridine, fluorescein) in order to generate probes that are useful for numerous applications. Examples of non-radioactive labeling of nucleic acid fragments are described in French patent N° FR 78 10975 and by Urdea *et al.* (1988, *Nucleic Acids Research* 11:4937-4957) or Sanchez-Pescador *et al.* (1988, *J. Clin. Microbiol.* 26(10):1934-1938). Other labeling techniques can also be used, such as those described in French patents FR 2 422 956 and FR 2 518 755. The hybridization step may be performed in different ways. See, for example, Matthews *et al.,* 1988, *Anal. Biochem.* **169**:1-25. A general method comprises immobilizing the nucleic acid that has been extracted from the biological sample on a substrate (for example, nitrocellulose, nylon, polystyrene) and then incubating, in defined conditions, the target nucleic acid with the probe. Subsequent to the hybridization step, the excess amount of the specific probe is discarded and the hybrid molecules formed are detected by an appropriate method (radioactivity, fluorescence or enzyme activity measurement, etc.).

Amplified nucleotide fragments are useful, among other things, as probes used in hybridization reactions in order to detect the presence of one polynucleotide according to the present invention or in order to detect mutations. The primers may also be used as oligonucleotide probes to specifically detect a polynucleotide according to the invention.

The oligonucleotide probes according to the present invention may also be used in a detection device comprising a matrix library of probes immobilized on a substrate, the sequence of each probe of a given length being localized in a shift of one or several bases, one from the other, each probe of the matrix library thus being complementary to a distinct sequence of the target nucleic acid. Optionally, the substrate of the matrix may be a material able to act as an electron donor, the detection of the matrix positions in which an hybridization has occurred being subsequently determined by an electronic device. Such matrix libraries of probes and methods of specific detection of a target nucleic acid is described in the European patent application N° EP-0 713 016 (Affymax technologies) and also in the US patent N° US-5,202,231 (Drmanac). Since almost the whole length of a mycobacterial chromosome is covered by BAC-based genomic DNA library (i.e. 97% of the *M*. *tuberculosis* chromosome is covered by the BAC library I-1945), these DNA libraries will play an important role in a plurality of post-genomic applications, such as in mycobacterial gene expression studies where the canonical set of BACs could be used as a matrix for hybridization studies. Thus it is also described in the present application to provide a nucleic acid chips, more precisely a DNA chips or a protein chips that respectively comprises a nucleic acid or a polypeptide as previously described.

The present invention is also providing a vector comprising the isolated DNA molecule of the invention. A "vector" is a replicon in which another polynucleotide segment is attached, so as to bring the replication and/or expression to the attached segment. A vector can have one or more restriction endonuclease recognition sites at which the DNA sequences can be cut in a determinable fashion without loss of an essential biological function of the vector, and into which a DNA fragment can be spliced in order to bring about its replication and cloning. Vectors can further provide primer sites (e.g. for PCR), transcriptional and/or translational initiation and/or regulation sites, recombinational signals, replicons, selectable markers, etc. Beside the use of homologous recombination or restriction enzymes to insert a desired DNA fragment into the vector, UDG cloning of PCR fragments (US Pat. No. 5,334,575), T:A cloning, and the like can also be applied. The cloning vector can further contain a selectable marker suitable for use in the identification of cells transformed with the cloning vector.

The vector can be any useful vector known to the ordinary artisan, including, but not limited to, a cloning vector, an insertion vector, or an expression vector. Examples of vectors include plasmids, phages, cosmids, phagemid, yeast artificial chromosome (YAC), bacterial artificial chromosome (BAC), human artificial chromosome (HAC), viral vector, such as adenoviral vector, retroviral vector, and other DNA sequences which are able to replicate or to be replicated *in vitro* or in a host cell, or to convey a desired DNA segment to a desired location within a host cell. According to a preferred embodiment of the invention, the recombinant vector is a BAC pBeloBAC11 in which the genomic region of *Mycobacterium bovis-BCG* 1173P3 that spans the region corresponding to the locus 1,760,753 bp to 1,830,364 bp in the genome of *M. tuberculosis* H37Rv has been inserted into the HindIII restriction site; this recombinant vector is named X229. In this region, the inventors have demonstrated the deletion of a 2153 bp fragment in the vast majority of *M. tuberculosis* strain. That's the reason why the inventors named this deletion TbD1 (*"M. tuberculosis* specific deletion 1"). TbD1 is flanked by the sequence GGC CTG GTC AAA CGC GGC TGG ATG CTG and AGA TCC GTC TTT GAC ACG ATC GAC G. External primers hybridizing with such sequences or the complentary sequences thereof can be used for the amplification of TbD1 to check for the presence or the absence of the deletion of the TbD1. The inventors design for example the following primers:
5'- CTA CCT CAT CTT CCG GTC CA-3' (SEQ ID N° 17)
5'- CAT AGA TCC CGG ACA TGG TG-3'(SEQ ID N° 18)
In order to get a specific 500 pb probe for hybridization experiments, a PCR amplification of an internal fragment may be realized by using the plasmid X229 as a matrix. The amplification of a fragment of approximatively 500 bp contained in TbD1 can be performed by using the following primers:
5'- CGT TCA ACC CCA AAC AGG TA-3' (SEQ ID N° 13)
5'- AAT CGA ACT CGT GGA ACA CC-3' (SEQ ID N° 14)
The amplification of a fragment of approximatively 2,000 bp contained in TbD1 can be performed by using the following primers:
5'- ATT CAG CGT CTA TCG GTT GC-3' (SEQ ID N° 15)
5'- AGC AGC TCG GGA TAT CGT AG-3' (SEQ ID N° 16)
The PCR conditions are the following: denaturation 95°C 1 min, then 35 cycles of amplification [95°C during 30 seconds, 58°C during 1 min], then elongation 72°C during 4 min.

Thus, this invention also concerns a recombinant cell host which contains a polynucleotide or recombinant vector according to the invention. The cell host can be transformed or transfected with a polynucleotide or recombinant vector to provide transient, stable, or controlled expression of the desired polynucleotide. For example, the polynucleotide of interest can be subcloned into an expression plasmid at a cloning site downstream from a promoter in the plasmid and the plasmid can be introduced into a host cell where expression can occur. The recombinant host cell can be any suitable host known to the skilled artisan, such as a eukaryotic cell or a microorganism. For example, the host can be a cell selected from the group consisting of *Escherichia coli, Bacillus subtilis,* insect cells, and yeasts. Preferably, the recombinant cell host is a commercially available *Escherichia coli* DH10B (Gibco) containing the BAC named X229 previously described. This *Escherichia coli* DH10B (Gibco) containing the BAC named X229 has been deposited with the Collection Nationale de Cultures de Microorganismes (CNCM). Institut Pasteur, Paris, France, on February 18^{th}, 2002 under number CNCM 1-2799.

Another aspect of the invention is the isolated or purified polypeptides encoded by a polynucleotide of the invention in particular nucleic acid of claims 1 or 2. The purified polypeptide comprises an amino acid sequence SEQ ID N° 2, N° 8, N° 10, N°12, and their fragments thereof. For example, the purified polypeptide of the invention can comprise the amino acid sequence of SEQ ID N°8 a truncated form of mmpL6. The purified polypeptide of the invention can comprise the amino acid sequence of SEQ ID NO:10, which is the amino acid sequence of the mmpS6 protein or the amino acid sequence of SEQ ID N°12 a truncated form of mmpS6.

It is now easy to produce proteins in large amounts by genetic engineering techniques through the use of expression vectors, such as plasmids, phages, and phagemids. The polypeptide of the present invention can be produced by insertion of the appropriate polynucleotide into an appropriate expression vector at the appropriate position within the vector. Such manipulation of polynucleotides is well known and widely practiced by the ordinary artisan. The polypeptide can be produced from these recombinant vectors either *in vitro* or *in vivo.* All the isolated or purified nucleic acid encoding the polypeptide of the invention are in the scope of the invention. A polypeptide described in the present application is a polypeptide encoded by a polynucleotide which hybridizes to any of SEQ ID N°1 or N°4 under stringent conditions, as defined herein.
More preferably, said isolated or purified nucleic acid according the invention is selected among:
- SEQ ID N° 7 encoding the polypeptide of SEQ ID N°8;
- SEQ ID N° 9 encoding the polypeptide of SEQ ID N°10;
- SEQ ID N° 11 encoding the polypeptide of SEQ ID N°12.

The present application also describes a method for the discriminatory detection and identification of:
- *Mycobacterium tuberculosis* versus,
- *Mycobacterium africanum*, *Mycobacterium canettii, Mycobacterium microti, Mycobacterium bovis*, *Mycobacterium bovis BCG* in a biological sample, comprising the following steps:
   a) isolation of the DNA from the biological sample to be analyzed or production of a cDNA from the RNA of the biological sample,
   b) detection of the nucleic acid sequences of the mycobacterium present in said biological sample,
   c) analysis for the presence or the absence of a nucleic acid fragment as previously described.
By a biological sample according to the present invention, it is notably intended a biological fluid, such as sputum, saliva, plasma, blood, urine or sperm, ora tissue, such as a biopsy.

Analysis of the desired sequences may, for example, be carried out by agarose gel electrophoresis. If the presence of a DNA fragment migrating to the expected site is observed, it can be concluded that the analyzed sample contained mycobacterial DNA. This analysis can also be carried out by the molecular hybridization technique using a nucleic probe. This probe will be advantageously labeled with a nonradioactive (cold probe) or radioactive element. Advantageously, the detection of the mycobacterial DNA sequences will be carried out using nucleotide sequences complementary to said DNA sequences. By way of example, they may include labeled or nonlabeled nucleotide probes; they may also include primers for amplification. The amplification technique used may be PCR but also other alternative techniques such as the SDA (Strand Displacement Amplification) technique, the TAS technique (Transcription-based Amplification System), the NASBA (Nucleic Acid Sequence Based Amplification) technique or the TMA (Transcription Mediated Amplification) technique.

The primers in accordance with the invention have a nucleotide sequence chosen from the group comprising SEQ ID No. 13, SEQ ID No. 14, SEQ ID N°15, SEQ ID N°16, SEQ ID N°17, SEQ ID N°18. The pairs SEQ ID N°13/SEQ ID N°14 and SEQ ID N°15/SEQ ID N°16 specific for nucleic acid fragment SEQ ID N°4, and the pair SEQ ID N°17/SEQ ID N°18 specific for nucleic acid of the invention.

In a variant, the present application also describes a method for the discriminatory detection and identification of:
- *Mycobacterium tuberculosis* versus,
- *Mycobacterium africanum, Mycobacterium canettii, Mycobacterium microti, Mycobacterium bovis, Mycobacterium bovis BCG* in a biological sample, comprising the following steps:
   a) bringing the biological sample to be analyzed into contact with at least one pair of primers as defined above the DNA contained in the sample having been, where appropriate, made accessible to the hybridization beforehand,
   b) amplification of the DNA of the mycobacterium,
   c) visualization of the amplification of the DNA fragments.

The amplified fragments may be identified by agarose or polyacrylamide gel electrophoresis by capillary electrophoresis or by a chromatographic technique (gel filtration, hydrophobic chromatography or ion-exchange chromatography). The specification of the amplification may be controlled by molecular hybridization using probes, plasmids containing these sequences or their product of amplification. The amplified nucleotide fragments may be used as reagent in hybridization reactions in order to detect the presence, in a biological sample, of a target nucleic acid having sequences complementary to those of said amplified nucleotide fragments. These probes and amplicons may be labeled or otherwise with radioactive elements or with nonradioactive molecules such as enzymes or fluorescent elements.

The present application also describes a kit for the discriminatory detection and identification of
- *Mycobacterium tuberculosis* versus,
- *Mycobacterium africanum, Mycobacterium canettii, Mycobacterium microti, Mycobacterium bovis, Mycobacterium bovis BCG* in a biological sample, in a biological sample comprising the following elements:
   a) at least one pair of primers selected among nucleic acid fragments according to claims 1 to 3, 6 and 7.
   b) the reagents necessary to carry out a DNA amplification reaction.
      The present application describes a kit comprising optionally element c):
   c) the necessary components which make it possible to verify or compare the sequence and/or the size of the amplified fragment.

Indeed, depending on the pair of primers used, it is possible to obtain very different results. Thus, the use of primers which are internal to the deletion, such as for example SEQ ID N°13, SEQ ID N°14, SEQ ID N°15, SEQ ID N°16, is such that no amplification product is detectable in *M. tuberculosis* and that amplification product is detectable in *Mycobacterium africanum, Mycobacterium canettii, Mycobacterium microti, Mycobacterium bovis, Mycobacterium bovis BCG,* However, the use of primers external to the region of deletion does not necessarily give the same result, as regards for example the size of the amplified fragment, depending on the size of the deleted region in *M. tuberculosis.* Thus, the use of the pair of primers external to the deletion such as SEQ ID N° 17 and SEQ ID N° 18 is likely to give rise to an amplicon in *Mycobacterium africanum, Mycobacterium canettii, Mycobacterium microti, Mycobacterium bovis, Mycobacterium bovis BCG* of about 2100 bp whereas the use of the pair of primers external to the deletion will give rise in *M. tuberculosis* to an amplicon of about few bp.

More generally, the invention pertains to the use of at least one pair of primers as claimed in claim 16 for the amplification of a DNA sequence from *Mycobacterium tuberculosis or Mycobacterium africanum*, *Mycobacterium canettii, Mycobacterium microti*, *Mycobacterium bovis, Mycobacterium bovis BCG,*

The subject of the invention is also a protein, polypeptide or polypeptide fragment resulting from the expression of all or part of the nucleic acid fragment as defined in claim 2. The expression "product of expression" is understood to mean any protein, polypeptide or polypeptide fragment resulting from the expression of all or part of the above-mentioned nucleotide sequences. Among those product of expression, one can cite the membrane proteins mmpL6, mmpS6, and their truncated or rearranged form due to the deletion of the fragment of the invention.

Indeed, the present application also describes a method for the discriminatory detection *in vitro* of antibodies directed against *Mycobacterium tuberculosis* or *Mycobacterium africanum, Mycobacterium canettii, Mycobacterium microti, Mycobacterium bovis, Mycobacterium bovis BCG* in a biological sample, comprising the following steps:
a) bringing the biological sample into contact with at least one product as previously defined,
b) detecting the antigen-antibody complex formed.

The present application also describes a method for the discriminatory detection of a vaccination with *Mycobacterium bovis* BCG or an infection by *Mycobacterium tuberculosis*. in a mammal, comprising the following steps:
a) preparation of a biological sample containing cells, more particularly cells of he immune system of said mammal and more particularly T cells,
b) incubation of the biological sample of step a) with at least one product as previously defined,
c) detection of a cellular reaction indicating prior sensitization of the mammal to said product, in particular cell proliferation and/ot synthesis of proteins such as gamma-interferon. Cell proliferation may be measured, for example, by incorporating ³H-Thymidine.

The application also relates to a kit for the *in vitro* diagnosis of an *Mycobacterium tuberculosis* infection, in a mammal optionally vaccinated beforehand with *M. bovis* BCG comprising:
a) a product as previously defined,
b) where appropriate, the reagents for the constitution of the medium suitable for the immunological reaction,
c) the reagents allowing the detection of the antigen-antibody complexes produced by the immunological reaction,
d) where appropriate, a reference biological sample (negative control) free of antibodies recognized by said product,
e) where appropriate, a reference biological sample (positive control) containing a predetermined quantity of antibodies recognized by said product.
   The reagents allowing the detection of the antigen-antibody complexes may carry a marker or may be capable of being recognized in turn by a labeled reagent, more particularly in the case where the antibody used is not labeled.

The subject of the invention is also mono- or polyclonal antibodies, their chimeric fragments or antibodies, capable of specifically recognizing a product as defined in claim 13.

The present application therefore also relates to a method for the discriminatory detection of the presence of an antigen of *Mycobacterium tuberculosis* or *Mycobacterium africanum*, *Mycobacterium canettii*, *Mycobacterium microti*, *Mycobacterium bovis. Mycobacterium bovis-*BCG, in a biological sample comprising the following steps:
a) bringing the biological sample into contact with an antibody of the invention,
b) detecting the antigen-antibody complex formed.

The invention also relates to the kit for the discriminatory detection of the presence of an antigen of *Mycobacterium tuberculosis* with TbD₁ deletion according to SEQ ID N°4 or *Mycobacterium tuberculosis* without TbD₁ deletion or *Mycobacterium africanum, or Mycobacterium canettii, or Mycobacterium microti, or Mycobacterium bovis or Mycobacterium bovis BCG.* in a biological sample comprising the following elements :
a) an antibody as previously claimed in claim 19
b) the reagents for constituting the medium suitable for the immunological reaction,
c) the reagents allowing the detection of the antigen-antibody compass produced by the immunological reaction.

The above-mentioned reagents are well known to a person skilled in the art who will have no difficulty adapting them to the context of the present invention.

The present application also describes an immunological composition, characterized in that it comprises at least one product of expression as previously described

Advantageously, the immunological composition in accordance with the present application enters into the composition of a vaccine when it is provided in combination with a pharmaceutically acceptable vehicle and optionally with one or more immunity adjuvant(s) such as alum or a representative of the family of muramylpeptides or incomplete Freund's adjuvant.

The present application also relates to a vaccine comprising at least one product of expression as previously described in combination with a pharmaceutically compatible vehicle and, where appropriate, one ormore appropriate immunity adjuvant(s).

The invention also provide an in vitro method for the detection and identification of *Mycobacterium tuberculosis* in a biological sample,
comprising the following steps:
a) isolation of the DNA from the biological sample to be analyzed or production of a cDNA from the RNA of the biological sample,
b) detection of the nucleic acid sequences of the mycobacterium present in said biological sample,
c) analysis for the presence or the absence of a nucleic acid fragment according to claim 2.

Furthermore, the object of the invention is as claimed in claims 13, 14 and 15.

In another embodiment, the present application describes an *in vitro* method for the detection and identification of *Mycobacterium tuberculosis* in a biological sample, comprising the following steps:
a) bringing the biological sample to be analyzed into contact with at least one pair of primers selected among nucleic acid fragments as previously described, and more preferably selected among the primers chosen from the group comprising SEQ ID N°13, SEQ ID N°14, SEQ ID N°15, SEQ ID N°16, SEQ ID N°17, SEQ ID N°18, the DNA contained in the sample having been, where appropriate, made accessible to the hybridization beforehand,
b) amplification of the DNA of the mycobacterium,
c) visualization of the amplification of the DNA fragments.

The invention also provides a kit for the detection of the TbD1 detection according to SEQ ID N; *Mycobacterium tuberculosis* CTG in a biological sample, comprising the following elements:
a) at least one pair of primers comprising 8 to 30 consecutive nucleotides of the polynucleotides according to claims 2, 3 and 6 selected among nucleic acid fragments more preferably selected among the primers chosen from the group comprising SEQ ID N°13, SEQ ID N°14, SEQ ID N°15, SEQ ID N°16, SEQ ID N°17, SEQ ID N°18,
b) the reagents necessary to carry out a DNA amplification reaction,

The present application also described a kit comprising optionally element c):
c) the necessary components which make it possible to verify or compare the sequence and/or the size of the amplified fragment.

The present application also relates to a method for the detection *in vitro* of antibodies directed against *Mycobacterium tuberculosis* in a biological sample, comprising the following steps:
a) bringing the biological sample into contact with at least one product as defined previously,
b) detecting the antigen-antibody complex formed.

It is also a goal of the invention to use the TbD1 deletion according to SEQ ID N°4 as a as a genetic marker for the differentiation of *Mycobacterium* strain of *Mycobacterium tuberculosis* complex.

It is also a goal of the present application to use mmpL6⁵⁵¹ polymorphism as a genetic marker (see SEQ ID N°20) for the differentiation of *Mycobacterium* strain of *Mycobacterium tuberculosis* complex.

The use of such genetic marker(s) in association with at least one genetic markers selected among RD1, RD2, RD3, RD4, RD5, RD6, RD7, RD8, RD9, RD10, Rd11, RD13, RD14, RvD1, RvD2, RvD3, RvD4, RvD5, katG⁴⁶³, gyrA⁹⁵, oxyR'²⁸⁵, pncA⁵⁷ allows the differentiation of Mycobacterium strain of Mycobacterium Tuberculosis complex (see example 4).

The present application describes an *in vitro* method for the detection and identification of *Mycobacteria* from the *Mycobacterium Tuberculosis* complex in a biological sample, comprising the following steps:
a) analysis for the presence or the absence of a nucleic acid fragment of the sequence TbD1, and
b) analysis of at least one additional genetic marker selected among RD1, RD2, RD3, RD4, RD5, RD6, RD7, RD8, RD9, RD10, Rd11, RD13, RD14, RvD1, RvD2, RvD3, RvD4, RvD5, katG⁴⁶³, gyrA⁹⁵, oxyR'²⁸⁵, pncA⁵⁷.

Typically two additional markers are used, preferably RD4 and RD9. The analysis is performed by a technique selected among sequence hybridization, nucleic acid amplification, antigen-antibody complex.

It is also a goal of the present invention to provide a kit for the detection and dentification of *Mycobacteria* from the *Mycobacterium Tuberculosis* complex in a biological sample comprising the following elements:
a) at least one pair of primers comprising 8 to 30 consecutive nucleotides of the polynucleotide according to claims 2,3 and 6 and more preferably selected among the primers chosen from the group comprising SEQ ID N°13, SEQ ID N°14, SEQ ID N°15, SEQ ID N° 16, SEQ ID N°17, SEQ ID N°18,
b) the reagents necessary to carry out a DNA amplification reaction,
c) at least one pair of primers specific of the genetic markers selected among RD1, RD2, RD3, RD4, RD5, RD6, RD7, RD8, RD9, RD10, Rd11, RD13, RD14, RvD1, RvD2, RvD3, RvD4, RvD5, katG⁴⁶³, gyrA⁹⁵, oxyR'²⁸⁵, pncA⁵⁷.

The present application also describes a kit comprising optionally element d) :
d) the necessary components which make it possible to verify or compare the sequence and/or the size of the amplified fragment.

In a preferred embodiment, the kit according the present invention is characterized in that said pairs of primers specific of genetic markers according to c) are
b) one pair of primers specific of the genetic markers RD4, and
c) one pair of primers specific of the genetic markers RD9.

The figures and examples presented below are provided as further guide to the practitioner of ordinary skill in the art and are not to be construed as limiting the invention in anyway.

### FIGURES

**Figure 1** : Amplicons obtained from strains that have the indicated genomic region present or deleted. Sizes of amplicons in each group are uniform. Numbers correspond to strain designation used in Kremer et al. (1999, J. Clin Microbial. 37: 2607-2618) (Ref. 8) and Supply et al (2001, J. Clin. Microbiol. 39: 3563-3571) (ref.9).
**Figure 2**: Sequences in the TbD1 region obtained from strains of various geographic regions.
   * refers to groups based on *katG*^{c463}/*gyrA*^{c95} sequence polymorphism defined by Sreevatsan and colleagues (Ref. 2). Numbers correspond to strain designation used in Kremer et al. (1999, J. Clin Microbiol. 37: 2607-2618) (Ref. 8) and Supply et al (2001, J. Clin. Microbial. 39: 3563-3571) (ref.9).

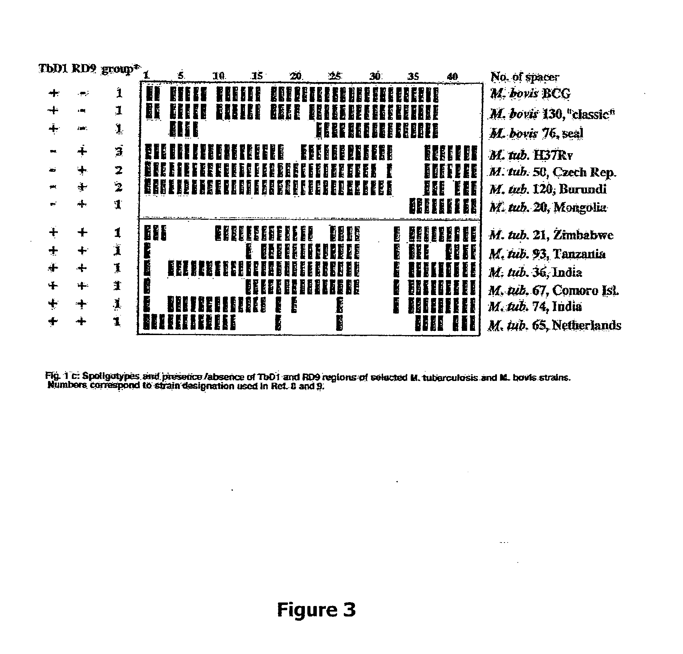

**Figure 3**: Spoligotypes of selected *M. tuberculosis* and *M. bovis* strains. Numbers correspond to strain designation used in Kremer et al. (1999, J. Clin Microbiol. 37: 2607-2618) (Ref. 8) and Supply et al (2001, J. Clin. Microbiol. 39: 3563-3571) (ref.9).

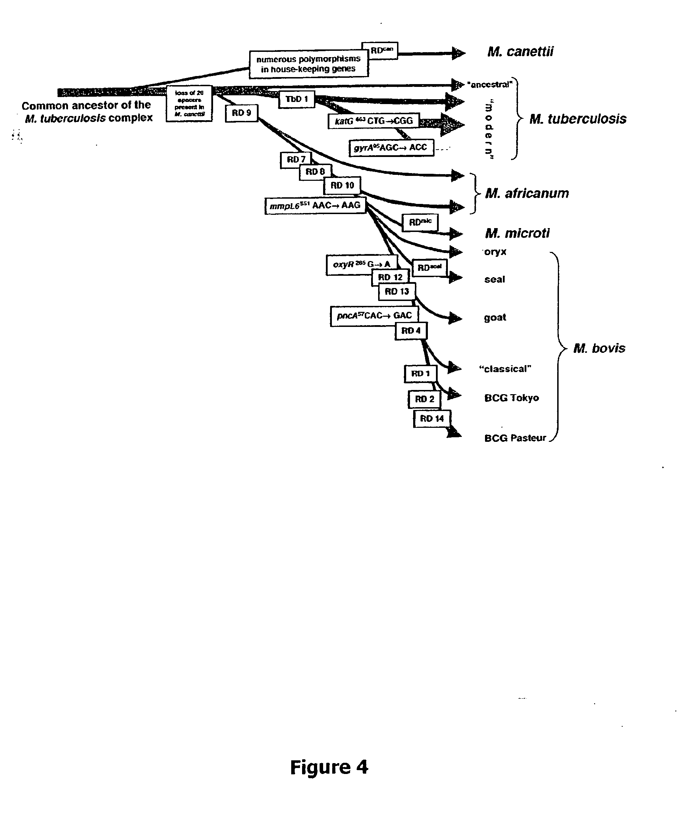

**Figure 4**: Scheme of the proposed evolutionary pathway of the tubercle bacilli illustrating successive loss of DNA in certain lineages (grey boxes) The scheme is based on presence or absence of conserved deleted regions and on sequence polymorphisms in five selected genes. Note that the distances between certain branches may not correspond to actual phylogenetic differences calculated by other methods.
   Blue arrows indicate that strains are characterized by *katG*^{c463} CTG (Leu), *gyrA*^{*c9*5} ACC (Thr), typical for group 1 organisms. Green arrows indicate that strains belong to group 2 characterized by *katG*^{*c*463} CGG (Arg), *gyrA^{c95}* ACC (Thr). The red arrow indicates that strains belong to group 3, charcterized by *katG^{c463}* CGG (Arg), *gyrA^{c95}* AGC (Ser), as defined by Sreevatsan and colleagues (Sreevastan et al., 1997 Proc. Natl. Acad.Sci USA 151: 9869-9874) (Ref. 2).

### EXAMPLES

### 1. MATERIAL AND METHODS:

**1.1. Bacterial Strains:** The 100 *M. tuberculosis* complex strains comprised 46 *M. tuberculosis* strains isolated in 30 countries, 14 *M. africanum* strains, 28 *M. bovis* strains originating in 5 countries, 2 *M. bovis* BCG vaccine strains (Pasteur and Japan), 5 *M. microti* strains, and 5 *M. canettii* strains. The strains were isolated from human and animal sources and were selected to represent a wide diversity including 60 strains that have been used in a multi-center study (8). The *M. africanum* strains were retrieved from the collection of the Wadsworth Center, New York State Department of Health, Albany, New York, whereas the majority of the *M. bovis* isolates came from the collection of the University of Zaragoza, Spain. Four *M. canettii* strains are from the culture collection of the Institut Pasteur, Paris, France. The strains have been extensively characterized by reference typing methods, i.e. IS*6110* restriction fragment length polymorphism (RFLP) typing and spoligotyping. *M. tuberculosis* H37Rv, *M. tuberculosis* H37Ra, *M. tuberculosis* CDC1551, *M. bovis* AF2122/97, *M. microti* OV254, and *M. canettii* CIPT 140010059 were included as reference strains. DNA was prepared as previously described (10).
**1.2. Genome comparisons and primer design**
   For preliminary genome comparisons between *M. tuberculosis* and *M. bovis* websites http://genolist.pasteur.fr/TubercuList/ and http://www.sanger.ac.uk/Projects/M bovis/ as well as inhouse databases were used. For primer design, sequences inside or flanking RD and RvD regions were obtained from the same websites. Primers were designed using the primer 3 website http://www-genome.wi.mit.edu/cgi-bin/primer/primer3 www.cgi that would amplify ca. 500 base pair fragments in the reference strains (Table 1).
**1.3. RD-PCR analysis**
   Reactions were performed in 96 well plates and contained per reaction 1.25 µl of 10 x PCR buffer (600mM Tris HCl pH 8.8, 20 mM MgCl₂, 170 mM (NH₄)₂SO₄, 100 mM β-mercaptoethanol), 1.25 µl 20mM nucleotide mix, 50 nM of each primer, 1-10 ng of template DNA, 10% DMSO, 0.2 units *Taq* polymerase (Gibco-BRL) and sterile distilled water to 12.5 µl. Thermal cycling was performed on a PTC-100 amplifier (MJ Inc.) with an initial denaturation step of 90 seconds at 95°C, followed by 35 cycles of 30 seconds at 95°C, 1 min at 58°C, and 4 min at 72°C.
**1.4. Sequencing of junction regions (RDs, TbD1,) *kalG*,*gyrA*, *oxyR* and *pncA* genes**
   PCR products were obtained as described above, using primers listed in Table 1.
   For primer elimination, 6 µl PCR product was incubated with 1 unit of Shrimp Alkaline phosphatase (USB), 10 units of exonuclease I (USB), and 2 µl of 5 x buffer (200mM Tris HCl pH 8.8, 5mM MgCl₂) for 15 min at 37°C and then for 15 min at 80°C. To this reaction mixture 2 µl of Big Dye sequencing mix (Applied Biosystems), 2 µl (2µM) of primer and 3 µl of 5 x buffer (5mM MgCl₂, 200mM Tris HCl pH 8.8) were added and 35 cycles (96°C for 30 sec; 56°C for 15 sec; 60°C for 4 min) performed in a thermocycler (MJ-research Inc., Watertown, MA). DNA was precipitated using 80 µl of 76% ethanol, centrifuged, rinsed with 70% ethanol, and dried. Reactions were dissolved in 2 µl of formamide/EDTA buffer, denatured and loaded onto 48 cm, 4 % polyacrylamide gels and electrophoresis performed on 377 automated DNA sequencers (Applied Biosystems) for 10 to 12 h. Alternatively, reactions were dissolved in 0.3 mM EDTA buffer and subjected to automated sequencing on a 3700 DNA sequencer (Applied Biosystems). Reactions generally gave between 500-700 bp of unambiguous sequence.
**1.5. Accession Numbers**
   The sequence of the TBD1 region from the ancestral *M. tuberculosis* strain No. 74 (Ref. 8) containing genes *mmpS6* and *mmpL6* was deposited in the EMBL database under accession No. AJ426486. Sequences bordering RD4, RD7, RD8, RD9 and RD10 in BCG are available under accession numbers AJ003103, AJ007301, AJ131210, Y18604, and AJ132559, respectively.

### 2. EXPERIMENTAL DATA:

The distribution of 20 variable regions resulting from insertion-deletion events in the genomes of the tubercle bacilli has been evaluated in a total of 100 strains of *Mycobacterium tuberculosis, M africanum, M canettii, M microti* and *M bovis.* This approach showed that the majority of these polymorphisms did not occur independently in the different strains of the *M tuberculosis* complex but, rather, result from ancient, irreversible genetic events in common progenitor strains. Based on the presence or absence of an *M. tuberculosis* specific deletion (TbD1), *M tuberculosis* strains can be divided into ancestral and "modern" strains, the latter comprising representatives of major epidemics like the Beijing, Haarlem and African *M. tuberculosis* clusters. Furthermore, successive loss of DNA, reflected by RD9 and other subsequent deletions, was identified for an evolutionary lineage represented by *M*. *africanum, M microti* and *M bovis* that diverged from the progenitor of the present *M tuberculosis* strains before TbD1 occurred. These findings contradict the often-presented hypothesis that *M. tuberculosis,* the etiological agent of human tuberculosis evolved from *M bovis,* the agent of bovine disease. *M canettii* and ancestral *M tuberculosis* strains lack none of these deleted regions and therefore appear to be direct descendants of tubercle bacilli that existed before the *M. africanum*→ *M bovis* lineage separated from the *M tuberculosis* lineage. This suggests that the common ancestor of the tubercle bacilli resembled *M tuberculosis* or *M. canettii* and could well have been a human pathogen already.

The mycobacteria grouped in the *M tuberculosis* complex are characterized by 99.9% similarity at the nucleotide level and identical 16S rRNA sequences (1,2) but differ widely in terms of their host tropisms, phenotypes and pathogenicity. Assuming that they are all derived from a common ancestor, it is intriguing that some are exclusive human (*M. tuberculosis, M africanum, M canettii)* or rodent pathogens *(M. microti)* whereas others have a wide host spectrum *(M. bovis).* What was the genetic organization of the last common ancestor of the tubercle bacilli and in which host did it live? Which genetic events may have contributed to the fact that the host spectrum is so different and often specific? Where and when did *M. tuberculosis* evolve? Answers to these questions are important for a better understanding of the pathogenicity and the global epidemiology of tuberculosis and may help to anticipate future trends in the spread of the disease.

Because of the unusually high degree of conservation in their housekeeping genes it has been suggested that the members of the *M tuberculosis* complex underwent an evolutionary bottleneck at the time of speciation, estimated to have occurred roughly 15,000 - 20,000 years ago (2). It also has been speculated that *M. tuberculosis,* the most widespread etiological agent of human tuberculosis has evolved from *M bovis,* the agent of bovine tuberculosis, by specific adaptation of an animal pathogen to the human host (3). However, both hypotheses were proposed before the whole genome sequence of *M. tuberculosis (4)* was available and before comparative genomics uncovered several variable genomic regions in the members of the *M tuberculosis* complex. Differential hybridization arrays identified 14 regions (RD1 -14) ranging in size from 2 to 12.7 kb that were absent from BCG Pasteur relative to *M. tuberculosis* H37Rv (5, 6). In parallel, six regions, RvD1-5, and TbD1, that were absent from the *M tuberculosis* H37Rv genome relative to other members of the *M. tuberculosis* complex were revealed by comparative genomics approaches employing pulsed-field gel electrophoresis (PFGE) techniques (5, 7) and *in silico* comparisons of the near complete *M bovis* AF2122/97 genome sequence and the *M tuberculosis* H37Rv sequence.

In the present study the inventors have analyzed the distribution of these 20 variable regions situated around the genome (Table 1) in a representative and diverse set of 100 strains belonging to the *M tuberculosis* complex. The strains were isolated from different hosts, from a broad range of geographic origins, and exhibit a wide spectrum of typing characteristics like *IS6110* and spoligotype hybridization patterns or variable-number tandem repeats of mycobacterial interspersed repetitive units (MIRU-VNTR) (8, 9). The inventors have found striking evidence that deletion of certain variable genomic regions did not occur independently in the different strains of the *M tuberculosis* complex and, assuming that there is little or no recombination of chromosomal segments between the various lineages of the complex, this allows the inventors to propose a completely new scenario for the evolution of the *M. tuberculosis* complex and the origin of human tuberculosis.

### Variable genomic regions and their occurrence in the members of the M. tuberculosis complex.

The PCR screening assay for the 20 variable regions (Table 1) within 46 *M. uberculosis,* 14 *M. africanum,* 5 *M. canettii,* 5 *M. microti,* 28 *M bovis* and 2 BCG strains employed oligonucleotides internal to known RDs and RvDs, as well as oligonucleotides flanking these regions (Table 1). This approach generated a large data set that was robust, highly reliable, and internally controlled since PCR amplicons obtained with the internal primer pair correlated with the absence of an appropriately sized amplicon with the flanking primer-pair, and *vice-versa.*

According to the conservation of junction sequences flanking the variable regions three types of regions were distinguished, each having different importance as an evolutionary marker. The first type included mobile genetic elements, like the prophages phiRv1 (RD3) and phiRv2 (RD11) and insertion sequences IS1532 (RD6) and IS6110 (RD5), whose distribution in the tubercle bacilli was highly divergent (Table 2). The second type of deletion is mediated by homologous recombination between adjacent IS*6110* insertion elements resulting in the loss of the intervening DNA segment (RvD2, RvD3, RvD4, and RvD5 (7)) and is variable from strain to strain (Table 2).

The third type includes deletions whose bordering genomic regions typically do not contain repetitive sequences. Often this type of deletion occurred in coding regions resulting in the truncation of genes that are still intact in other strains of the *M tuberculosis* complex. The exact mechanism leading to this type of deletion remains obscure, but possibly rare strand slippage errors of DNA polymerase may have contributed to this event. As shown in detail below, RD1, RD2, RD4, RD7, RD8, RD9, RD10, RD12, RD13, RD14, and TbD1 are representatives of this third group whose distribution among the 100 strains allows us to propose an evolutionary scenario for the members of the *M tuberculosis* complex, that identified *M. tuberculosis* and/or *M. canettii* as most closely related to the common ancestor of the tubercle bacilli.

### 2.1. M. tuberculosis strains:

Investigation of the 46 *M. tuberculosis* strains by deletion analysis revealed that most RD regions were present in all *M tuberculosis* strains tested (Table 2). Only regions RD3 and RD11, corresponding to the two prophages phiRv1 and phiRv2 of *M. tuberculosis* H37Rv (4), RD6 containing the insertion sequence IS*1532*, and RD5 that is flanked by a copy of IS*6110* (5) were absent in some strains. This is an important observation as it implies that *M tuberculosis* strains are highly conserved with respect to RD1, RD2, RD4, RD7, RD8, RD9, RD10, RD12, RD13, and RD14, and that these RDs represent regions that can differentiate *M tuberculosis* strains independent of their geographical origin and their typing characteristics from certain other members of the *M tuberculosis* complex. Furthermore, this suggests that these regions may be involved in the host specificity of *M*. *tuberculosis.*

In contrast, the presence or absence of RvD regions in *M. tuberculosis* strains was variable. The region which showed the greatest variability was RvD2, since 18 from 46 tested *M tuberculosis* strains did not carry the RvD2 region. Strains with a high copy number of IS*6110* (>14) missed regions RvD2 to RvD5 more often than strains with only a few copies. As an example, all six tested strains belonging to the Beijing cluster (8) lacked regions RvD2 and RvD3. This is in agreement with the proposed involvement of recombination of two adjacent copies of IS6*110* in this deletion event (7).

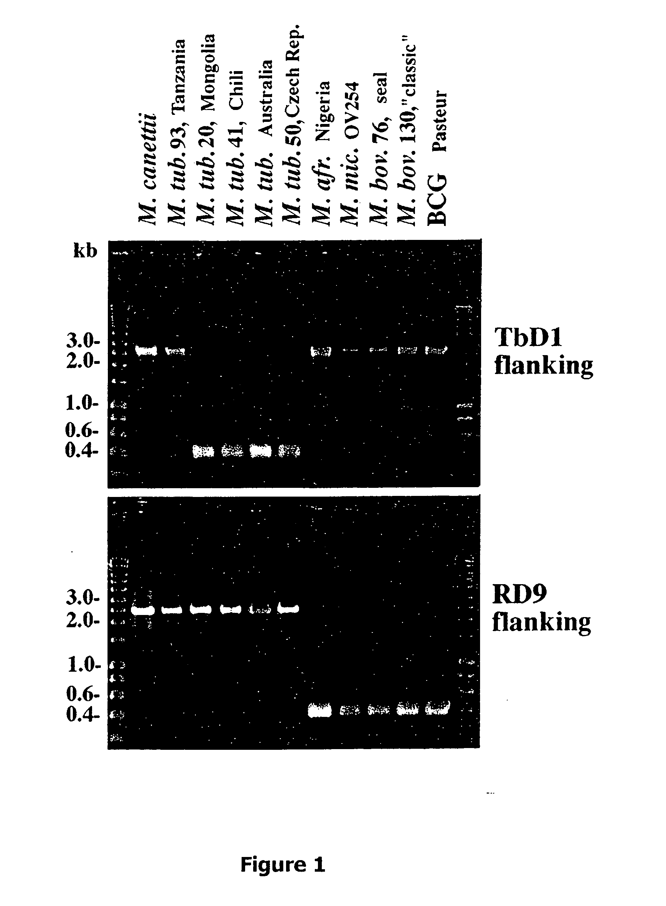

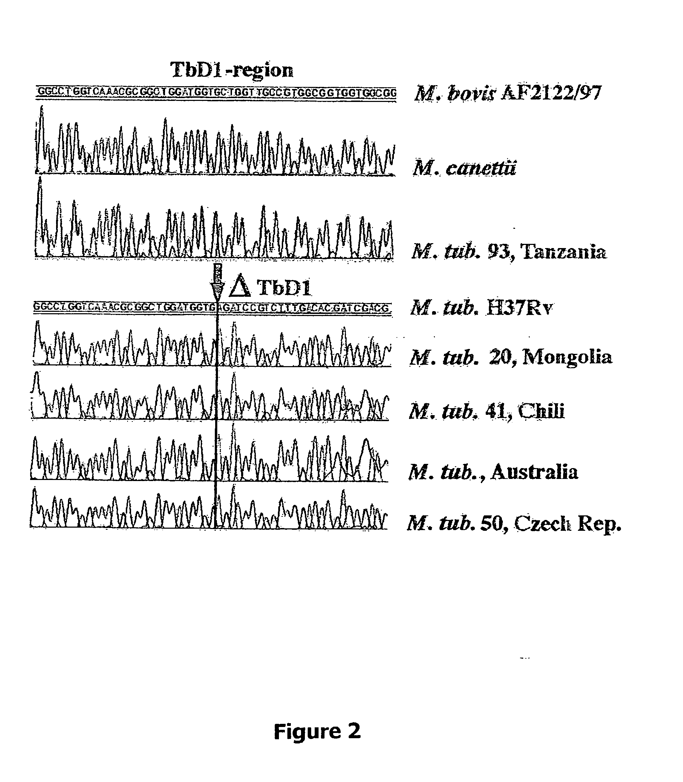

However, the most surprising finding concerning the RvD regions was that TbD1 was absent from 40 of the tested *M. tuberculosis* strains (87 %), including representative strains from major epidemics such as the Haarlem, Beijing and Africa clusters (8). To accentuate this result we named this region *"M. tuberculosis* specific deletion 1" (TbDI). *In silico* sequence comparison of *M. tuberculosis* H37Rv with the corresponding section in *M bovis* AF2122/97 revealed that in *M bovis* this locus comprises two genes encoding membrane proteins belonging to a large family, whereas in *M tuberculosis* H37Rv one of these genes *(mmpS6)* was absent and the second was truncated *(mmpL6).* Unlike the RvD2-RvD5 deletions, the TbD1 region is not flanked by a copy of IS*6110* in *M tuberculosis* H37Rv, suggesting that insertion elements were not involved in the deletion of the 2153 bp fragment. To further investigate whether the 40 *M tuberculosis* strains lacking the TbD1 region had the same genomic organization of this locus as *M tuberculosis* H37Rv, we amplified the TbD1-junction regions of the various strains by PCR using primers flanking the deleted region (Table 1). This approach showed that the size of the amplicons obtained from multiple strains was uniform (Fig. 1) and subsequent sequence analysis of the PCR products revealed that in all tested TbD1-deleted strains the sequence of the junction regions was identical to that of *M. tuberculosis* H37Rv (Fig.2). The perfect conservation of the junction sequences in TbD1-deleted strains of wide geographical diversity suggests that the genetic event which resulted in the deletion occurred in a common progenitor. However, six *M*. *tuberculosis* strains, all characterized by very few or no copies of *IS6110* and spoligotypes that resembled each other (Fig. 3) still had the TbD1 region present. Interestingly, these six strains were also clustered together by MIRU-VNTR analysis (9).

Analysis of partial gene sequences of *oxyR, pncA, katG,* and *gyrA* which have been described as variable between different tubercle bacilli (2, 11, 12, 13) revealed that all tested *M tuberculosis* strains showed *oxyR* and *pncA* partial sequences typical for *M tuberculosis (oxyR -* nucleotide 285 *(oxyR*²⁸⁵):G*, pncA* - codon 57 *(pncA*⁵⁷: CAC ). Based on the *katG* codon 463 *(katG*⁴⁶³) and *gyrA* codon 95 *(gyrA^{9s})* sequence polymorphism, Sreevatsan and colleagues (2) defined three groups among the tubercle bacilli, group 1 showing *katG*⁴⁶³ CTG (Leu), *gyrA⁹⁵* ACC (Thr), group 2 exhibiting *katG*⁴⁶³ CGG (Arg), *gyrA⁹⁵* ACC (Thr), and group 3 showing *katG*⁴⁶³ CGG (Arg), *gyrA⁹⁵* AGC (Ser). According to this scheme, in our study 16 of the 46 tested *M tuberculosis* strains belonged to group 1, whereas 27 strains belonged to group 2 and only 3 isolates to group 3. From the 40 strains that were deleted for region TbD1, 9 showed characteristics of group 1, including the strains belonging to the Beijing cluster, 28 of group 2, including the strains from the Haarlem and Africa clusters and 3 of group 3, including H37Rv and H37Ra. Most interestingly, all six *M. tuberculosis* strains where the TbD1 region was not deleted, contained a leucine (CTG) at *katG*⁴⁶³, which was described as characteristic for ancestral *M. tuberculosis* strains (group 1) (2). As shown in Figure 4, this suggests that during the evolution of *M. tuberculosis* the *katG* mutation at codon 463 CTG (Leu) → CGG (Arg) occurred in a progenitor strain that had region TbD1 deleted. This proposal is supported by the finding that strains belonging to group 1 may or may not have deleted region TbD1, whereas all 30 strains belonging to groups 2 and 3 lacked TBD1 (Fig. 4). Furthermore, all strains of groups 2 and 3 characteristically lacked spacer sequences 33-36 in the direct repeat (DR) region (Fig. 3). It appears that such spacers may be lost but not gained (14). Therefore, TbD1 deleted strains will be referred to hereafter as "modern" *M*. *tuberculosis* strains.

### 2.2. M. canettii:

*M canettii* is a very rare smooth variant of *M. tuberculosis,* isolated usually from patients from, or with connection to, Africa. Although it shares identical 16S rRNA sequences with the other members of the *M tuberculosis* complex, *M canettii* strains differ in many respects including polymorphisms in certain house-keeping genes, IS*1081* copy number, colony morphology, and the lipid content of the cell wall (15, 16). Therefore, we were surprised to find that in *M canettii* all the RD, RvD, and TbD1 regions except the prophages (phiRv1, phiRv2) were present. In contrast, we identified a region (RD^{can}) being specifically absent from all five *M. canettii* strains that partially overlapped RD12 (Fig. 4).

The conservation of the RD, RvD, and TbD1 regions in the genome of *M. canettii* in conjunction with the many described and observed differences suggest that *M. canettii* diverged from the common ancestor of the *M tuberculosis* complex before RD, RvD and TbD1 occurred in the lineages of tubercle bacilli (Fig. 4). This hypothesis is supported by the finding that *M canettii* was shown to carry 26 unique spacer sequences in the direct repeat region (14), that are no longer present in any other member of the *M tuberculosis* complex. Therefore, *M. canettii* represents a fascinating tubercle bacillus, whose detailed genomic analysis may reveal further insights into the evolution of the *M. tuberculosis* complex.

### 2.3. M. africanum:

The isolates designated as *M africanum* studied here originate from West and East-African sources. 11 strains were isolated in Sierra Leone, Nigeria and Guinea and 2 strains in Uganda. One strain comes from the Netherlands.

For the 11 West African isolates, RD analysis indicated that these strains all lack the RD9 region containing *cobL.* Sequence analysis of the RD9 junction region showed that the genetic organization of this locus in West African strains was identical to that of *M bovis* and *M microti* in that the 5' part of *cobL* as well as the genes Rv2073c and Rv2074c were absent. In addition, six strains (2 from Sierra Leone, 4 from Guinea) also lacked RD7, RD8 and RD10 (Table 2). The junction sequences bordering RD7, RD8 and RD10, like those for RD9, were identical to those of *M bovis* and *M microti* strains. As regards the two prophages phiRv1 and phiRv2, the West African strains all contained phiRv2, whereas phiRv1 was absent. No variability was seen for the RvD regions. RvD1-RvD5 and TbD1 were present in all tested West African strains. This shows that *M. africanum* prevalent in West Africa can be differentiated from "modern" *M. tuberculosis* by at least two variable genetic markers, namely the absence of region RD9 and the presence of region TbD1.

In contrast, for East African *M. africanum* and for the isolate from the Netherlands, no genetic marker was found which could differentiate them from *M. tuberculosis* strains. With the exception of prophage phiRv1 (RD3) the 3 strains from Uganda and the Netherlands did not exhibit any of the RD deletions, but lacked the TbD1 region, as do "modem" *M tuberculosis* strains. The absence of the TbD1 region was also confirmed by sequence analysis of the TbD1 junction region, which was found to be identical to that of TbD1 deleted *M. tuberculosis* strains. These results indicate a very close genetic relationship of these strains to *M tuberculosis* and suggest that they should be regarded as *M tuberculosis* rather than *M africanum* strains.

### 2.4. M. microti:

*M microti* strains were isolated in the 1930's from voles (17) and more recently from immuno-suppressed patients (18). These strains are characterized by an identical, characteristic spoligotype, but differ in their *IS6110* profiles. Both, the vole and the human isolates, lacked regions RD7, RD8, RD9, and RD10 as well as a region that is specifically deleted from *M. microti* (RD^{mic}). RD^{mic} was revealed by a detailed comparative genomics study of *M. microti* isolates (19) using clones from a *M. microti* Bacterial Artificial Chromosome (BAC) library. RD^{mic} partially overlaps RD1 from BCG (data not shown). Furthermore, vole isolates missed part of the RD5 region, whereas this region was present in the human isolate. As the junction region of RD5 in *M. microti* was different to that in BCG (data not shown), RD5 was not used as an evolutionary marker.

### 2.5. M. bovis and M. bovis BCG:

*M. bovis* has a very large host spectrum infecting many mammalian species, including man. The collection of *M bovis* strains that was screened for the RD and RvD regions consisted of 2 BCG strains and 18 "classical" *M bovis* strains generally characterized by only one or two copies of IS*6110* from bovine, llama and human sources in addition to three goat isolates, three seal isolates, two oryx isolates, and two *M. bovis* strains from humans that presented a higher number of IS*6110* copies.

Excluding prophages, the distribution of RDs allowed us to differentiate five main groups among the tested *M bovis* strains. The first group was formed by strains that lack RD7, RD8, RD9, and RD10. Representatives of this group are three seal isolates and two human isolates containing between three and five copies of IS*6110* (data not shown). Two oryx isolates harboring between 17 and 20 copies of IS*6110* formed the second group that lacked parts of RD5 in addition to RD7-RD10, and very closely resembled the *M*. *microti* isolates. However, they did not show RD^{mic}, the deletion characteristic of *M. microti* strains (data not shown). Analysis of partial *oxyR* and *pncA* sequences from strains belonging to groups one and two, showed sequence polymorphisms characteristic of *M tuberculosis* strains (*oxyR²⁸⁵*: *G,pncA⁵⁷:* CAC, Ref. 12, 13).

Group three consists of goat isolates that lack regions RD5, RD7, RD8, RD9, RD10, RD12, and RD13. As previously described by Aranaz and colleagues, these strains exhibited an adenosine at position 285 of the *oxyR* pseudogene that is specific for "classical" *M bovis* strains whereas the sequence of the *pncA*⁵⁷ polymorphism was identical to that in *M tuberculosis* (20). This is in good agreement with our results from sequence analysis (Table 2) and the finding that except for RD4, the goat isolates displayed the same deletions as "classical" *M bovis* strains. Taken together, this suggests that the *oxyR*²⁸⁵ mutation (G → A) occurred in *M. bovis* strains before RD4 was lost. Interestingly, the most common *M bovis* strains ("classical" *M. bovis* (21)), isolated from cattle from Argentina, the Netherlands, the UK and Spain, as well as from humans (e. g. multi-drug resistant *M. bovis* from Spain (22)) showed the greatest number of RD deletions and appear to have undergone the greatest loss of DNA relative to other members of the *M tuberculosis* complex. These lacked regions RD4, RD5, RD6, RD7, RD8, RD9, RD10, RD12 and RD13, confirming results obtained with reference strains (5, 6). These strains together with the two BCG strains were the only ones that showed the *pncA*⁵⁷ polymorphism GAC (Asp) in addition to the *oxyR²⁸⁵* mutation (G → A) characteristic of *M. bovis.* Analysis of BCG strains indicate that BCG lacked the same RD regions as "classical" *M bovis* strains in addition to RD1, RD2 and RD14 which apparently occurred during and after the attenuation process (Fig. 4) (6, 23).

In contrast to RDs, the RvD regions were highly conserved in the *M bovis* strains. With the exception of the two IS*6110*-rich oryx isolates, that lacked RvD2, RvD3 and RvD4, all other strains had the five RvD regions present. It is particularly noteworthy that TbD1 was present in all *M. bovis* strains.

However, except for the two human isolates, containing between three and five copies of IS*6110* from group 1, strains designated as *M bovis* showed a single nucleotide polymorphism in the TbD1 region at codon 551 (AAG) of the *mmpL6* gene, relative to *M. canettii, M. africanum* and ancestral *M tuberculosis* strains, which are characterized by codon AAC. Even the strains isolated from seals and from oryx with *oxyR* or *pncA* loci like those of *M. tuberculosis* and with fewer deleted regions than the classical *M. bovis* strains, showed the *mmpL6*⁵⁵¹AAG polymorphism typical for *M bovis* and *M microti* (Table 2, Fig. 4). As such, this polymorphism could serve as a very useful genetic marker for the differentiation of strains that lack RD7, RD8, RD9, and RD 10 and have been classified as *M*. *bovis* or *M. africanum,* but may differ from other strains of the same taxon.

### 3. DISCUSSION

### 3.1. Origin of human tuberculosis

For many years, it was thought that human tuberculosis evolved from the bovine disease by adaptation of an animal pathogen to the human host (3). This hypothesis is based on the property of *M. tuberculosis* to be almost exclusively a human pathogen, whereas *M bovis* has a much broader host range. However, the results from this study unambiguously show that *M bovis* has undergone numerous deletions relative to *M tuberculosis.* This is confirmed by the preliminary analysis of the near complete genome sequence of *M bovis* AF2122/97, a "classical" *M bovis* strain isolated from cattle, which revealed no new gene clusters that were confined specifically to *M bovis.* This indicates that the genome of *M. bovis* is smaller than that of *M. tuberculosis* (24). It seems plausible that *M. bovis* is the final member of a separate lineage represented by *M africanum* (RD9), *M microti* (RD7, RD8, RD9, RD10) and *M bovis* (RD4, RD5, RD7, RD8, RD9, RD10, RD12, RD13) (25) that branched from the progenitor of *M. tuberculosis* isolates. Successive loss of DNA may have contributed to clonal expansion and the appearance of more successful pathogens in certain new hosts.

Whether the progenitor of extant *M tuberculosis* strains was already a human pathogen when the *M africanum* → *M. bovis* lineage separated from the *M tuberculosis* lineage is a subject for speculation. However, we have two reasons to believe that this was the case. Firstly, the six ancestral *M tuberculosis* strains (TbD1⁺, RD9⁺) (Fig.3) that resemble the last common ancestor before the separation of *M tuberculosis* and *M. africanum* are all human pathogens. Secondly, *M canettii,* which probably diverged from the common ancestor of today's *M tuberculosis* strains prior to any other known member of the *M tuberculosis* complex is also a human pathogen. Taken together, this means that those tubercle bacilli, which are thought to most closely resemble the progenitor of *M. tuberculosis* are human and not animal pathogens. It is also intriguing that most of these strains were of African or Indian origin (Fig. 3). It is likely that these ancestral strains predominantly originated from endemic foci (15, 26), whereas "modern" *M. tuberculosis* strains that have lost TbD1 may represent epidemic *M tuberculosis* strains that were introduced into the same geographical regions more recently as a consequence of the worldwide spread of the tuberculosis epidemic.

### 3.2. The evolutionary timescale of the M. tuberculosis complex

Because of the high sequence conservation in housekeeping genes, Sreevatsan *et al.* previously hypothesized that the tubercle bacilli encountered a major bottleneck 15,000 - 20,000 years ago (2). As the conservation of the TbD1 junction sequence in all tested TbD1 deleted strains suggests descendance from a single clone, the TbD1 deletion is a perfect indicator that "modern" *M tuberculosis* strains that account for the vast majority of today's tuberculosis cases definitely underwent such a bottleneck and then spread around the world.

As described in detail in the results section, our analysis showed that the *katG⁴⁶³* CTG→CGG and the subsequent *gyrA*⁹⁵ ACC →AGC mutations, that were used by Sreevatsan and colleagues to designate groups 2 and 3 of their proposed evolutionary pathway of the tubercle bacilli (2), occurred in a lineage of *M. tuberculosis* strains that had already lost TbD1 (Fig.4). Although deletions are more stable markers than point mutations, which may be subject to reversion, a perfect correlation of deletion and point mutation data was found for the tested strains.

This information, together with results from a recent study by Fletcher and colleagues (27), who have shown that *M. tuberculosis* DNAs amplified from naturally mummified Hungarian villagers from the 18^{th} and 19^{th} century belonged to *kat*G⁴⁶³/*gyrA*⁹⁵ groups 2 and 3, suggests that the TbD1 deletion occurred in the lineage of *M. tuberculosis* before the 18^{th} century. This could mean that the dramatic increase of tuberculosis cases later in the 18^{th} century in Europe mainly involved "modern" *M tuberculosis* strains. In addition, it shows that tuberculosis was caused by *M tuberculosis* and not by *M bovis,* a fact which is also described for cases in rural medieval England (28).

There is good evidence that mycobacterial infections occurred in man several thousand years ago. We know that tuberculosis occurred in Egypt during the reign of the pharaohs because spinal and rib lesions pathognomonic of tuberculosis have been identified in mummies from that period (29). Identification of acid fast bacilli as well as PCR amplification of IS*6110* from Peruvian mummies (30) also suggest that tuberculosis existed in pre-Columbian societies of Central and South America. To estimate when the TbD1 bottleneck occurred, it would now be very interesting to know whether the Egyptian and South American mummies carried *M*. *tuberculosis* DNA that had TbD1 deleted or not.

The other major bottleneck, which seems to have occurred for members of the *M.* a*fricanum → M. microti → M. bovis* lineage is reflected by RD9 and the subsequent RD7, RD8 and RD 10 deletions (Fig. 4). These deletions seem to have occurred in the progenitor of tubercle bacilli that - today - show natural host spectra as diverse as humans in Africa, voles on the Orkney Isles (UK), seals in Argentina, goats in Spain, and badgers in the UK. For this reason it is difficult to imagine that spread and adaptation of RD9-deleted bacteria to their specific hosts could have appeared within the postulated 15,000 - 20,000 years of speciation of the *M tuberculosis* complex.

However, more insight into this matter could be gained by RD analysis of ancient DNA samples, e. g. mycobacterial DNA isolated from a 17,000 year old bison skeleton (31). The mycobacterium whose DNA was amplified showed a spoligotype that was most closely related to patterns of *M africanum* and could have been an early representative of the lineage *M. africanum →M. bovis.* With the TbD1 and RD9 junction sequences that we supply here, PCR analyses of ancient DNAs should enable very focused studies to be undertaken to learn more about the timescale within which the members of the *M. tuberculosis* complex have evolved.

### 3.3. Concluding comments

Our study provides an overview of the diversity and conservation of variable regions in a broad range of tubercle bacilli. Deletion analysis of 100 strains from various hosts and countries has identified some evolutionarily "old" *M. canettii, M. tuberculosis* and *M. africanum* strains, most of them of African origin, as well as "modern" *M. tuberculosis* strains, the latter including representatives from major epidemic clusters like Beijing, Haarlem and Africa. The use of deletion analysis in conjunction with molecular typing and analysis of specific mutations was shown to represent a very powerful approach for the study of the evolution of the tubercle bacilli and for the identification of evolutionary markers. In a more practical perspective, these regions, primarily RD9 and TbD1 but also RD1, RD2, RD4, RD7, RD8, RD10, RD12 and RD13 represent very interesting candidates for the development of powerful diagnostic tools for the rapid and unambiguous identification of members of the *M tuberculosis* complex (32). This genetic approach for differentiation can now be used to replace the often confusing traditional division of the *M tuberculosis* complex into rigidly defined subspecies.

Moreover, functional analyses will show whether the TbD1 deletion confers some selective advantage to "modem" *M tuberculosis,* or whether other circumstances contributed to the pandemic of the TbD1 deleted *M tuberculosis* strains.

### EXAMPLE 4

The members of the *M tuberculosis* complex share en unusually high degree of conservation such that the commercially-available nucleic acid probes and amplification assays cannot differentiate these organisms. In addition conventional identification methods are often ambiguous, cumbersome and time consuming because of the slow growth of the organisms.

In the present invention the inventors, by a deletion analysis, solve the problem faced by clinical mycobacteriology laboratories for differentiation within the *M tuberculosis* complex.

This approach allows to perform a diagnostic on a biological fluid by using at least three markers including TBD1. The following table 3 illustrates such a combinaison sufficient to realize the distinction between the members of the *M tuberculosis* complex.

**Table 3**

| | **MARKERS** | | |
|---|---|---|---|
| **MYCOBACTERIUM STRAIN** | **RD4** | **RD9** | **TBD1** |
| M. bovis BCG | - | - | + |
| M. bovis | - | - | + |
| M. africanum | + | - | + |
| M. tuberculosis | + | + | - |
| M. tuberculosis ancêtre | + | + | + |
| M. canettii | + | + | + |

Beside TbD1 marker, preferably at least 2 other markers should be used. Examples of such additional markers available in the literature are listed in the following table 1.

### Supplemental data:

**Table 1: RD, RvD and TbD1 regions and selected primers**

| **Region absent from BCG** | Gene | Size (kb) | Internal Primerpair | **Flanking primers or** 2^{nd} **internal** * **primerpair** |
|---|---|---|---|---|
| RD1 | Rv3871-Rv3879c | 9.5 | RD1in-Rv3878F | RD1-flank.left |
| | | | GTC AGC CAA GTC AGG CTA CC | GAA ACA GTC CCC AGC AGG T |
| | | | RD1in-Rv3878R | RD1-flank.right |
| | | | CAA CGT TGT GGT TGT TGA GG | TTC AAC GGG TTA CTG CGA AT |
| RD2 | Rv1978-Rv1988 | 10.8 | RD2-Rv1979.int.F | RD2-flank.F |
| | | | TAT AGC TCT CGG CAG GTT CC | CTC GAC CGC GAC GAT GTG C |
| | | | RD2-Rv1979-in.tR | RD2-flank.R |
| | | | ATC GGC ATC TAT GTC GGT GT | CCT CGT TGT CAC CGC GTA TG |
| RD3* | Rv1573-Rv1586c | 9.2 | RD3-Rv1586.int.F | RD3-int-REP.F |
| | | | TTA TCT TGG CGT TGA CGA TG | CTG ACG TCG TTG TCG AGG TA* |
| | | | RD3-Rv1586.int.R | RD3-int-REP.R |
| | | | CAT ATA AGG GTG CCC GCT AC | GTA CCC CCA GGC GAT CTT* |
| RD4 | Rv1505c-Rv1516c | 12.7 | RD4-Rv1516.int.F | RD4-flank.F |
| | | | CAA GGG GTA TGA GGT TCA CG | CTC GTC GAA GGC CAC TAA AG |
| | | | RD4-Rv1516.int.R | RD4-flank.R |
| | | | CGG TGA TTC GTG ATT GAA CA | AAG GCG AAC AGA TTC AGC AT |
| RD5* | Rv2346c-Rv2353c | 9.0 | RD5A-Rv2348.int.F | RD5B-plcA.int.F |
| | | | AAT CAC GCT GCT ACT CC | CAA GTT GGG TCT GGT CGA AT |
| | | | RDSA-Rv2348.int.R | RD5B-plcA.int.R |
| | | | GTG CTT TTG CCT CTT GGT C | GCT ACC CAA GGT CTC CTG GT |
| RD6* | Rv3425-Rv3428c | 4.9 | RD6-IS1532F | ND |
| | | | CAG CTG GTG ACT TCA AAT GC | |
| | | | RD6-IS1532R | ND |
| | | | CTC CCG ACA CCT GTT CGT | |
| RD7 | Rv1964-Rv1977 | 12.7 | RD7-Rv1976.int.F | RD7-flank.F |
| | | | TGG ATT GTC GAC GGT ATG AA | GGT AAT CGT GGC CGA CAA G |
| | | | RD7-Rv1976.int.R | RD7-flank.R |
| | | | GGT CGA TAA GGT CAC GGA AC | CAG CTC TTC CCC TCT CGA C |
| RD8 | *ephA-lpqG* | 5.9 | RD8-ephA.F | RD8-flank.F |
| | | | GGT GTG ATT TGG TGA GAC GAT G | CAA TCA GGG CTG TGC TAA CC |
| | | | RD8.ephA.R R | RD3-flank.R |
| | | | ACT TCC TCC TGA CTA ATC CAG GC | CGA CAG TTG TGC GTA CTG GT |
| RD9 | *cobL-Rv2075* | 2.0 | RD9-intF | RD9-flankF |
| | | | CGA TGG TCA ACA CCA CTA CG | GTG TAG GTC AGC CCC ATC C |
| | | | RD9-intR | RD9-flankR |
| | | | CTG GAC CTC GAT GAC CAC TC | GCC CAA CAG CTC GAC ATC |
| RD10 | Rv0221-Rv0223 | 1.9 | RD10-intF | RD10-flankF |
| | | | GTA ACC GCT TCA CCG GAA T | CTG CAA CCA TCC GGT ACA C |
| | | | RD10-intR | RD10-flankR |
| | | | GTC AAC TCC ACG GAA AGA CC | GTC ATG AAC GCC GGA CAG |
| RD11 | Rv2645-Rv2695c | 11.0 | RD11-Rv2646F | RD11-fla-F |
| | | | CGG CAG CTA GAC GAC CTC | TCA CAT AGG GGC TGC GAT AG |
| | | | RD11-Rv2646R | RD11-fla-R |
| | | | AAC GTG CTG CGA TAG GTT TT | AGA GGA ACC TTT CGG TGG TT |
| RD12 | *sseC-*Rv3121 | 2.8 | RD12-Rv3120.int.F | RD12-flank.F |
| | | | GAA ATA CGA GTG CGC TGA CC | GCC ATC AAC GTC AAG AAC CT |
| | | | RD12-Rv3120.int.R | RD12-flank.R |
| | | | CTC TGA ACC ATC GGT GTC G | CGG CCA GGT AAC AAG GAG T |
| RD13 | Rv1255c-Rv1257c | 3.0 | RD13intF | RD13-flank.F |
| | | | GGA TGT CAC TCG GAA CGG CA | CGA TGG TGT TTC TTG GTG AG |
| | | | RDl3intR | RD13-flank.R |
| | | | CAC CGG GCT GAT CGA GCG A | GGA TCG GCT CAG TGA ATA CC |
| RD14 | Rv1765c-Rv1773c | 9.0 | RD14-Rv1769.int.F | RD14-flank.F |
| | | | GTG GAG CAC CTT GAC CTG AT | TTG ATT CGC CAA CAA CTG AA |
| | | | RD14-Rv1769.int.R | RD14-flank.R |
| | | | CGT CGA ATA CGA GTC GAA CA | GGG CTG GTT ACT GTC GAT TC |

| **Region missing from *M. tuberculosis* H37Rv** | | | | |
|---|---|---|---|---|
| RvD1* | | 5.0 | RvD1-int1F | RvD1-int2.F |
| | | | AGC GCG TCG AAC ACC GGC | GAG CCA CTC CGA TGT TGA CT |
| | | | RvD1-int1R | RvD1-int2.R |
| | | | CCT GAA TCC GCG CAA TTC CAT | CAC GCG AAC CCT ACC TAC AT |
| RvD2* | *plcD* | 5.1 | RvD2-int1F - | RvD2-int2F |
| | | | GTT CTC CTG TCG AAC CTC CA | GGA CGG TGA CGG TAT TTG TC |
| | | | RvD2-int R | RvD2-int2R |
| | | | ACT TCA CCG GTT TCA TCT CG | TCG CCA ACT TCT ATG GAC CT |
| RvD3 | | 1.0 | RvD3-intF | RvD3-flank.F |
| | | | ATC GAT CAG GTC GTC AAT GC | AAA CCA TGC AGC GTC TGC CA |
| | | | RvD3-intR | RvD3-flankR |
| | | | ACG CCA CCA TCA AGA TCC | GCG TTT CTG CGT CTG GTT GA |
| RvD4* | PPE gene | 0.8 | RvRD4-intF-PPE | ND |
| | | | GGT TGC CAA CGT TAC CGA TGC | |
| | | | RvD4-intR-PPE | ND |
| | | | CCG GTG GTG GTG GCG GCT | |
| RvD5 | *moa* | 4.0 | RvD5intF | RvD5-flankF |
| | | | GGG TTC ACG TTC ATT ACT GTT C | CCC ATC GTG GTC GTT CAC C |
| | | | RvD5intR | RvD5-flankR |
| | | | CCT GCG CTT ATC TCT AGC GG | GTA CCC GCA CCA CCT GCT G |
| TbD1 | *mmpL6* | 2.1 | TBD1intS.F | TBD1flaI-F |
| | | | CGT TCA ACC CCA AAC AGG TA | CTA CCT CAT CTT CCG GTC CA |
| | | | TBD1intS.R | TBDIflaI-R |
| | | | AAT CGA ACT CGT GGA ACA CC | CAT AGA TCC CGG ACA TGG TG |

| ***katG, gyrA, oxyR', pncA* and *mmpL6* PCR and sequencing primers** | | | | |
|---|---|---|---|---|
| *katG*⁴⁶³ | | | *katG-*2154,225-PCR-F | *katG-*2154,872-SEQ-R |
| | | | CTA CCA GCA CCG TCA TCT CA | ACA AGC TGA TCC ACC GAG AC |
| | | | *kat*G-2155, 157-PCR-R | |
| | | | AGG TCG TAT GGA CGAACA CC | |
| *gyrA⁹⁵* | | | *gyrA-*7*,*127-PCR-F | *gyrA-*7,461F |
| | | | GTT CGT GTG TTG CGT CAA GT | CGG GTG CTC TAT GCA ATG TT |
| | | | *gyrA-* 8,312-PCR-R | |
| | | | CAG CTG GGT GTG CTT GTA AA | |
| *oxyR'²⁸⁵* | | | *oxyR* 2725,559F | oxy*R*-2726,024-SEQ-R |
| | | | TAT GCG ATC AGG CGT ACT TG | CAA AGC AGT GGT TCA GCA GT |
| | | | *oxyR*-2726,024-PCR-R | |
| | | | CAA AGC AGT GGT TCA GCA GT | |
| *pncA*⁵⁷ | | | *pncA*-2288,678-PCR-F | *pncA-* 2289,319-SEQ-R |
| | | | ATC AGG AGC TGC AAA CCA AC | GGC GTC ATG GAC CCT ATA TC |
| | | | *pncA*-2289,319-PCR-R | |
| | | | GGC GTC ATG GAC CCT ATA TC | |
| *mmpL6⁵⁵¹* | | | *mmpL*-seq5F | *mmpL-seq5F* |
| | | | GTA TCA GAG GGA CCG AGC AG | GTA TCA GAG GGA CCG AGC AG |
| | | | TBD1flal-R | |
| | | | CAT AGA TCC CGG ACA TGG TG | |

The RD nomenclature used in this table is based on that used by Brosch *et al.* (2000), (Ref. 25) and differs from that proposed by Behr and coworkers (1999), (Ref. 6). Primer sequences are shown in 5' →3' direction.
* Regions where a second pair of internal primers was used rather than flanking primers, due to flanking repetitive regions, and/or mobile genetic elements.

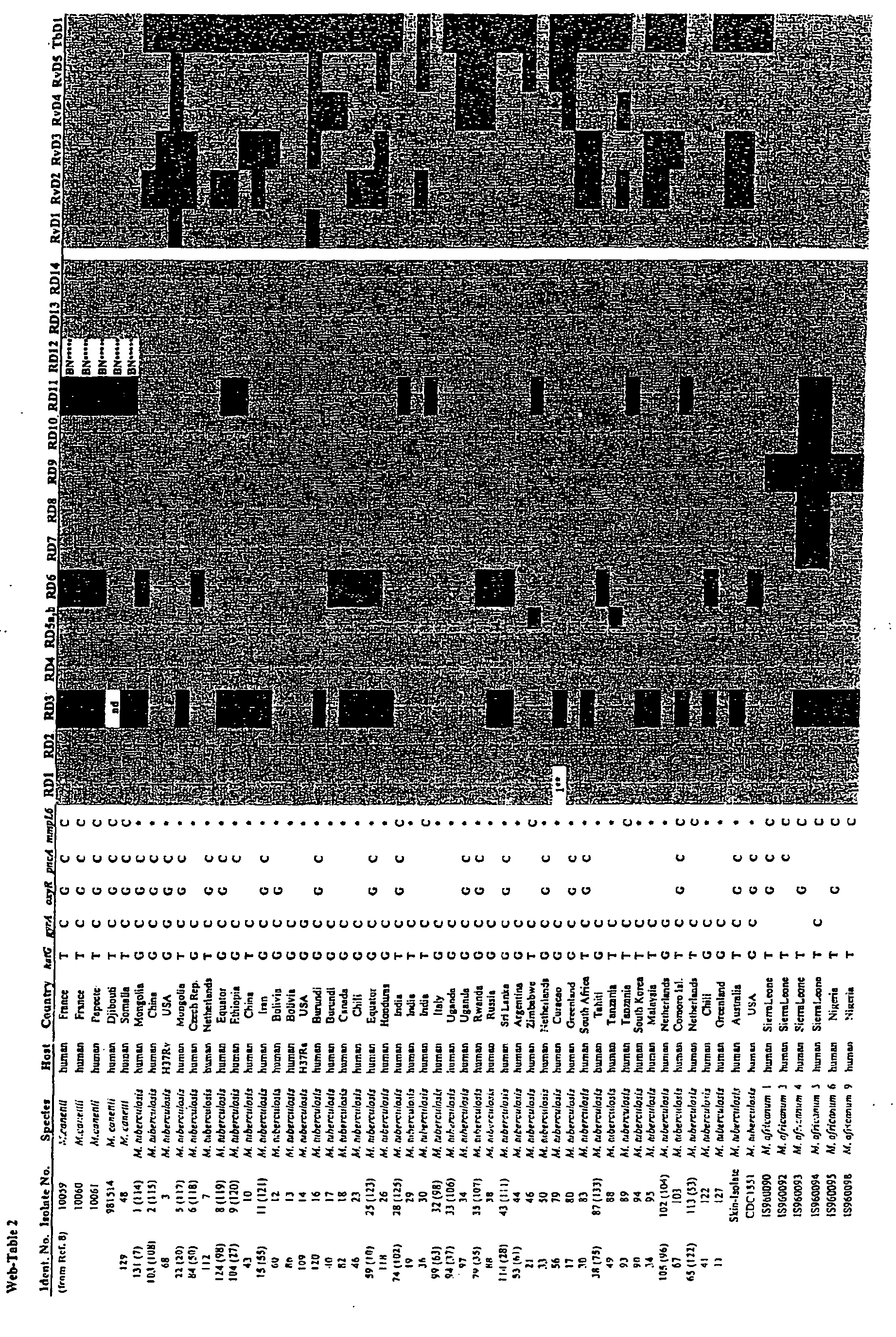

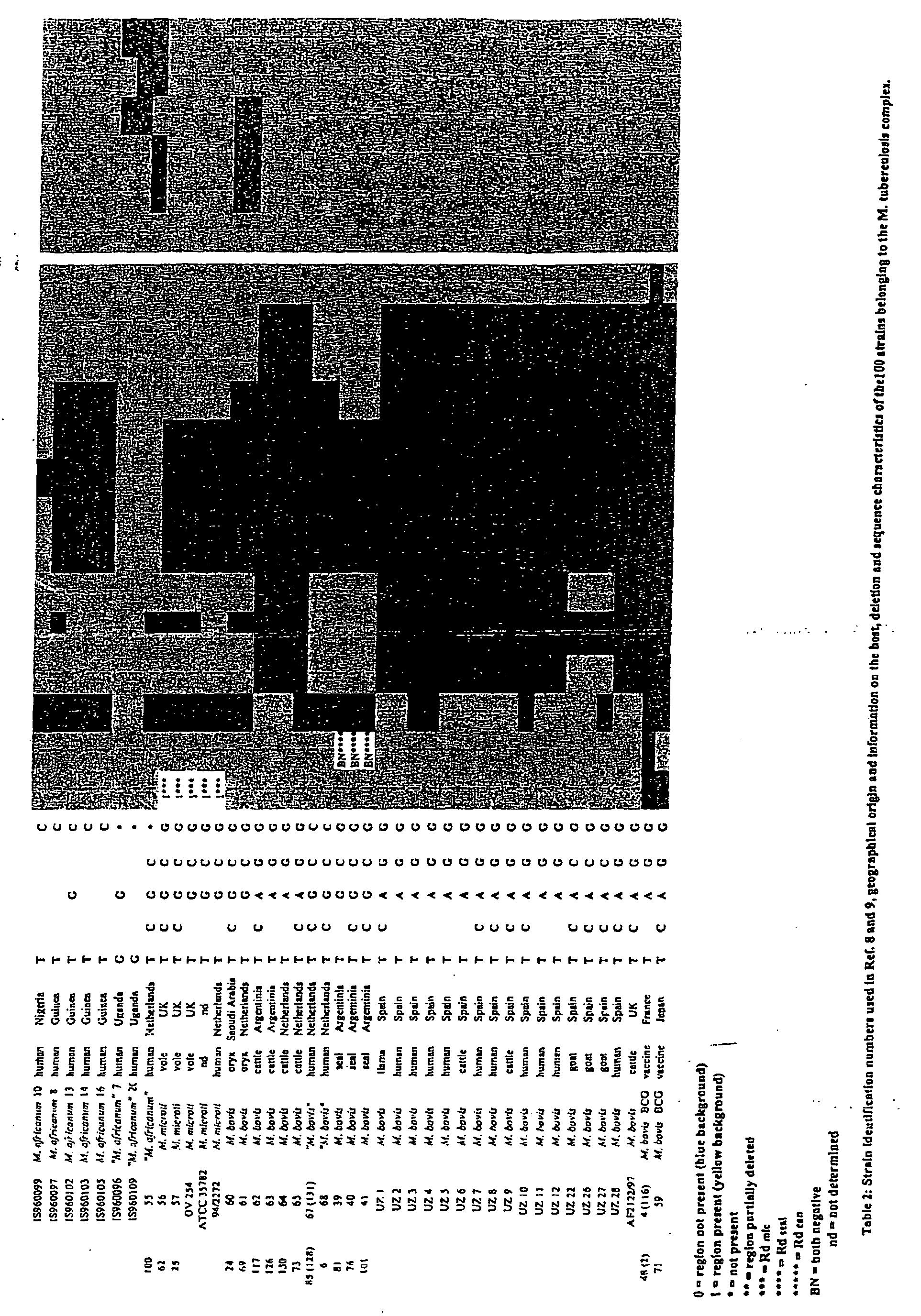

### REFERENCES

1. Boddinghaus, B., Rogall, T., Flohr, T., Blocker, H. & Bottger, E. C. (1990) *J Clin Microbiol* **28***,* 1751-9.
2. Sreevatsan, S., Pan, X., Stockbauer, K. E., Connell, N. D., Kreiswirth, B. N., Whittam, T. S. & Musser, J. M. (1997) *Proc Natl Acad Sci USA* **94**, 9869-74.
3. Stead, W. W., Eisenach, K. D., Cave, M. D., Beggs, M. L., Templeton, G. L., Thoen, C. O. & Bates, J. H. (1995) *Am J Respir Crit Care Med* **151**, 1267-8.
4. Cole, S. T., Brosch, R., Parkhill, J., Garnier, T., Churcher, C., Harris, D., Gordon, S. V., Eiglmeier, K., Gas, S., Barry, C. E., 3rd, Tekaia, F., Badcock, K., Basham, D., Brown, D., Chillingworth, T., Connor, R., Davies, R., Devlin, K., Feltwell, T., Gentles, S., Hamlin, N., Holroyd, S., Hornsby, T., Jagels, K., Barrell, B. G. & et al. (1998) *Nature* **393**, 537-44.
5. Gordon, S. V., Brosch, R., Billault, A., Garnier, T., Eiglmeier, K. & Cole, S. T. (1999) *Mol Microbiol* **32***,* 643-55.
6. Behr, M. A., Wilson, M. A., Gill, W. P., Salamon, H., Schoolnik, G. K., Rane, S. & Small, P. M. (1999) *Science* **284**, 1520-3.
7. Brosch, R., Philipp, W. J., Stavropoulos, E., Colston, M. J., Cole, S. T. & Gordon, S. V. (1999) *Infect Immun* **67**, 5768-74.
8. Kremer, K., van Soolingen, D., Frothingham, R., Haas, W. H., Hermans, P. W., Martin, C., Palittapongampim, P., Plikaytis, B. B., Riley, L. W., Yakrus, M. A., Musser, J. M. & van Embden, J. D. (1999) *J Clin Microbiol* **37***,* 2607-18.
9. Supply, P., Lesjean, S., Savine, E., Kremer, K., van Soolingen, D., & Locht, C. (2001) *J Clin Microbiol* **39***,* 3563-71.
10. van Soolingen, D., de Haas, P. E. W., Hermans, P. W. M. & van Embden, J. D. A. (1994) *Methods Enzymol* **235,** 196-205.
11. Heym, B., Honore, N., Truffot-Pernot, C., Banerjee, A., Schurra, C., Jacobs, W. R., Jr., van Embden, J. D., Grosset, J. H. & Cole, S. T. (1994) *Lancet* **344**, 293-8.
12. Scorpio, A., Collins, D., Whipple, D., Cave, D., Bates, J. & Zhang, Y. (1997) *J Clin Microbiol* **35,** 106-10.
13. Sreevatsan, S., Escalante, P., Pan, X., Gillies, D. A., 2nd, Siddiqui, S., Khalaf, C. N., Kreiswirth, B. N., Bifani, P., Adams, L. G., Ficht, T., Perumaalla, V. S., Cave, M. D., van Embden, J. D. & Musser, J. M. (1996) *J Clin Microbiol* **34,** 2007-10.
14. van Embden, J. D., van Gorkom, T., Kremer, K., Jansen, R., van Der Zeijst, B. A. & Schouls, L. M. (2000) *J Bacteriol* **182,** 2393-401.
15. van Soolingen, D., Hoogenboezem, T., de Haas, P. E., Hermans, P. W., Koedam, M. A., Teppema, K. S., Brennan, P. J., Besra, G. S., Portaels, F., Top, J., Schouls, L. M. & van Embden, J. D. (1997) *Int J Syst Bacteriol* **47***,* 1236-45.
16. Papa, F., Laszlo, A., David, H. L. & Daffe, M. (1989) *Acta Leprol 7* ( Suppl.) 98-101.
17. Wells, A. Q., (1937) *Lancet* 1221.
18. van Soolingen, D., van der Zanden, A. G., de Haas, P. E., Noordhoek, G. T., Kiers, A., Foudraine, N. A., Portaels, F., Kolk, A. H., Kremer, K. & van Embden, J. D. (1998) J *Clin Microbiol* **36**, 1840-5.
19. Brodin, P., *et al.* (2002) in preparation
20. Aranaz, A., Liebana, E., Gomez-Mampaso, E., Galan, J. C., Cousins, D., Ortega, A., Blazquez, J., Baquero, F., Mateos, A., Suarez, G. & Dominguez, L. (1999) *Int J Syst Bacteriol* **49***,* 1263-73.
21. van Soolingen, D., P.E.W. de Haas, J. Haagsma, T. Eger, P.W.M. Hermans, V. Ritacco, A. Alito, & J.D.A van Embden. (1994) *J. Clin. Microbiol.* 32, 2425-33.
22. Samper, S., Martin, C., Pinedo, A., Rivero, A., Blazquez, J., Baquero, F., van Soolingen, D. & van Embden, J*.* (1997) *Aids* **11***,* 1237-42.
23. Mahairas, G. G., Sabo, P. J., Hickey, M. J., Singh, D. C. & Stover, C. K. (1996) *J Bacteriol* **178***,* 1274-82.
24. Gordon, S. V., Eiglmeier, K., Garnier, T., Brosch, R., Parkhill, J., Barrell, B., Cole, S. T. & Hewinson, R. G. (2001) *Tuberculosis* **81**, 157-63.
25. Brosch, R., S. V. Gordon, K. Eiglmeier, T. Garnier, F. Tekaia, E. Yeramanian,& S. T. Cole. (1999) in Molecular genetics of mycobacteria, eds. Hatful G. F. & Jacobs, W. R. Jr. (American Society for Microbiology, Washington, D.C.), pp. 19-36.
26. Radhakrishnan, I., K, M. Y., Kumar, R. A. & Mundayoor, S. (2001) *J Clin Microbiol* **39***,* 1683.
27. Fletcher, H. A., Donoghue, H. D., Holton, J., Pap, I. & Spigelman, M. (2002) *Am. J. Phys. Anthropol,* in press.
28. Mays, S., Taylor, G. M., Legge, A. J., Young, D. B. & Turner-Walker, G. (2001) *Am J Phys Anthropol* **114,** 298-311.
29. Nerlich, A. G., Haas, C. J., Zink, A., Szeimies, U. & Hagedorn, H. G. (1997) *Lancet* **350**, 1404.
30. Salo, W. L., Aufderheide, A. C., Buikstra, J. & Holcomb, T. A. (1994) *Proc Natl Acad Sci USA* **91**, 2091-4.
31. Rothschild, B. M., Martin, L. D., Lev, G., Bercovier, H., Bar-Gal, G. K., Greenblatt, C., Donoghue, H., Spigelman, M. & Brittain, D. (2001) *Clin Infect Dis* **33**, 305-11.
32. Parsons, L.M., Brosch, R., Cole, S. T., Somoskovi, A., Loder, A., Britzel, G., van Soolingen, D., Hale, Y., & Salfinger, M. (2001) in preparation

## Claims

1. An isolated or purified nucleic acid wherein said nucleic acid is selected from the group consisting of:
a) SEQ ID N°1;
b) Nucleic acid having a sequence fully complementary to SEQ ID N°1;

2. A nucleic acid fragment of SEQ ID N°1 according to claim 1 selected from the group consisting of:
a) SEQ ID N°4;
b) Nucleic acid having a sequence fully complementary to SEQ D N°4;
c) Nucleic acid fragment of SEQ ID N°4 comprising at least 20 consecutive nucleotides of SEQ ID N°4;

3. The isolated or purified nucleic acid of claim 1 wherein said nucleic acid comprises at least a deletion of a nucleic acid fragment according to claim 2.

4. Isolated or purified polypeptides encoded by the nucleic acid of claims 1 or 2.

5. The polypeptide of claim 4 selected among polypeptide with sequence SEQ ID N° 8, N° 10, N°12 and N°2

6. Isolated or purified nucleic acid encoding the polypeptide according to claim 5.

7. Isolated or purified nucleic acid according to claim 6 selected among:
- SEQ ID N° 7 encoding the polypeptide of SEQ ID N°8;
- SEQ ID N° 9 encoding the polypeptide of SEQ ID N°10;
- SEQ ID N°11 encoding the polypeptide of SEQ ID N°12;

8. A recombinant vector comprising a nucleic acid sequence selected among nucleic acids according to claims 1, 2, 6 and 7.

9. The recombinant vector of claim 8 consisting of vector named X229 introduced into the recombinant *Escherichia coli* deposited with the CNCM on February 18, 2002 under N° I-2799.

10. A recombinant cell comprising a nucleic acid sequence selected among nucleic acids according to claims 1, 2, 6 and 7, and vectors according to claims 8 and 9.

11. The recombinant cell according to claim 10 consisting of the *Escherichia coli* deposited with the CNCM on February 18, 2002 under N° 1-2799.

12. An *in vitro* method for the detection and identification of Mycobacterium tuberculosis in a biological sample, comprising the following steps:
a) isolation of the DNA from the biological sample to be analyzed or production of a cDNA from the RNA of the biological sample,
b) detection of the nucleic acid sequences of the mycobacterium present in said biological sample,
c) analysis for the presence or the absence of a nucleic acid fragment according to claim 2.

13. The method as claimed in claim 12, in which the detection of the mycobacterial DNA sequences is carried out using nucleotide sequences complementary to said DNA sequences.

14. The method as claimed in claim 12 or 13, in which the detection of the mycobacterial DNA sequences is carried out by amplification of these sequences using primers.

15. The method of claim 12, in which the detection of the mycobacterial DNA sequences is carried out by amplification of these sequences using primers comprising 8 to 30 consecutive nucleotides of the polynucleotide according to anyone of claims 2, 3 and 6, and selected in order to amplify the nucleic acid fragment of claim 2.

16. The method as claimed in claim 14 or 15, in which the primers have a nucleotide sequence chosen from the group comprising SEQ ID N°13, SEQ ID N°14, SEQ ID N°15, SEQ ID N°16, SEQ ID N°17 and SEQ ID N°18.

17. The use of at least one pair of primers as defined in claim 16 for the amplification of a DNA sequence from *Mycobacterium tuberculosis, Mycobacterium africanum, Mycobacterium canetti, Mycobacterium microti, Mycobacterium bovis,* or *Mycobacterium bovis BCG.*

18. A protein, polypeptide or polypeptide fragment resulting from the expression of all or part of the nucleic acid fragment as defined in claim 2 .

19. A mono- or polyclonal antibody, its chimeric fragments or antibodies, **characterized in that** they are capable of specifically recognizing a product as defined in claim 18.

20. A kit for the discriminatory detection of the presence of an antigen of *Mycobacterium tuberculosis* with TbD1 deletion according to SEQ ID n°4, or Mycobacterium Tuberculosis without TbD1 deletion, or *Mycobacterium africanum,* or *Mycobacterium canetti,* or *Mycobacterium microti,* or *Mycobacterium bovis,* or *Mycobacterium bovis BCG,* in a biological sample comprising the following elements:
a) an antibody as claimed in claim 19,
b) the reagents for constituting the medium suitable for the immunological reaction,
c) the reagents allowing the detection of the antigen-antibody complexes produced by the immunological reaction.

21. A kit for the detection of the TbD1 deletion according to SEQ ID n°4 in Mycobacterium tuberculosis in a biological sample, comprising the following elements:
a) at least one pair of primers comprising 8 to 30 consecutive nucleotides of the polynucleotide according to claims 2, 3 and 6.
b) the reagents necessary to carry out a DNA amplification reaction.

22. The kit of claim 21, wherein said pair of primers is selected among the primers chosen from the group comprising SEQ ID N°13, SEQ ID N°14, SEQ ID N°15, SEQ ID N°16, SEQ ID N°17 and SEQ ID N°18.

23. Use of the TbD1 deletion according to SEQ ID N°4 as a genetic marker for the differentiation of *Mycobacterium* strain of *Mycobacterium Tuberculosis* complex.

24. Use of the genetic marker according to claim 23 in association with at least one genetic marker selected among RD1, RD2, RD3, RD4, RD5, RD6, RD7, RD8, RD9, RD10, RD11, RD13, RD14, RvD1, RvD2, RvD3, RvD4, RvD5, katG⁴⁶³, gyrA⁹⁵, oxyR^{'285}, pncA⁵⁷ for the differentiation of *Mycobacterium* strain of *Mycobacterium Tuberculosis* complex.

25. The use of claim 24 wherein two genetic markers RD4 and RD9 are selected to be used in association with the genetic marker of claim 24.

26. The use of claim 24 wherein the analysis is performed by a technique selected among sequence hybridization, nucleic acid amplification, antigen-antibody complex.

27. A kit according to claim 21 or 22, for the detection and identification of *Mycobacteria* from the *Mycobacterium Tuberculosis* complex in a biological sample, further comprising the following elements:
c) at least one pair of primers specific of the genetic markers selected among RD1, RD2, RD3, RD4, RD5, RD6, RD7, RD8, RD9, RD10, RD11, RD13, RD14, RvD1, RvD2, RvD3, RvD4, RvD5, katG⁴⁶³, gyrA⁹⁵, oxyR^{'285} and pncA⁵⁷.

28. A kit according to claim 27, **characterized in that** said pairs of primers specific of genetic markers according to c) are:
d) one pair of primers specific of the genetic marker RD4, and
e) one pair of primers specific of the genetic marker RD9.

## Patentansprüche

1. Isolierte oder gereinigte Nukleinsäure, wobei die Nukleinsäure ausgewählt wird aus der Gruppe bestehend aus:
a) SEQ ID NO: 1;
b) einer Nukleinsäure, welche eine Sequenz aufweist, welche zu SEQ ID NO: 1 vollständig komplementär ist.

2. Nukleinsäurefragment von SEQ ID NO: 1 nach Anspruch 1, welches ausgewählt wird aus der Gruppe bestehend aus:
a) SEQ ID NO: 4;
b) einer Nukleinsäure, welche eine Sequenz aufweist, welche zu SEQ ID NO: 4 vollständig komplementär ist;
c) einem Nukleinsäurefragment von SEQ ID NO: 4, weiches wenigstens 20 aufeinander folgende Nukleotide von SEQ ID NO: 4 umfasst.

3. Isolierte oder gereinigte Nukleinsäure nach Anspruch 1, wobei die Nukleinsäure wenigstens eine Deletion eines Nukleinsäurefragments nach Anspruch 2 umfasst.

4. Isolierte oder gereinigte Polypeptide, die durch die Nukleinsäure nach Anspruch 1 oder 2 kodiert werden.

5. Polypeptid nach Anspruch 4, welches unter Polypeptiden mit der Sequenz SEQ ID NO: 8, NO: 10, NO: 12 und NO: 2 ausgewählt wird.

6. Isolierte oder gereinigte Nukleinsäure, welche das Polypeptid nach Anspruch 5 kodiert.

7. Isolierte oder gereinigte Nukleinsäure nach Anspruch 6, welche ausgewählt wird unter:
- SEQ ID NO: 7, welche das Polypeptid von SEQ ID NO: 8 kodiert;
- SEQ ID NO: 9, welche das Polypeptid von SEQ ID NO: 10 kodiert;
- SEQ ID NO: 11, welche das Polypeptid von SEQ ID NO: 12 kodiert.

8. Rekombinanter Vektor, welcher eine Nukleinsäuresequenz umfasst, welche unter den Nukleinsäuren nach den Ansprüchen 1, 2, 6 und 7 ausgewählt wird.

9. Rekombinanter Vektor nach Anspruch 8, bestehend aus dem als X229 bezeichneten Vektor, welcher in das rekombinante *Escherichia coli,* das bei der CNCM am 18. Februar 2002 unter der Nr. 1-2799 hinterlegt worden ist, eingeführt vorliegt.

10. Rekombinante Zelle, welche eine Nukleinsäuresequenz umfasst, welche unter den Nukleinsäuren nach den Ansprüchen 1, 2, 6 und 7 und Vektoren nach den Ansprüchen 8 und 9 ausgewählt wird.

11. Rekombinante Zelle nach Anspruch 10, bestehend aus dem bei der CNCM am 18. Februar 2002 unter der Nr. I-2799 hinterlegten *Escherichia coli.*

12. *In* vitro-Verfahren zum Nachweis und zur Identifizierung von Mycobacterium tuberculosis in einer biologischen Probe, welches die folgenden Schritte umfasst:
a) Isolierung der DNA aus der zu analysierenden biologischen Probe oder Herstellung einer cDNA ausgehend von der RNA der biologischen Probe;
b) Nachweis der Nukleinsäuresequenzen des Mycobacteriums, die in der biologischen Probe vorhanden sind;
c) Analyse des Vorhandenseins oder der Abwesenheit eines Nukleinsäurefragments nach Anspruch 2.

13. Verfahren nach Anspruch 12, bei welchem der Nachweis der mycobakteriellen DNA-Sequenzen unter Verwendung von Nukleotidsequenzen, die zu den DNA-Sequenzen komplementär sind, ausgeführt wird.

14. Verfahren nach Anspruch 12 oder 13, bei welchem der Nachweis der mycobakteriellen DNA-Sequenzen durch Amplifizierung dieser Sequenzen unter Verwendung von Primern ausgeführt wird.

15. Verfahren nach Anspruch 12, bei welchem der Nachweis der mycobakteriellen DNA-Sequenzen durch Amplifizierung dieser Sequenzen unter Verwendung von Primern, welche 8 bis 30 aufeinander folgende Nukleotide des Polynukleotids nach einem der Ansprüche 2, 3 und 6 umfassen und entsprechend ausgewählt sind, um das Nukleinsäurefragment von Anspruch 2 zu amplifizieren, ausgeführt wird.

16. Verfahren nach Anspruch 14 oder 15, bei welchem die Primer eine Nukleotidsequenz aufweisen, die aus der Gruppe umfassend SEQ ID NO: 13, SEQ ID NO: 14, SEQ ID NO: 15, SEQ ID NO: 16, SEQ ID NO: 17 und SEQ ID NO: 18 ausgewählt wird.

17. Verwendung von wenigstens einem Paar von Primern, wie in Anspruch 16 definiert, zur Amplifizierung einer DNA-Sequenz aus *Mycobacterium tuberculosis, Mycobacterium africanum, Mycobacterium canetti, Mycobacterium microti, Mycobacterium bovis* oder *Mycobacterium bovis* BCG.

18. Protein, Polypeptid oder Polypeptidfragment, welches aus der Expression der Gesamtheit oder eines Teils des Nukleinsäurefragments, wie in Anspruch 2 definiert, resultiert.

19. Mono- oder polyklonaler Antikörper, dessen chimäre Fragmente oder Antikörper, **dadurch gekennzeichnet, dass** sie in der Lage sind, spezifisch ein Produkt, wie in Anspruch 18 definiert, zu erkennen.

20. Kit zum unterscheidenden Nachweis des Vorhandenseins eines Antigens von *Mycobacterium tuberculosis* mit TbD1-Deletion gemäß SEQ ID NO: 4 oder Mycobacterium tuberculosis ohne TbD1-Deletion oder *Mycobacterium africanum* oder *Mycobacterium canetti* oder *Mycobacterium microti* oder *Mycobacterium bovis* oder *Mycobacterium bovis* BCG in einer biologischen Probe, welcher die folgenden Elemente umfasst:
a) einen Antikörper nach Anspruch 19,
b) die Reagenzien zum Herstellen des Mediums, welches für die immunologische Reaktion geeignet ist,
c) die Reagenzien, welche den Nachweis der durch die immunologische Reaktion erzeugten Antigen-Antikörper-Komplexe erlauben.

21. Kit zum Nachweis der TbD1-Deletion gemäß SEQ ID NO: 4 in Mycobacterium tuberculosis in einer biologischen Probe, welcher die folgenden Elemente umfasst:
a) wenigstens ein Paar vom Primern, welche 8 bis 30 aufeinander folgende Nukleotide des Polynukleotids gemäß den Ansprüchen 2, 3 und 6 umfassen;
b) die Reagenzien, welche erforderlich sind, um eine DNA-Amplifizierungsreaktion auszuführen.

22. Kit nach Anspruch 21, wobei das Paar von Primern ausgewählt wird unter den Primern, die ausgewählt werden aus der Gruppe umfassend SEQ ID NO: 13, SEQ ID NO: 14, SEQ ID NO: 15, SEQ ID NO: 16, SEQ ID NO: 17 und SEQ ID NO: 18.

23. Verwendung der TbD1-Deletion gemäß SEQ ID NO: 4 als genetischen Marker für die Differenzierung des *Mycobacterium*-Stammes des *Mycobacterium tuberculosis*-Komplexes*.*

24. Verwendung des genetischen Markers nach Anspruch 23 in Verbindung mit wenigstens einem genetischen Marker, ausgewählt unter RD1, RD2, RD3, RD4, RD5, RD6, RD7, RD8, RD9, RD10, RD11, RD13, RD14, RvD1, RvD2, RvD3, RvD4, RvD5, katG⁴⁶³, gyrA⁹⁵, oxyR^{,285}, pncA⁵⁷, zur Differenzierung des *Mycobacterium*-Stammes des *Mycobacterium tuberculosis-*Komplexes.

25. Verwendung nach Anspruch 24, wobei zwei genetische Marker RD4 und RD9 ausgewählt werden, um in Verbindung mit dem genetischen Marker nach Anspruch 24 verwendet zu werden.

26. Verwendung nach Anspruch 24, wobei die Analyse durch eine Technik, welche unter Sequenzhybridisierung, Nukleinsäureamplifizierung, Antigen-Antikörper-Komplex ausgewählt wird, ausgeführt wird.

27. Kit nach Anspruch 21 oder 22 für den Nachweis und die Identifizierung von *Mycobacteria* aus dem *Mycobacterium tuberculosis*-Komplex in einer biologischen Probe, welcher ferner die folgenden Elemente umfasst:
c) wenigstens ein Paar von Primern, die spezifisch sind für die genetischen Marker, die unter RD1, RD2, RD3, RD4, RD5, RD6, RD7, RD8, RD9, RD10, RD11, RD13, RD14, RvD1, RvD2, RvD3, RvD4, RvD5, katG⁴⁶³, gyrA⁹⁵, oxyR'²⁸⁵ und pncA⁵⁷ ausgewählt werden.

28. Kit nach Anspruch 27, **dadurch gekennzeichnet, dass** das Paar von Primern, die für die genetischen Marker gemäß c) spezifisch sind, :
d) ein Paar von Primern, die für den genetischen Marker RD4 spezifisch sind, und
e) ein Paar von Primern, die für den genetischen Marker RD9 spezifisch sind,
ist.

## Revendications

1. Acide nucléique isolé ou purifié, où ledit acide nucléique est choisi dans le groupe constitué de :
a) SEQ ID N° 1 ;
b) un acide nucléique ayant une séquence totalement complémentaire de SEQ ID N° 1.

2. Fragment d'acide nucléique de SEQ ID N° 1 selon la revendication 1, choisi dans le groupe constitué de :
a) SEQ ID N° 4 :
b) un acide nucléique ayant une séquence totalement complémentaire de SEQ ID N° 4 ;
c) un fragment d'acide nucléique de SEQ ID N° 4 comprenant au moins 20 nucléotides consécutifs de SEQ ID N° 4.

3. Acide nucléique isolé ou purifié selon la revendication 1, où ledit acide nucléique comprend au moins une délétion d'un fragment d'acide nucléique selon la revendication 2.

4. Polypeptides isolés ou purifiés codés par l'acide nucléique selon la revendication 1 ou 2.

5. Polypeptide selon la revendication 4, choisi parmi les polypeptides avec les séquences SEQ ID N° 8, N°10, N° 12 et N° 2.

6. Acide nucléique isolé ou purifié codant pour le polypeptide selon la revendication 5.

7. Acide nucléique isolé ou purifié selon la revendication 6, choisi parmi:
- SEQ ID N° 7 codant pour le polypeptide de SEQ ID N° 8;
- SEQ ID N° 9 codant pour le polypeptide de SEQ ID N° 10 ;
- SEQ ID N° 11 codant pour le polypeptide de SEQ ID N° 12.

8. Vecteur recombinant comprenant une séquence d'acide nucléique choisie parmi des acides nucléiques selon les revendications 1, 2, 6 et 7.

9. Vecteur recombinant selon la revendication 8 constitué du vecteur nommé X229 introduit dans *l'Escherichia coli* recombinant déposé à la CNCM le 18 février 2002 sous le N° I-2799.

10. Cellule recombinante comprenant une séquence d'acide nucléique choisie parmi des acides nucléiques selon les revendications 1, 2, 6 et 7, et vecteurs selon les revendications 8 et 9.

11. Cellule recombinante selon la revendication 10 constituée de l*'Escherichia coli* recombinant déposé à la CNCM le 18 février 2002 sous le N° I-2799.

12. Procédé *in vitro* de détection et d'identification de Mycobacterium tuberculosis dans un échantillon biologique, comprenant les étapes suivantes :
a) isolement de l'ADN à partir de l'échantillon biologique à analyser ou production d'un ADNc à partir de l'ARN de l'échantillon biologique,
b) détection des séquences d'acide nucléique de la mycobactérie présente dans ledit échantillon biologique,
c) analyse de la présence ou de l'absence d'un fragment d'acide nucléique selon la revendication 2.

13. Procédé selon la revendication 12, dans lequel la détection des séquences d'ADN mycobactérien est réalisée en utilisant des séquences nucléotidiques complémentaires des dites séquences d'ADN.

14. Procédé selon la revendication 12 ou 13, dans lequel la détection des séquences d'ADN mycobactérien est réalisée par amplification de ces séquences en utilisant des amorces.

15. Procédé selon la revendication 12, dans lequel la détection des séquences d'ADN mycobactérien est réalisée par amplification de ces séquences en utilisant des amorces comprenant 8 à 30 nucléotides consécutifs du polynucléotide selon l'une quelconque des revendications 2, 3 et 6, et choisies afin d'amplifier le fragment d'acide nucléique selon la revendication 2.

16. Procédé selon la revendication 14 ou 15, dans lequel les amorces ont une séquence nucléotidique choisie dans le groupe comprenant SEQ ID N° 13, SEQ ID N° 14, SEQ ID N° 15, SEQ ID N° 16, SEQ ID N° 17 et SEQ ID N° 18.

17. Utilisation d'un moins une paire d'amorces selon la revendication 16, pour l'amplification d'une séquence d'ADN de *Mycobacterium tuberculosis, Mycobacterium africanum, Mycobacterium canetti, Mycobacterium microti, Mycobacterium bovis* ou *Mycobacterium bovis BCG.*

18. Protéine, polypeptide ou fragment polypeptidique résultant de l'expression de la totalité ou d'une partie du fragment d'acide nucléique selon la revendication 2.

19. Anticorps monoclonal ou polyclonal, ses fragments ou anticorps chimériques, **caractérisés en ce qu'**ils sont capables de reconnaître spécifiquement un produit selon la revendication 18.

20. Kit pour la détection discriminatoire de la présence d'un antigène de *Mycobacterium tuberculosis* avec délétion TbD1 selon SEQ ID N° 4, ou de *Mycobacterium tuberculosis* sans délétion TbD1, ou de *Mycobacterium africanum,* ou de *Mycobacterium canetti,* ou de *Mycobacterium microti,* ou de *Mycobacterium bovis,* ou de *Mycobacterium bovis BCG,* dans un échantillon biologique comprenant les éléments suivants :
a) un anticorps selon la revendication 19,
b) les réactifs pour constituer le milieu approprié à la réaction immunologique,
c) les réactifs permettant la détection des complexes antigène-anticorps produits par la réaction immunologique.

21. Kit pour la détection de la délétion TbD1 selon SEQ ID N° 4 dans Mycobacterium tuberculosis dans un échantillon biologique, comprenant les éléments suivants :
a) au moins une paire d'amorces comprenant 8 à 30 nucléotides consécutifs du polynucléotide selon les revendications 2, 3 et 6,
b) les réactifs nécessaires pour réaliser une réaction d'amplification d'ADN.

22. Kit selon la revendication 21, dans lequel ladite paire d'amorces est choisie parmi les amorces choisies dans le groupe comprenant SEQ ID N° 13, SEQ ID N° 14, SEQ ID N° 15, SEQ ID N° 16, SEQ ID N° 17 et SEQ ID N° 18.

23. Utilisation de la délétion TbD1 selon SEQ ID N° 4 en tant que marqueur génétique pour la différenciation de la souche *Mycobacterium* du complexe *Mycobacterium tuberculosis.*

24. Utilisation du marqueur génétique selon la revendication 23, en association avec au moins un marqueur génétique choisi parmi RD1, RD2, RD3, RD4, RD5, RD6, RD7, RD8, RD9, RD10, RD11, RD13, RD14, RvD1, RvD2, RvD3, RvD4, RvD5, katG⁴⁶³, gyrA⁹⁵, oxyR'²⁸⁵, pncA⁵⁷ pour la différenciation de la souche *Mycobacterium* du complexe Mycobacterium *tuberculosis.*

25. Utilisation selon la revendication 24, dans laquelle deux marqueurs génétiques RD4 et RD9 sont choisis pour être utilisés en association avec le marqueur génétique selon la revendication 24.

26. Utilisation selon la revendication 24, dans laquelle l'analyse est effectuée par une technique choisie parmi l'hybridation de séquences, l'amplification d'acide nucléique, le complexe antigène-anticorps.

27. Kit selon la revendication 21 ou 22, pour la détection et l'identification de *Mycobactéries* du complexe *Mycobacterium tuberculosis* dans un échantillon biologique, comprenant en outre les éléments suivants :
c) au moins une paire d'amorces spécifiques des marqueurs génétiques choisis parmi RD1, RD2, RD3, RD4, RD5, RD6, RD7, RD8, RD9, RD10, RD11, RD13, RD14, RvD1, RvD2, RvD3, RvD4, RvD5, katG⁴⁶³, gyrA⁹⁵, oxyR^{'285} et pncA⁵⁷.

28. Kit selon la revendication 27, **caractérisé en ce que** lesdites paires d'amorces spécifiques des marqueurs génétiques selon c) sont :
d) une paire d'amorces spécifiques du marqueur génétique RD4, et
e) une paire d'amorces spécifiques du marqueur génétique RD9.
